(19) Europäisches Patentamt
European Patent Office
Office européen des brevets

(11) EP 3 492 571 A1

(12) EUROPEAN PATENT APPLICATION

(43) Date of publication:
**05.06.2019 Bulletin 2019/23**

(51) Int Cl.:
***C11D 3/386*** *(2006.01)*

(21) Application number: **18192650.2**

(22) Date of filing: **30.04.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.05.2014 EP 14166842**
**02.05.2014 EP 14166844**
**28.05.2014 EP 14170247**
**16.06.2014 EP 14172551**
**09.03.2015 EP 15158240**

(62) Document number(s) of the earlier application(s) in accordance with Art. 76 EPC:
**15720956.0 / 3 137 587**

(27) Previously filed application:
**30.04.2015 PCT/EP2015/059566**

(71) Applicant: **Novozymes A/S**
**2880 Bagsvaerd (DK)**

(72) Inventors:
- **GORI, Klaus**
  **2880 Bagsvaerd (DK)**
- **BALTSEN, Lilian, Eva, Tang**
  **2880 Bagsvaerd (DK)**
- **ALLESEN-HOLM, Marie**
  **2880 Bagsvaerd (DK)**
- **NOERGAARD, Allan**
  **2880 Bagsvaerd (DK)**
- **LEHMBECK, Jan**
  **2880 Bagsvaerd (DK)**
- **SCHNORR, Kirk, Matthew**
  **2880 Bagsvaerd (DK)**

(74) Representative: **NZ EPO Representatives**
**Krogshoejvej 36**
**2880 Bagsvaerd (DK)**

Remarks:
This application was filed on 05.09.2018 as a divisional application to the application mentioned under INID code 62.

(54) **DETERGENT COMPOSITION**

(57) The present invention concerns the use of a polypeptide having DNase activity for preventing, reducing or removing a biofilm from an item, wherein the item is a hard surface, a composition comprising such polypeptide and a method for cleaning.

EP 3 492 571 A1

## Description

### Reference to a Sequence Listing

This application contains a Sequence Listing in computer readable form, which is incorporated herein by reference.

### Field of the Invention

The present invention concerns the use of a polypeptide having deoxyribonuclease (DNase) activity for preventing, reducing or removing a biofilm from an item, a composition comprising such polypeptide and a cleaning method.

### Background of invention

Microorganisms generally live attached to surfaces in many natural, industrial, and medical environments, encapsulated by extracellular substances including biopolymers and macromolecules. The resulting layer of slime encapsulated microorganism is termed a biofilm. Biofilms are the predominant mode of growth of bacteria in the natural environment, and bacteria growing in biofilms exhibit distinct physiological properties.

Hard surfaces are exposed to DNA and bacteria from the environment in which they are used. Dishware are exposed to DNA and bacteria from the food served or cooked in the dishes. Some of these bacteria are capable of adhering to the item and form a biofilm on the item. The presence of DNA and bacteria implies that the items become sticky and therefore soil adheres to the sticky areas. This soil has shown difficult to remove by commercially available detergent compositions. Further, when very dirty items are washed together with less dirty items, the dirt present in the wash liquor tends to stick to the biofilm. As a result hereof the item is more "soiled" after cleaning than before wash. Further, these bacteria are a source of bad odor, which develops after use of the item. The bad odor is difficult to remove and may remain as a malodour in the item even after wash. The reason for this bad odor is adhesion of bacteria to the surface, e.g. in a crack in a floor or a dish. Because of the adhesion to the surface, the bacteria may remain even after wash, and continue to be a source of bad odor.

Also, the interior of dishwashing machines or laundry washing machines may be subject to growth of biofilm. The growth and proliferation of microbes in a these machines generally occurs from exposure to prolonged warm, moist environments which may contain soap residue and clothing or food residues. This environment leads to the development of undesirable odors and biofilm. Biofilm growth further leads to degradation of the rubber which potentially results in reduced life cycle of the rubber parts or the entire washing machine.

International patent application WO 2011/098579 concerns bacterial deoxyribonuclease compounds and methods for biofilm disruption and prevention.

### Summary of the Invention

The present invention concerns the use of a polypeptide having DNase activity for preventing, reducing or removing a biofilm from an item. The invention further concerns a detergent composition, which composition comprises a polypeptide having deoxyribonuclease (DNase) activity and a metal care agent. Further is claimed a detergent composition comprising a polypeptide having deoxyribonuclease (DNase) activity and a strong sequestering builder and/or a builder selected from the group consisting of sodium citrate, citric acid, alcanol amines, sodium carbonate, sodium bicarbonate and Amino-tris-(methylene-phosphonic acid) (AMP). In addition is claimed a cleaning method for preventing, reducing or removing a biofilm from an item comprising the steps of:

a) contacting an item to a composition comprising a polypeptide having DNase activity or to a liquid solution comprising a polypeptide having DNase activity;
b) completing at least one cleaning cycle; and
c) optionally rinsing the item,
wherein the item is a hard surface and/or a dishware.

### Definitions

<u>Allelic variant:</u> The term "allelic variant" means any of two or more alternative forms of a gene occupying the same chromosomal locus. Allelic variation arises naturally through mutation, and may result in polymorphism within populations. Gene mutations can be silent (no change in the encoded polypeptide) or may encode polypeptides having altered amino acid sequences. An allelic variant of a polypeptide is a polypeptide encoded by an allelic variant of a gene.

The term automatic dishwashing composition refers to compositions intended for cleaning dishware such as plates, cups, glasses, bowls, pots, cutlery, spoons, knives, forks, serving utensils, ceramics, plastics, cutting boards, china and glass ware in a dishwashing machine. The terms encompass any materials/compounds selected for domestic or industrial washing applications and the form of the product can be liquid, powder or granulate. In addition to lipase, the automatic dishwashing composition contains detergent components such as polymers, bleaching systems, bleach activators, bleach catalysts, silicates, dyestuff and metal care agents.

Bacterial: In the context of the present invention, the term "bacterial" in relation to polypeptide (such as an enzyme, e.g. a DNAse) refers to a polypeptide encoded by and thus directly derivable from the genome of a bacteria, where such bacteria has not been genetically modified to encode said polypeptide, e.g. by introducing the encoding sequence in the genome by recombinant DNA technology. In the context of the present invention, the term "bacterial DNAse" or "polypeptide having DNAse activity obtained from a bacterial source" or "polypeptide is of bacterial origin" thus refers to a DNAse encoded by and thus directly derivable from the genome of a bacterial species, where the bacterial species has not been subjected to a genetic modification introducing recombinant DNA encoding said DNAse. Thus, the nucleotide sequence encoding the bacterial polypeptide having DNAse activity is a sequence naturally in the genetic background of a bacterial species. The bacterial polypeptide having DNAse activity encoding by such sequence may also be referred to a wildtype DNAse (or parent DNAse). In a further aspect, the invention provides provides polypeptides having DNase activity, wherein said polypeptides are substantially homologous to a bacterial DNase. In the context of the present invention, the term "substantially homologous" denotes a polypeptide having DNase activity which is at least 80%, preferably at least 85%, more preferably at least 90%, more preferably at least 95%, even more preferably at least 96%, 97%, 98%, and most preferably at least 99% identical to the amino acid sequence of a selected bacterial DNase.

Biofilm: A biofilm is any group of microorganisms in which cells stick to each other or stick to a surface, such as a textile, dishware or hard surface or another kind of surface. These adherent cells are frequently embedded within a self-produced matrix of extracellular polymeric substance (EPS). Biofilm EPS is a polymeric conglomeration generally composed of extracellular DNA, proteins, and polysaccharides. Biofilms may form on living or non-living surfaces. The microbial cells growing in a biofilm are physiologically distinct from planktonic cells of the same organism, which, by contrast, are single-cells that may float or swim in a liquid medium.

Bacteria living in a biofilm usually have significantly different properties from planktonic bacteria of the same species, as the dense and protected environment of the film allows them to cooperate and interact in various ways. One benefit of this environment is increased resistance to detergents and antibiotics, as the dense extracellular matrix and the outer layer of cells protect the interior of the community.

On laundry biofilm producing bacteria can be found among the following species: Acinetobacter sp., Aeromicrobium sp., Brevundimonas sp., Microbacterium sp., Micrococcus luteus, Pseudomonas sp., Staphylococcus epidermidis, and Stenotrophomonas sp. On hard surfaces biofilm producing bacteria can be found among the following species: Acinetobacter sp., Aeromicrobium sp., Brevundimonas sp., Microbacterium sp., Micrococcus luteus, Pseudomonas sp., Staphylococcus epidermidis, and Stenotrophomonas sp In one embodiment the biofilm producing strain is *Brevundimonas* sp.. In one embodiment the biofilm producing strain is *Pseudomonas alcaliphila* or *Pseudomonas fluorescens.*

cDNA: The term "cDNA" means a DNA molecule that can be prepared by reverse transcription from a mature, spliced, mRNA molecule obtained from a eukaryotic or prokaryotic cell. cDNA lacks intron sequences that may be present in the corresponding genomic DNA. The initial, primary RNA transcript is a precursor to mRNA that is processed through a series of steps, including splicing, before appearing as mature spliced mRNA.

Coding sequence: The term "coding sequence" means a polynucleotide, which directly specifies the amino acid sequence of a polypeptide. The boundaries of the coding sequence are generally determined by an open reading frame, which begins with a start codon such as ATG, GTG, or TTG and ends with a stop codon such as TAA, TAG, or TGA. The coding sequence may be a genomic DNA, cDNA, synthetic DNA, or a combination thereof.

Color difference (L value); A Lab color space is a color-opponent space with dimension L for lightness. L value, L* represents the darkest black at L* = 0, and the brightest white at L* = 100. In the context of the present invention L value is also referred to as color difference. The color difference method is used in example 2 of the present patent application.

The term "cleaning cycle" is defined herein as a cleaning operation wherein a hard surface or a dishware is contacted to a wash liquor for a period of time by circulating the wash liquor and spraying the wash liquor onto the dishware in order to clean the dishware and finally the superfluous wash liquor is removed. A cleaning cycle may be repeated one, two, three, four, five or even six times at the same or at different temperatures. Hereafter the hard surface dishware is generally rinsed and dried. One of the cleaning cycles can be a soaking step, where the hard surface or the dishware is left soaking in the wash liquor for a period of time.

Control sequences: The term "control sequences" means nucleic acid sequences necessary for expression of a polynucleotide encoding a mature polypeptide of the present invention. Each control sequence may be native (i.e., from the same gene) or foreign (i.e., from a different gene) to the polynucleotide encoding the polypeptide or native or foreign to each other. Such control sequences include, but are not limited to, a leader, polyadenylation sequence,

propeptide sequence, promoter, signal peptide sequence, and transcription terminator. At a minimum, the control sequences include a promoter, and transcriptional and translational stop signals. The control sequences may be provided with linkers for the purpose of introducing specific restriction sites facilitating ligation of the control sequences with the coding region of the polynucleotide encoding a polypeptide.

By the term "deep cleaning" is meant disruption or removal of a biofilm or components of a biofilm such as polysaccharides, proteins, DNA, soil or other components present in the biofilm.

Detergent components: the term "detergent components" is defined herein to mean the types of chemicals which can be used in detergent compositions. Examples of detergent components are alkalis, surfactants, metal care agents, hydrotropes, builders, co-builders, chelators or chelating agents, bleaching system or bleach components, polymers, fabric hueing agents, fabric conditioners, foam boosters, suds suppressors, dispersants, dye transfer inhibitors, fluorescent whitening agents, perfume, optical brighteners, bactericides, fungicides, soil suspending agents, soil release polymers, anti-redeposition agents, enzyme inhibitors or stabilizers, enzyme activators, antioxidants and solubilizers.

Detergent Composition: The term "detergent composition" refers to compositions that find use in the removal of undesired compounds from items to be cleaned, such as hard surfaces or dishware. The detergent composition may be used to e.g. clean dishware for both household cleaning and industrial cleaning. The terms encompass any materials/compounds selected for the particular type of cleaning composition desired and the form of the product (e.g., liquid, gel, powder, granulate, paste, or spray compositions) and includes, but is not limited to, detergent compositions (e.g., liquid and/or solid laundry detergents and fine fabric detergents; fabric fresheners; fabric softeners; and textile and laundry pre-spotters/pretreatment). In addition to containing the enzyme of the invention, the detergent formulation may contain one or more additional enzymes (such as proteases, amylases, lipases, cutinases, cellulases, endoglucanases, xyloglucanases, pectinases, pectin lyases, xanthanases, peroxidaes, haloperoxygenases, catalases and mannanases, or any mixture thereof), and/or detergent components such as surfactants, builders, chelators or chelating agents, bleach system or bleach components, polymers, fabric conditioners, foam boosters, suds suppressors, dyes, perfume, tannish inhibitors, optical brighteners, bactericides, fungicides, soil suspending agents, anti-corrosion agents, enzyme inhibitors or stabilizers, enzyme activators, transferase(s), hydrolytic enzymes, oxido reductases, bluing agents and fluorescent dyes, antioxidants, and solubilizers.

Dishware: The term "dish ware" is intended to mean any form of kitchen utensil, dinner set or tableware such as but not limited to pans, plates, cups, knives, forks, spoons, porcelain etc. The dishware can be made of any suitable material such as metal, glass, rubber, plastic, PVC, acrylics, ceramics, china or porcelain.

Dish washing composition: The term "dish washing composition" refers to compositions comprising detergent components, which composition is intended for cleaning dishes, table ware, glass ware, cutting boards, pots, pans, cutlery and all forms of compositions for cleaning hard surfaces areas in kitchens. The present invention is not restricted to any particular type of dish wash composition or any particular detergent. The dish washing composition can be used for both domestic dish washing, industrial and institutional dish washing including composition for ADW.

DNase (deoxyribonuclease): The term "DNase" means a polypeptide with DNase activity activity that catalyzes the hydrolytic cleavage of phosphodiester linkages in the DNA backbone, thus degrading DNA. For purposes of the present invention, DNase activity is determined according to the procedure described in the Assay I. In one aspect, the polypeptides of the present invention have at least 20%, e.g., at least 40%, at least 50%, at least 60%, at least 70%, at least 80%, at least 90%, at least 95%, or at least 100% of the DNase activity of the mature polypeptide of SEQ ID NO: 2. For purposes of the present invention, DNase activity is determined according to the procedure described in the Assay I. In one embodiment of the present invention, the DNAse activity of polypeptide having is at least 105%, e.g., at least 110%, at least 120%, at least 130%, at least 140%, at least 160%, at least 170%, at least 180%, or at least 200% with reference to the DNase activity of the mature polypeptide of SEQ ID NO: 2, a polypeptide comprising or consisting of the sequence set forth in SEQ ID NO: 3, a polypeptide comprising or consisting of the sequence set fort in SEQ ID NO: 5, a polypeptide comprising or consisting of the mature polypeptide of SEQ ID NO: 6, a polypeptide comprising or consisting of the mature polypeptide of SEQ ID NO: 7 or a polypeptide comprising or consisting of the mature polypeptide of SEQ ID NO: 8.

Expression: The term "expression" includes any step involved in the production of a polypeptide including, but not limited to, transcription, post-transcriptional modification, translation, post-translational modification, and secretion.

Expression vector: The term "expression vector" means a linear or circular DNA molecule that comprises a polynucleotide encoding a polypeptide and is operably linked to control sequences that provide for its expression.

Fragment: The term "fragment" means a polypeptide having one or more (e.g., several) amino acids absent from the amino and/or carboxyl terminus of a mature polypeptide or domain; wherein the fragment has DNase activity. In one aspect, a fragment contains at least 206 amino acid residues (e.g., amino acids 1 to 206 of SEQ ID NO: 2), at least 205 amino acid residues (e.g., amino acids 2 to 206 of SEQ ID NO: 2), or at least 204 amino acid residues (e.g., amino acds 3 to 206 of SEQ ID NO: 2). In one aspect, a fragment contains at least 139 amino acid residues (e.g., amino acids 50 to 188 of SEQ ID NO: 5),at least 188 amino acid residues (e.g., amino acids 1 to 188 of SEQ ID NO: 5).

Fungal: In the context of the present invention the term "fungal" in relation to polypeptide (such as an enzyme,

e.g. a DNAse) refers to a polypeptide encoded by and thus directly derivable from the genome of a fungus, where such fungus has not been genetically modified to encode said polypeptide, e.g. by introducing the encoding sequence in the genome by recombinant DNA technology. In the context of the present invention, the term "fungal DNAse" or "polypeptide having DNAse activity obtained from a fungal source" or "polypeptide is of fungal origin" thus refers to a DNAse encoded by and thus directly derivable from the genome of a fungal species, where the fungal species has not been subjected to a genetic modification introducing recombinant DNA encoding said DNAse. Thus, the nucleotide sequence encoding the fungal polypeptide having DNAse activity is a sequence naturally in the genetic background of a fungal species. The fungal polypeptide having DNAse activity encoding by such sequence may also be referred to a wildtype DNAse (or parent DNAse). In a further aspect, the invention provides provides polypeptides having DNase activity, wherein said polypeptides are substantially homologous to a fungal DNase. In the context of the present invention, the term "substantially homologous" denotes a polypeptide having DNase activity which is at least 80%, preferably at least 85%, more preferably at least 90%, more preferably at least 95%, even more preferably at least 96%, 97%, 98%, and most preferably at least 99% identical to the amino acid sequence of a selected fungal DNase.

Hard surface: The term "Hard surface" is defined herein as hard surfaces including floors, tables, walls, roofs etc. as well as surfaces of hard objects such as cars (car wash) and dishes (dishware). The term "hard surface" includes also the surfaces in the interior of washing machines, such as the interior of laundry washing machines or dishwashing machines, this includes soap intake box, walls, windows, baskets, racks, nozzles, pumps, sump, filters, pipelines, tubes, joints, seals, gaskets, fittings, impellers, drums, drains, traps, coin traps inlet and outlets. The term hard surface does not encompass textile or fabric.

Hard surface cleaning: The term "Hard surface cleaning" is defined herein as cleaning of hard surfaces, such as reducing or removing biofilm from a hard surface, wherein hard surfaces may include floors, tables, walls, roofs etc. as well as surfaces of hard objects such as cars (car wash) and dishes (dish wash). Hard surface cleaning also includes cleaning the interior of washing machines, such as the interior of laundry washing machines or dishwashing machines, this includes cleaning soap intake box, walls, windows, baskets, racks, nozzles, pumps, sump, filters, pipelines, tubes, joints, seals, gaskets, fittings, impellers, drums, drains, traps, coin traps inlet and outlets. Dish washing includes but are not limited to cleaning of plates, cups, glasses, bowls, pots, cutlery, spoons, knives, forks, serving utensils, ceramics, plastics, cutting boards, china and glass ware.

Host cell: The term "host cell" means any cell type that is susceptible to transformation, transfection, transduction, or the like with a nucleic acid construct or expression vector comprising a polynucleotide of the present invention. The term "host cell" encompasses any progeny of a parent cell that is not identical to the parent cell due to mutations that occur during replication.

Isolated: The term "isolated" means a substance in a form or environment that does not occur in nature. Non-limiting examples of isolated substances include (1) any non-naturally occurring substance, (2) any substance including, but not limited to, any enzyme, variant, nucleic acid, protein, peptide or cofactor, that is at least partially removed from one or more or all of the naturally occurring constituents with which it is associated in nature; (3) any substance modified by the hand of man relative to that substance found in nature; or (4) any substance modified by increasing the amount of the substance relative to other components with which it is naturally associated (e.g., recombinant production in a host cell; multiple copies of a gene encoding the substance; and use of a stronger promoter than the promoter naturally associated with the gene encoding the substance). An isolated substance may be present in a fermentation broth sample; e.g. a host cell may be genetically modified to express the polypeptide of the invention. The fermentation broth from that host cell will comprise the isolated polypeptide.

Laundering: The term "laundering" relates to both household laundering and industrial laundering and means the process of treating textiles with a solution containing a cleaning or detergent composition of the present invention. The laundering process can for example be carried out using e.g. a household or an industrial washing machine or can be carried out by hand.

By the term "malodor" is meant an odor which is not desired on clean items. The cleaned item should smell fresh and clean without malodors adhered to the item. One example of malodor is compounds with an unpleasant smell, which may be produced by microorganisms. Another example is unpleasant smells can be sweat or body odor adhered to an item which has been in contact with human or animal. Another example of malodor can be the odor from spices, which sticks to items for example curry or other exotic spices which smells strongly. One way of measuring the ability of an item to adhere malodor is by using Assay II.

Mature polypeptide: The term "mature polypeptide" means a polypeptide in its final form following translation and any post-translational modifications, such as N-terminal processing, C-terminal truncation, glycosylation, phosphorylation, etc. In one aspect, the mature polypeptide is amino acids 1 to 206 of SEQ ID NO: 2 and amino acids -37 to -16 of SEQ ID NO: 2 are a signal peptide and amino acids -15 to -1 of SEQ ID NO: 2 are a propeptide. In one aspect, the mature polypeptide is amino acids 1 to 188 of SEQ ID NO: 5 and amino acids -17 to -1 of SEQ ID NO: 2 are a signal peptide. In one aspect, the mature polypeptide is amino acids 1 to 110 of SEQ ID NO: 6, the mature polypeptide is amino acids 1 to 109 of SEQ ID NO: 7 or the mature polypeptide is amino acids 1 to 206 of SEQ ID NO: 8. It is known in the

art that a host cell may produce a mixture of two of more different mature polypeptides (*i.e.,* with a different C-terminal and/or N-terminal amino acid) expressed by the same polynucleotide. It is also known in the art that different host cells process polypeptides differently, and thus, one host cell expressing a polynucleotide may produce a different mature polypeptide (*e.g.,* having a different C-terminal and/or N-terminal amino acid) as compared to another host cell expressing the same polynucleotide. In one aspect, a mature polypeptides contains up to 206 amino acid residues and of SEQ ID NO: 2, SEQ ID NO: 3 or SEQ ID NO: 8 (*e.g.,* amino acids 1 to 206 of SEQ ID NO: 2), or up to 204 amino acid residues (*e.g.,* amino acids 3 to 206 of SEQ ID NO: 2).

Mature polypeptide coding sequence: The term "mature polypeptide coding sequence" means a polynucleotide that encodes a mature polypeptide having DNase activity. In one aspect, the mature polypeptide coding sequence is join nucleotides 1 to 242, 309 to 494, 556 to 714 and 766 to 907 of SEQ ID NO: 1. In one aspect, the mature polypeptide coding sequence is nucleotides 52 to 864 of SEQ ID NO: 4, where three introns are predicted in the sequence in amino acids in position 76-164, 289-362 and 520-615 of SEQ ID NO: 4. A secretion signal is present at amino acids in positions 1-51 of SEQ ID NO: 4.

Nucleic acid construct: The term "nucleic acid construct" means a nucleic acid molecule, either single- or double-stranded, which is isolated from a naturally occurring gene or is modified to contain segments of nucleic acids in a manner that would not otherwise exist in nature or which is synthetic, which comprises one or more control sequences.

Operably linked: The term "operably linked" means a configuration in which a control sequence is placed at an appropriate position relative to the coding sequence of a polynucleotide such that the control sequence directs expression of the coding sequence.

Rubber: The term "rubber" is intended to cover any standard rubber which must be vulcanized to provide a dimensionally stable rubber article. The term "dimensionally stable" is intended to encompass a vulcanized rubber article that is structurally able to be handled without disintegrating into smaller portions. Thus, the article must exhibit some degree of structural integrity and, being a rubber, a certain degree of flexural modulus. The specific types of rubber are listed below and have been utilized previously within the rubber industry for a variety of applications and are generally well known and taught throughout the prior art.

The rubber component or components of the inventive rubber formulation and cured article is preferably selected from the group consisting of nitrile rubber [such as acrylonitrile-butadiene rubber (NBR)], ethylene propylene diene monomer (EPDM) rubber, hydrogenated NBR, carboxylated NBR, and mixtures thereof. It is important to consider the desired physical properties of the rubber article when selecting the polymer and the curing system. For example, high molecular weight EPDM polymers tend to exhibit higher green strength and tensile strength and lower compression set compared to lower molecular weight polymers. In peroxide cured elastomers, it is often more desirable to use these high molecular weight polymers as peroxide composites exhibit poorer 'hot tear' strength at elevated temperatures compared to sulfur cured composites.

Sequence identity: The relatedness between two amino acid sequences or between two nucleotide sequences is described by the parameter "sequence identity". For purposes of the present invention, the sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), pref-erably version 5.0.0 or later. The parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

(Identical Residues x 100)/(Length of Alignment – Total Number of Gaps in Alignment).

For purposes of the present invention, the sequence identity between two deoxyribonucleotide sequences is determined using the Needleman-Wunsch algorithm (Needleman and Wunsch, 1970, supra) as implemented in the Needle program of the EMBOSS package (EM-BOSS: The European Molecular Biology Open Software Suite, Rice et al., 2000, supra), prefer-ably version 5.0.0 or later. The parameters used are gap open penalty of 10, gap extension penalty of 0.5, and the EDNAFULL (EMBOSS version of NCBI NUC4.4) substitution matrix. The output of Needle labeled "longest identity" (obtained using the -nobrief option) is used as the percent identity and is calculated as follows:

(Identical Deoxyribonucleotides x 100)/(Length of Alignment – Total Number of Gaps in Alignment).

Stringency conditions:

The term "very low stringency conditions" means for probes of at least 100 nucleotides in length, prehybridization and hybridization at 42°C in 5X SSPE, 0.3% SDS, 200 micrograms/ml sheared and denatured salmon sperm DNA, and 25% formamide, following standard Southern blotting procedures for 12 to 24 hours. The carrier material is finally washed three times each for 15 minutes using 2X SSC, 0.2% SDS at 45°C.

The term "low stringency conditions" means for probes of at least 100 nucleotides in length, prehybridization and hybridization at 42°C in 5X SSPE, 0.3% SDS, 200 micrograms/ml sheared and denatured salmon sperm DNA, and 25% formamide, following standard Southern blotting procedures for 12 to 24 hours. The carrier material is finally washed three times each for 15 minutes using 2X SSC, 0.2% SDS at 50°C.

The term "medium stringency conditions" means for probes of at least 100 nucleotides in length, prehybridization and hybridization at 42°C in 5X SSPE, 0.3% SDS, 200 micrograms/ml sheared and denatured salmon sperm DNA, and 35% formamide, following standard Southern blotting procedures for 12 to 24 hours. The carrier material is finally washed three times each for 15 minutes using 2X SSC, 0.2% SDS at 55°C.

The term "medium-high stringency conditions" means for probes of at least 100 nucleotides in length, prehybridization and hybridization at 42°C in 5X SSPE, 0.3% SDS, 200 micrograms/ml sheared and denatured salmon sperm DNA, and 35% formamide, following standard Southern blotting procedures for 12 to 24 hours. The carrier material is finally washed three times each for 15 minutes using 2X SSC, 0.2% SDS at 60°C.

The term "high stringency conditions" means for probes of at least 100 nucleotides in length, prehybridization and hybridization at 42°C in 5X SSPE, 0.3% SDS, 200 micrograms/ml sheared and denatured salmon sperm DNA, and 50% formamide, following standard Southern blotting procedures for 12 to 24 hours. The carrier material is finally washed three times each for 15 minutes using 2X SSC, 0.2% SDS at 65°C.

The term "very high stringency conditions" means for probes of at least 100 nucleotides in length, prehybridization and hybridization at 42°C in 5X SSPE, 0.3% SDS, 200 micrograms/ml sheared and denatured salmon sperm DNA, and 50% formamide, following standard Southern blotting procedures for 12 to 24 hours. The carrier material is finally washed three times each for 15 minutes using 2X SSC, 0.2% SDS at 70°C.

Subsequence: The term "subsequence" means a polynucleotide having one or more (*e.g.,* several) nucleotides absent from the 5' and/or 3' end of a mature polypeptide coding sequence; wherein the subsequence encodes a fragment having DNase activity. In one aspect, a subsequence contains at least 796 nucleotides (*e.g.,* nucleotides 112 to 907 of SEQ ID NO: 1), at least 793 nucleotides (*e.g.,* nucleotides 115 to 907 of SEQ ID NO: 1), or at least 790 nucleotides (*e.g.,* nucleotides 118 to 907 of SEQ ID NO: 1). In one aspect, a subsequence contains at least 587 nucleotides (*e.g.,* nucleotides 278 to 864 of SEQ ID NO: 4), at least 650 nucleotides (*e.g.,* nucleotides 215 to 864 of SEQ ID NO: 4), or at least 816nucleotides (*e.g.,* nucleotides 52 to 864 of SEQ ID NO: 4).

Variant: The term "variant" means a polypeptide having same activity as the parent enzyme comprising an alteration, *i.e.,* a substitution, insertion, and/or deletion, at one or more (*e.g.,* several) positions. A substitution means replacement of the amino acid occupying a position with a different amino acid; a deletion means removal of the amino acid occupying a position; and an insertion means adding an amino acid adjacent to and immediately following the amino acid occupying a position. In the context of the present invention, a variant of an identified DNAse has the enzymatic activity of the parent, *i.e.* the capacity of catalyzing the hydrolytic cleavage of phosphodiester linkages in the DNA backbone (deoxyribonuclease activity). In one embodiment, the deoxyribonuclease activity of the variant is increased with reference to the parent DNAse, e.g. the mature polypeptide of SEQ ID NO: 2.

Wash liquor: The term "wash liquor" is intended to mean the solution or mixture of water and detergents optionally including enzymes used for hard surface cleaning or for dishwashing.

**Detailed Description of the Invention**

The inventors have surprisingly found that polypeptides having deoxyribonuclease (DNase) activity can be used for preventing, reducing or removing biofilm from items such as hard surfaces.

Hard surfaces, which are exposed to DNA and bacteria from the environment can develop a biofilm on the surface. Such biofilm may be difficult to remove and tend to stick on the surface. One example is the internal surfaces in washing machines and dish washing machines which are often covered by biofilm. The growth and proliferation of microbes in a these machines generally occurs from exposure to prolonged warm, moist environments which may contain soap residue and clothing or food residues. This environment leads to the development of undesirable odors and biofilm. Biofilm growth further leads to degradation of the rubber which potentially results in reduced life cycle of the rubber parts or the entire washing machine.

However, the inventors have found that polypeptides having deoxyribonuclease (DNase) activity can be used for preventing, reducing or removing biofilm from washing machines or dish washing machines.

Another example of biofilm formation on hard surfaces is hard surfaces present in warm, moist environments

such as hard surfaces in kitchen areas, bathrooms or swimming pool areas. The hard surfaces can be made of metal, glass, rubber, plastic, PVC, acrylics, ceramics, china or porcelain.

The polypeptide having DNase activity can be used for preventing, reducing or removing stickiness of the item. Biofilm comprising DNA can be sticky and thus soil adheres to the biofilm. In one embodiment the polypeptide having DNase activity can be used for preventing, reducing or removing adherence of soil to the item.

The polypeptide having DNase activity can be used on items which have a pronounced biofilm growth or if parts of the item have a pronounced growth of biofilm. The polypeptide having DNase activity can be used for pretreating these parts of the item, where biofilm stains are pronounced on the item.

The presence of biofilm on an item may cause bad odor, such as a malodor. One source of bad odor produced by biofilm is E-2-nonenal. Therefore presence of biofilm in a washing machine or a dishwashing machine is not desired. In addition to bad smell from the washing machine or dishwashing machine, the bad odor can stick to the items washed in the machines. For example laundry items or dishware may smell bad when removed wet from the machine or even when the item is dry. The use of polypeptides having DNase activity can prevent, reduce or remove the amount of E-2-nonenal.

In one embodiment of the invention, the polypeptide having DNase activity can prevent, reduce or remove biofilm and odor from at least one strain of *Brevundimonas sp.,* at least one strain of *Pseudomonas fluorescens* or at least one strain of *Pseudomonas alcaliphila.*

In one embodiment of the invention, the amount of E-2-nonenal present on a wet item is prevented, reduced or removed. In one embodiment the amount of E-2-nonenal present on the dried item is prevented, reduced or removed.

The polypeptide having DNase activity can be sprayed onto the item. For example the polypeptide having DNase activity can be sprayed into the interior of a washing machine for textile or a dishwashing machine or can be sprayed onto a hard surface in kitchen or bathroom areas.

In one embodiment of the invention, the item is contacted to a liquid solution comprising a polypeptide having DNase activity. The liquid solution can further comprise detergent components such as a surfactant. The liquid solution may be wash liquor for laundering or hard surface cleaning.

All the interior part of laundry washing machines or dishwashing machines can be contacted with the polypeptide having DNase activity. All parts of the machines including parts made of metal, glass, rubber, plastic, PVC, acrylics, ceramics, china or porcelain can be contacted with the polypeptide and the polypeptide will prevent, reduce or remove biofilm and malodor from the item. The metal items can be made of iron, copper, magnesium, chromium, nickel, aluminium, titanium, lead, gold, silver or an alloy thereof. In one embodiment the item is made of stainless steel. The dishwashing machines or washing machines may also contain parts made of rubber, such as natural rubber or synthetic rubber. Hard surfaces in kitchen or bathroom areas can also be made of materials such as metal, glass, rubber, plastic, PVC, acrylics, ceramics, china or porcelain.

In one embodiment of the invention, the polypeptide having DNase activity is used in industrial or institutional ware washing. The term ware washing is a term generally used in industries and institutions and it means dishwashing of dishware.

Industrial and institutional ware washing is a process applied in an industrial, commercial or institutional situation to provide clean and hygienic ware in as short a timeframe as possible. To achieve this result, ware washers generally apply high temperatures and strong mechanical and chemical action during the washing process. Given the broad range of potential applications of ware washers, there is a large variety of systems available. These include single wash undercounter systems (similar to household dishwashers), hooded single use systems, systems for larger or heavily soiled equipment and large conveyor or flight machines that operate continuously. Ware washers commonly contain a sump or reservoir of washing water. The purpose of this sump is to reduce water and ware wash chemical consumption by allowing the re-use and re-circulation of the water over a period of time or washes.

In the majority of ware wash applications, the time available for washing is limited due to capacity constraints. Generally, a wash cycle is between 50-90 seconds, but can be up to 10 minutes. In order to overcome these time constraints and deliver clean and hygienic ware, ware washers generally apply a high level of mechanical action to the ware. This is generally done using high pressure water distributed through nozzles and that is re-circulated in the ware washer. In some cases, an abrasive element can be introduced to the system (for example polymer beads) to enhance the mechanical effect of the water on the soiled ware. Despite the high degree of mechanical action applied in ware washing processes, a strong chemical action is relied upon to deliver the required levels of cleanliness and if required, hygiene. Ware wash chemicals are characterized by generally being highly alkaline and containing other elements to enhance the cleaning performance to ensure a satisfactory result, and to protect the ware wash machine from the potentially corrosive alkaline chemicals.

During operation of ware wash processes the inner surfaces of the warewash machine are exposed to water containing potentially high levels of organic soils, and over time a soil film or deposit can form on the inner surfaces of the ware wash machine. This film can potentially be resistant to removal during normal daily cleaning operations. In ware wash processes where a soil film is present on the inner surfaces of the machine, it is not uncommon to see reduced

performance, increase chemical dosing requirements, malodours and the formation of biofilms within the machine.

In order to protect the metal parts of machines or other hard surfaces, the polypeptide having DNase activity may be used together with a metal care agent. The invention further concerns a detergent composition comprising a polypeptide having DNase activity and a metal care agent.

The metal care agent may be selected from the group consisting of:

a) benzatriazoles, including benzotriazole or bis-benzotriazole and substituted derivatives thereof, which derivatives include substituents with linear or branch-chain Ci-C20- alkyl groups and hydroxyl, thio, phenyl or halogen such as fluorine, chlorine, bromine and iodine.

b) metal salts and complexes chosen from the group consisting of zinc, manganese, titanium, zirconium, hafnium, vanadium, cobalt, gallium and cerium salts and/or complexes, the metals being in one of the oxidation states II, III, IV, V or VI, such as metal salts and/or metal complexes may be chosen from the group consisting of Mn(II) sulphate, Mn(II) citrate, Mn(II) stearate, Mn(II) acetylacetonate, KTiF6, KZrF6, CoSO4, Co(NOs)2 and Ce(NOs)3, zinc salts, for example zinc sulphate, hydrozincite, zinc acetate or zinc carbonate;

c) silicates, including sodium or potassium silicate, sodium disilicate, sodium metasilicate, crystalline phyllosilicate and mixtures thereof.

The invention further concerns a detergent composition comprising a polypeptide having deoxyribonuclease (DNase) activity and a strong sequestering builder.

A strong builder is classified as high efficiency chelators that can bind the divalent cations such as $Ca^{2+}$ strongly with a logarithmic stability constant of the cation/chelator complex of above 4, particular above 5, above 6 or above 7. The stability constants are determined at an ionic strength of 0.1 M and at a temperature of 25°C.

Strong sequestering builders include for example, such materials as water-soluble tripolyphosphate, ethylene diamine tetraacetate, and organic phosphonates. Alkali metal pyrophosphates are also classed as strong sequestering builders. Strong sequestering builder are phosphorus-containing builder or non-phosphorus builder. In the present invention both phosphorus and non-phosphorus builders can be used. The non-phosphorus builders builders are preferred because they are better for the environment.

A phosphorus-containing builder generally comprises an inorganic phosphate or a phosphonate, typically an alkali metal salt such as sodium or potassium.

The inorganic phosphate may be a diphosphate, a triphosphate, a tripolyphosphate or pyrophosphate. Specific examples of inorganic phosphates include $Na_5P_3O_{10}$ (STPP or sodium tripolyphosphate) and $Na_4P_2O_7$ (tetrasodium pyrophosphate).

The phosphonate may be an alkyl phosphonate, an aryl phosphonate or an alkaryl phosphonate, wherein the alkyl, aryl or alkaryl group may be substituted. Examples of phosphonates include 1-Hydroxy Ethylidene-1,1-Diphosphonic Acid (HEDP, etidronic acid), Diethylenetriamine Penta(Methylene Phosphonic acid) (DTPMP), Ethylene diamine tetra(methylene phosphonic acid) (EDTMPA), amino tris(methylenephosphonic acid) (ATMP), Nitrilo trimethylene phosphonic acid (NTMP),2-Amino ethyl phosphonic acid (AEPn), Dimethyl methylphosphonate (DMMP),Tetramethylene diamine tetra(methylene phosphonic acid) (TDTMP), Hexamethylene diamine tetra(methylene phosphonic acid) (HDTMP), Phosphonobutane-tricarboxylic acid (PBTC), N-(phosphonomethyl) iminodiacetic acid (PMIDA), 2-carboxyethyl phosphonic acid (CEPA), 2-Hydroxy phosphonocarboxylic acid (HPAA).

A non-phosphorus strong sequestering builder may include for example Ethylenediaminetetraacetic acid (EDTA), methylglycinediacetic acid (MGDA), Nitrilotriacetic acid (NTA), iminodisuccinic acid (IDS), ethylenediaminedisuccinic acid (EDDS), and L-glutamic acid N,N-diacetic acid tetra sodium salt (GLDA).

Examples of stability constants on the builder calcium complex and content of phosphate are listed below:

| Builder | Builder type | Phosphorus | Log $K_{Ca}$ |
|---|---|---|---|
| **Strong sequestering builders** | | | |
| EDTA | Sequestering | no | 10.7 |
| EDTMP | Sequestering | yes | 10.0 |
| NTMP | Sequestering | yes | 7.6 |
| DTPMP | Sequestering | yes | 7.1 |
| MGDA | Sequestering | no | 7 |
| NTA | Sequestering | no | 6.4 |
| HEDP | Sequestering | yes | 6 |

(continued)

| Builder | Builder type | Phosphorus | Log $K_{Ca}$ |
|---|---|---|---|
| **Strong sequestering builders** | | | |
| STPP | Sequestering | yes | 5.36 |
| IDS | Sequestering | no | 5.2 |
| GLDA | Sequestering | no | 5.2 |
| Pyrophosphate | Sequestering | yes | 5 |
| EDDS | Sequestering | no | 4.6 |
| **Other builder** | | | |
| Carbonate | Precipitating | no | 7.8 |
| Citric acid | Sequestering | no | 3.5 |
| AMP | Sequestering | yes | 1.7 |

The concentration of the strong sequestering builder in the detergent composition can be from 0.5% (w/w) to 80 % of the strong sequestering builder, such as in the range of 1.0-75%, in the range of 1-70%, in the range of 1-65%, in the range of 1-60%, in the range of 1-55%, in the range of 1-50%, in the range of 1-45%, in the range of 1-40%, in the range of 1-35%, in the range of 1-30% or in the range of 1-25%. When washing with an automated dishwasher the detergent composition is released in main wash.

The concentration of the strong sequestering builder in the wash liquor can be from 0.01 to 5.0 gram of the strong sequestering builder/liter of wash liquor (g/L), such as in the range of 0.01-4.0 g/L, in the range of 0.01-3.0 g/L, in the range of 0.01-2.8 g/L, in the range of 0.01-2.6 g/L, in the range of 0.01-2.4 g/L, in the range of 0.01-2.2 g/L, in the range of 0.01-2.0 g/L, in the range of 0.01-1.8 g/L, in the range of 0.01-1.6 g/L, in the range of 0.01-1.4 g/L, in the range of 0.01-1.2 g/L or in the range of 0.01-1.0 g/L.

The concentration of GLDA in the detergent composition can be from 0.5% (w/w) to 80 % of the strong sequestering builder, such as in the range of 1.0-75%, in the range of 1-70%, in the range of 1-65%, in the range of 1-60%, in the range of 1-55%, in the range of 1-50%, in the range of 1-45%, in the range of 1-40%, in the range of 1-35%, in the range of 1-30% or in the range of 1-25%.

The concentration of MGDA in the detergent composition can be from 0.5% (w/w) to 50 % of the strong sequestering builder, such as in the range of 1-45%, in the range of 1-40%, in the range of 1-35%, in the range of 1-30% or in the range of 1-25%.

The concentration of carbonate, such as sodium carbonate in the detergent composition can be from 0.5% (w/w) to 26% of the strong sequestering builder, such as in the range of 1.0-20%, in the range of 1-70%, in the range of 1-15%, in the range of 1-10%, in the range of 1-5% or in the range of 1-3%.

The concentration of citrate, such as sodium citrate in the detergent composition can be from 0.5% (w/w) to 50 % of the strong sequestering builder, such as in the range of 1-45%, in the range of 1-40%, in the range of 1-35%, in the range of 1-30% or in the range of 1-25%.

The concentration of STTP in the detergent composition can be from 0.5% (w/w) to 50 % of the strong sequestering builder, such as in the range of 1-45%, in the range of 1-40%, in the range of 1-35%, in the range of 1-30% or in the range of 1-25%.

In addition to the strong sequestering builder, the detergent composition may optionally comprise one or more other builders, e.g.a weak builder or a precipitating builder. Precipitating builders are materials such as carbonates, bicarbonates, sesquicarbonates, silicates, aluminates, oxylates, and fatty acids, particularly as an alkali metal salt such as sodium or potassium.

In one embodiment of the invention, the builder used in the detergent composition can be selected from the group consisting of sodium citrate, citric acid, alcanol amines such as Mono- di- or Triethanol amine (MEA, DEA or TEA), sodium carbonate (precipitating, log $K_{Ca}$ = 7.8), sodium bicarbonate and Amino-tris-(methylene-phosphonic acid) (AMP).

The present detergent composition ensures that hard surfaces appear clean and attractive after being washed and no malodour is present on the cleaned surfaces. In addition, when the consumer is satisfied with the result of the automatic washing process with the present ADW detergent composition and with the fact that no unpleasant smell is released from the clean dishware or the interior of the dishwasher, the consumer do not tend to overdose the ADW detergent composition in order to improve the cleaning result. This has a positive influence on the local environment where the drained wash liquor is released.

The detergent composition may further comprise other detergent components such as surfactants, builders, flocculating aid, chelating agents, dye transfer inhibitors, enzymes, enzyme stabilizers, enzyme inhibitors, catalytic materials, bleach activators, hydrogen peroxide, sources of hydrogen peroxide, preformed peracids, polymeric dispersing agents, clay soil removal/anti-redeposition agents, brighteners, suds suppressors, dyes, perfumes, structure elasticizing agents, fabric softeners, carriers, hydrotropes, builders and co-builders, fabric hueing agents, anti-foaming agents, dispersants, processing aids, bacteriocides, fungicides and/or pigments and combinations thereof. In one embodiment, the detergent composition comprises a surfactant. In one embodiment the detergent composition comprises a builder. In one embodiment the detergent composition comprises a clay soil removal/anti-redeposition agent.

In order to enhance the cleaning of hard surfaces e.g. dishware, the detergent composition may further comprise one or more enzymes selected from the group consisting of proteases, lipases, cutinases, amylases, carbohydrases, cellulases, pectinases, mannanases, arabinases, galactanases, xylanases, peroxidases and oxidases.

The detergent composition may be used for preventing, reducing or removing biofilm from a surface. The surface can be a hard surface e.g. a dishware.

In one embodiment, the composition is a bar, a homogenous tablet, a tablet having two or more layers, a pouch having one or more compartments, a regular or compact powder, a granule, a paste, a gel, or a regular, compact or concentrated liquid. The composition may be a a liquid detergent, a powder detergent or a granule detergent.

The invention further concerns a liquid detergent composition comprising a surfactant and a detergent and a detergent builder in a total concentration of at least 3% by weight, and a detergent enzyme containing microcapsule, wherein the membrane of the microcapsule is produced by cross-linking of a polybranched polyamine having a molecular weight of more than 1 kDa. The inventors have found, that encapsulating enzymes in a microcapsule with a semipermeable membrane of the invention, and having a water activity inside these capsules (prior to addition to the liquid detergent) higher than in the liquid detergent, the capsules will undergo a (partly) collapse when added to the detergent (water is oozing out), thus leaving a more concentrated and more viscous enzyme containing interior in the capsules. The collapse of the membrane may also result in a reduced permeability. This can be further utilized by addition of stabilizers/polymers, especially ones that are not permeable through the membrane. The collapse and resulting increase in viscosity will reduce/hinder the diffusion of hostile components (e.g., surfactants or sequestrants) into the capsules, and thus increase the storage stability of the enzyme in the liquid detergent. Components in the liquid detergent that are sensitive to the enzyme (e.g., components that act as substrate for the enzyme) are also protected against degradation by the enzyme. During wash the liquid detergent is diluted by water, thus increasing the water activity. Water will now diffuse into the capsules (osmosis). The capsules will swell and the membrane will either become permeable to the enzyme so they can leave the capsules, or simply burst and in this way releasing the enzyme. The concept is very efficient in stabilizing the enzymes against hostile components in liquid detergent, and vice versa also protects enzyme sensitive components in the liquid detergent from enzymes.

Examples of detergent components which are sensitive to, and can be degraded by, enzymes include (relevant enzyme in parenthesis): xanthan gum (xanthanase), polymers with ester bonds (lipase), hydrogenated castor oil (lipase), perfume (lipase), methyl ester sulfonate surfactants (lipase), cellulose and cellulose derivatives (e.g. CMC) (cellulase), and dextrin and cyclodextrin (amylase).

Also sensitive detergent ingredients can be encapsulated, and thus stabilized, in the microcapsules of the invention. Sensitive detergent ingredients are prone to degradation during storage. Such detergent ingredients include bleaching compounds, bleach activators, perfumes, polymers, builder, surfactants, etc.

Generally, the microcapsules of the invention can be used to separate incompatible components/compounds in detergents.

Addition of the microcapsules to detergents can be used to influence the visual appearance of the detergent product, such as an opacifying effect (small microcapsules) or an effect of distinctly visible particles (large microcapsules). The microcapsules may also be colored.

The microcapsules can be used to reduce the enzyme dust levels during handling and processing of enzyme products.

Unless otherwise indicated, all percentages are indicated as percent by weight (% w/w) throughout the application.

Microcapsule: The microcapsules are typically produced by forming water droplets into a continuum that is non-miscible with water - i.e., typically by preparing a water-in-oil emulsion - and subsequently formation of the membrane by interfacial polymerization via addition of a cross-linking agent. After eventual curing the capsules can be harvested and further rinsed and formulated by methods known in the art. The capsule formulation is subsequently added to the detergent.

The payload, the major membrane constituents and eventual additional component that are to be encapsulated are found in the water phase. In the continuum is found components that stabilize the water droplets towards coalescence (emulsifiers, emulsion stabilizers, surfactants etc.) and the cross linking agent is also added via the continuum.

The emulsion can be prepared be any methods known in the art, e.g., by mechanical agitation, dripping processes, membrane emulsification, microfluidics, sonication etc. In some cases simple mixing of the phases automatically will result in an emulsion, often referred to as self-emulsification. Using methods resulting in a narrow size distribution is an advantage.

The cross-linking agent(s) is typically subsequently added to the emulsion, either directly or more typically by preparing a solution of the crosslinking agent in a solvent which is soluble in the continuous phase. The emulsion and cross-linking agent or solution hereof can be mixed by conventional methods used in the art, e.g., by simple mixing or by carefully controlling the flows of the emulsion and the cross-linking agent solution through an in-line mixer.

In some cases, curing of the capsules is needed to complete the membrane formation. Curing is often simple stirring of the capsules for some time to allow the interfacial polymerization reaction to end. In other cases the membrane formation can be stopped by addition of reaction quencher.

The capsules may be post modified, e.g., by reacting components onto the membrane to hinder or reduce flocculation of the particles in the detergent as described in WO 99/01534.

The produced capsules can be isolated or concentrated by methods known in the art, e.g., by filtration, centrifugation, distillation or decantation of the capsule dispersion.

The resulting capsules can be further formulated, e.g., by addition of surfactants to give the product the desired properties for storage, transport and later handling and addition to the detergent. Other microcapsule formulation agents include rheology modifiers, biocides (e.g., Proxel), acid/base for adjustment of pH (which will also adjust inside the microcapsules), and water for adjustment of water activity.

The capsule forming process may include the following steps:

- Preparation of the initial water and oil phase(s),
- Forming a water-in-oil emulsion,
- Membrane formation by interfacial polymerization,
- Optional post modification,
- Optional isolation and/or formulation,
- Addition to detergent.

The process can be either a batch process or a continuous or semi-continuous process.

A microcapsule according to the invention is a small aqueous sphere with a uniform membrane around it. The material inside the microcapsule is referred to as the core, internal phase, or fill, whereas the membrane is sometimes called a shell, coating, or wall. The microcapsules of the invention have diameters between 0.5 μm and 2 millimeters. Preferably, the mean diameter of the microcapsules is in the range of 1 μm to 1000 μm, more preferably in the range of 5 μm to 500 μm, even more preferably in the range of 10 μm to 500 μm, even more preferably in the range of 50 μm to 500 μm, and most preferably in the range of 50 μm to 200 μm. Alternatively, the diameter of the microcapsules is in the range of 0.5 μm to 30 μm; or in the range of 1 μm to 25 μm. The diameter of the microcapsule is measured in the oil phase after polymerization is complete. The diameter of the capsule may change depending on the water activity of the surrounding chemical environment.

Microencapsulation of enzymes, as used in the present invention, may be carried out by interfacial polymerization, wherein the two reactants in a polymerization reaction meet at an interface and react rapidly. The basis of this method is a reaction of a polyamine with an acid derivative, usually an acid halide, acting as a crosslinking agent. The polyamine is preferably substantially water-soluble (when in free base form). Under the right conditions, thin flexible membranes form rapidly at the interface. One way of carrying out the polymerization is to use an aqueous solution of the enzyme and the polyamine, which are emulsified with a non-aqueous solvent (and an emulsifier), and a solution containing the acid derivative is added. An alkaline agent may be present in the enzyme solution to neutralize the acid formed during the reaction. Polymer (polyamide) membranes form instantly at the interface of the emulsion droplets. The polymer membrane of the microcapsule is typically of a cationic nature, and thus bind/complex with compounds of an anionic nature.

The diameter of the microcapsules is determined by the size of the emulsion droplets, which is controlled, for example by the stirring rate.

Emulsion: An emulsion is a temporary or permanent dispersion of one liquid phase within a second liquid phase. The second liquid is generally referred to as the continuous phase. Surfactants are commonly used to aid in the formation and stabilization of emulsions. Not all surfactants are equally able to stabilize an emulsion. The type and amount of a surfactant needs to be selected for optimum emulsion utility especially with regard to preparation and physical stability of the emulsion, and stability during dilution and further processing. Physical stability refers to maintaining an emulsion in a dispersion form. Processes such as coalescence, aggregation, adsorption to container walls, sedimentation and creaming, are forms of physical instability, and should be avoided. Examples of suitable surfactants are described in WO 97/24177, page 19-21; and in WO 99/01534.

Emulsions can be further classified as either simple emulsions, wherein the dispersed liquid phase is a simple

homogeneous liquid, or a more complex emulsion, wherein the dispersed liquid phase is a heterogeneous combination of liquid or solid phases, such as a double emulsion or a multiple-emulsion. For example, a water-in-oil double emulsion or multiple emulsion may be formed wherein the water phase itself further contains an emulsified oil phase; this type of emulsion may be specified as an oil-in-water-in oil (o/w/o) emulsion. Alternatively, a water-in-oil emulsion may be formed wherein the water phase contains a dispersed solid phase often referred to as a suspension- emulsion. Other more complex emulsions can be described. Because of the inherent difficulty in describing such systems, the term emulsion is used to describe both simple and more complex emulsions without necessarily limiting the form of the emulsion or the type and number of phases present

Polyamine: The rigidity/flexibility and permeability of the membrane is mainly influenced by the choice of polyamine. The polyamine according to the invention is a polybranched polyamine. Each branch, preferably ending with a primary amino group serves as a tethering point in the membrane network, thereby giving the favorable properties of the invention. A polybranched polyamine according to the present invention is a polyamine having more than two branching points and more than two reactive amino groups (capable of reacting with the crosslinking agent, i.e., primary and secondary amino groups). The polybranched polyamine is used as starting material when the emulsion is prepared - it is not formed in situ from other starting materials. To obtain the attractive properties of the invention, the polybranched structure of the polyamine must be present as starting material.

There is a close relation between number of branching points and number of primary amines, since primary amines will always be positioned at the end of a branch: A linear amine can only contain two primary amines. For each branching point hypothetically introduced in such a linear di-amine will allow one or more primary amine(s) to be introduced at the end of the introduced branch(es). In this context we understand the primary amino group as part of the branch, i.e., the endpoint of the branch. For example, we consider both tris(2-aminoethyl)amine and 1,2,3-propanetriamine as molecules having one branching point. For the invention the polyamine has at least four primary amines. Branching points can be introduced from an aliphatic hydrocarbon chain as in the previously stated examples or from unsaturated carbon bonds, such as in, e.g., 3,3'-diaminobenzidine, or from tertiary amino groups, such as in N,N,N',N'-tetrakis-(2-aminoethyl)ethylenediamine.

In addition to the number of branching points, we have found that the compactness of the reactive amino groups is of high importance. A substance such as, e.g., N,N,N',N'-tetrakis-(12-aminododecyl)ethylenediamine would not be suitable. Neither would a peptide or protein, such as an enzyme, be suitable for membrane formation. Thus, the polybranched polyamine is not a peptide or protein.

In an embodiment, the reactive amino groups constitute at least 15% of the molecular weight of the polybranched polyamine, such as more than 20%, or more than 25%. Preferably, the molecular weight of the polybranched polyamine is at least 1 kDa; more preferably, the molecular weight of the polybranched polyamine is at least 1.3 kDa.

In a preferred embodiment, the polybranched polyamine is a polyethyleneimine (PEI), and modifications thereof, having more than two branching points and more than two reactive amino groups; wherein the reactive amino groups constitute at least 15% of the molecular weight of the PEI, such as more than 20%, or more than 25%. Preferably, the molecular weight of the PEI is at least 1 kDa.

Combinations of different polybranched polyamines may be used for preparing the microcapsule according to the invention.

The advantageous properties (e.g., enzyme storage stability, reduced enzyme leakage, reduced in-flux of detergent ingredients) of the microcapsule of the invention may be improved by adding one or more small amines with a molecular weight of less than 1 kDa. The small amine is preferably substantially water-soluble (when in free base form) and can be a material such as ethylene diamine, hexamethylene diamine, hexane diamine, diethylene tetramine, ethylene tetramine, diamino benzene, piperazine, tetramethylene pentamine or, preferably, diethylene triamine (DETA). The small amines may be added in an amount of up to 50%, preferably up to 40%, up to 30%, up to 20%, up to 10%, or up to 5%, by weight of the total content of small amine and polybranched polyamine, when preparing the microcapsule of the invention.

Crosslinking agent: The crosslinking agent as used in the present invention is a molecule with at least two groups/sites capable of reacting with amines to form covalent bonds.

The crosslinking agent is preferably oil soluble and can be in the form of an acid anhydride or acid halide, preferably an acid chloride. For example, it can be adipoyl chloride, sebacoyl chloride, dodecanedioc acid chloride, phthaloyl chloride, terephthaloyl chloride, isophthaloyl chloride, or trimesoyl chloride; but preferably, the crosslinking agent is terephthaloyl chloride or trimesoyl chloride.

The invention further concerns a cleaning method for preventing, reducing or removing a biofilm from an item comprising the steps of:

a) contacting an item to a composition according to the invention or to a liquid solution comprising a polypeptide having DNase activity;
b) completing at least one cleaning cycle; and

c) optionally rinsing the item,

wherein the item is a hard surface.

In one embodiment, the is a hard surface, such as the interior surface of a dishwashing machine or a washing machine. The interior surface of a dishwashing machine or washing machine may comprise soap intake box, walls, windows, baskets, racks, nozzles, pumps, sump, filters, pipelines, tubes, joints, seals, gaskets, fittings, impellers, drums, drains, traps, coin traps inlet and outlets. The interior surfaces in a dishwashing machine or dishwashing machine can be made of various materials such as metal, glass, rubber, plastic, PVC, acrylics, ceramics, china or porcelain.

In one embodiment of the invention, the a dishware, such as plates, cups, glasses, bowls, pots, cutlery, spoons, knives, forks, serving utensils, ceramics, plastics, cutting boards, china and glass ware. In one embodiment, the dishware is cleaned simultaneously with the cleaning of the hard surface, for example the interior of the dishwashing machine is cleaned at the same time as the dishware is washed or cleaned. Or the interior of a washing machine is cleaned at the same time as the laundry items are washed.

The liquid solution used in the method can further comprise antistatic agents, surfactants, builders, flocculating aid, chelating agents, dye transfer inhibitors, enzymes, enzyme stabilizers, enzyme inhibitors, catalytic materials, bleach activators, hydrogen peroxide, sources of hydrogen peroxide, preformed peracids, polymeric dispersing agents, clay soil removal/anti-redeposition agents, brighteners, suds suppressors, dyes, perfumes, structure elasticizing agents, fabric softeners, carriers, hydrotropes, builders and co-builders, fabric hueing agents, anti-foaming agents, dispersants, processing aids, bacteriocides, fungicides and/or pigments or combinations thereof.

In one embodiment, the liquid solution further comprises one or more enzymes selected from the group consisting of proteases, lipases, cutinases, amylases, carbohydrases, cellulases, pectinases, mannanases, arabinases, galactanases, xylanases, peroxidases and oxidases.

The pH of the liquid solution is in the range of 1 to 11, such as in the range of 5.5 to 11, such as in the range of 7 to 9, in the range of 7 to 8 or in the range of 7 to 8.5.

The temperature of the liquid solution can be in the range of 5°C to 95°C, or in the range of 10°C to 80°C, in the range of 10°C to 70°C, in the range of 10°C to 60°C, in the range of 10°C to 50°C, in the range of 15°C to 40°C or in the range of 20°C to 30°C. In one embodiment, the temperature of the liquid solution is 30°C.

In one embodiment, the item is rinsed after being contacted with to the liquid solution or the composition. The item can be rinsed with water or with water comprising a conditioner.

The polypeptide having DNase activity can be of animal, vegetable, microbial origin. In one embodiment the polypeptide is of human origin. In one embodiment the polypeptide is obtained from plant material such as mung bean. In one embodiment the polypeptide is of bacterial or fungal origin.

A polypeptide of fungal origin may be selected from the group consisting of:

a. a polypeptide having at least 60% sequence identity to the mature polypeptide of SEQ ID NO: 2, a polypeptide having at least 60% sequence identity to the mature polypeptide of SEQ ID NO: 3 or a polypeptide having at least 60% sequence identity to the mature polypeptide of SEQ ID NO: 5

b. a polypeptide encoded by a polynucleotide that hybridizes under low stringency conditions with

   i. the mature polypeptide coding sequence of SEQ ID NO: 1 or the mature polypeptide coding sequence of SEQ ID NO: 4

   ii. the cDNA sequence thereof, or

   iii. the full-length complement of (i) or (ii);

c. a polypeptide encoded by a polynucleotide having at least 60% sequence identity to the mature polypeptide coding sequence of SEQ ID NO: 1 or the cDNA sequence thereof or a polypeptide encoded by a polynucleotide having at least 60% sequence identity to the mature polypeptide coding sequence of SEQ ID NO: 4 or the cDNA sequence thereof;

d. a variant of the mature polypeptide of SEQ ID NO: 2 comprising a substitution, deletion, and/or insertion at one or more positions, a variant of the mature polypeptide of SEQ ID NO: 3 comprising a substitution, deletion, and/or insertion at one or more positions or a variant of the mature polypeptide of SEQ ID NO: 5 comprising a substitution, deletion, and/or insertion at one or more positions; and

e. a fragment of the polypeptide of (a), (b), (c), or (d) that has DNase activity.

European patent application number 14164424.5 discloses in examples 1 to 3 how the polypeptide of SEQ ID NO: 2 and SEQ ID NO: 3 are produced. European patent application number 14164429.4 discloses in examples 1 to 2 how the polypeptide of SEQ ID NO: 5 is produced.

A polypeptide of bacterial origin may be selected from the group consisting of:

a. a polypeptide having at least 60% sequence identity to the mature polypeptide of SEQ ID NO: 6 or a polypeptide having at least 60% sequence identity to the mature polypeptide of SEQ ID NO: 7;
b. a variant of the mature polypeptide of SEQ ID NO: 6 comprising a substitution, deletion, and/or insertion at one or more positions or a variant of the mature polypeptide of SEQ ID NO: 7 comprising a substitution, deletion, and/or insertion at one or more positions; and
c. a fragment of the polypeptide of (a) or (b) that has DNase activity;

The polypeptide can have at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% sequence identity to the mature polypeptide of SEQ ID NO: 2, to the mature polypeptide of SEQ ID NO: 3, or to the mature polypeptide of SEQ ID NO: 5, or to the mature polypeptide of SEQ ID NO: 6 or to the mature polypeptide of SEQ ID NO: 7.

International patent application published under number WO2011098579 discloses in example 3 how to clone and express the polypeptide of SEQ ID NO: 6.

The polypeptide can comprise or consist of SEQ ID NO: 2 or the mature polypeptide of SEQ ID NO: 2, the polypeptide comprises or consists of SEQ ID NO: 3 or the mature polypeptide of SEQ ID NO: 3, the polypeptide comprises or consists of SEQ ID NO: 5 or the mature polypeptide of SEQ ID NO: 5, the polypeptide comprises or consists of SEQ ID NO: 6 or the mature polypeptide of SEQ ID NO: 6 or the polypeptide comprises or consists of SEQ ID NO: 7 or the mature polypeptide of SEQ ID NO: 7.

The mature polypeptide can comprise amino acids 1 to 206 of SEQ ID NO: 2, amino acids 1 to 206 of SEQ ID NO: 3, amino acids 1 to 188 of SEQ ID NO: 5, amino acids 1 to 110 of SEQ ID NO: 6 or amino acids 1 to 109 of SEQ ID NO: 7

The polypeptide can be a variant of the mature polypeptide of SEQ ID NO: 2, SEQ ID NO: 3, SEQ ID NO: 5, SEQ ID NO: 6 or SEQ ID NO: 7, wherein the variant comprises a substitution, deletion, and/or insertion at one or more positions or a variant of the mature polypeptide of SEQ ID NO: 2, SEQ ID NO: 3, SEQ ID NO: 5, SEQ ID NO: 6 or SEQ ID NO: 7 which comprises a substitution, deletion, and/or insertion at one or more positions.

The polypeptide can be a fragment of of SEQ ID NO: 2, SEQ ID NO: 3, SEQ ID NO: 5, SEQ ID NO: 6 or SEQ ID NO: 7, wherein the fragment has DNase activity.

The polypeptide having DNase activity can be obtained from from *Aspergillus,* for example from *Aspergillus oryzae.*

In an embodiment, the present invention relates to polypeptides having a sequence identity to the mature polypeptide of SEQ ID NO: 2 of at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%, which have DNase activity. In one aspect, the polypeptides differ by up to 10 amino acids, e.g., 1, 2, 3, 4, 5, 6, 7, 8, 9, or 10, from the mature polypeptide of SEQ ID NO: 2.

In an embodiment, the present invention relates to polypeptides having a sequence identity to the mature polypeptide of SEQ ID NO: 3 of at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%, which have DNase activity. In one aspect, the polypeptides differ by up to 10 amino acids, e.g., 1, 2, 3, 4, 5, 6, 7, 8, 9, or 10, from the mature polypeptide of SEQ ID NO: 3.

In another embodiment, the present invention relates to an polypeptide having DNase activity encoded by a polynucleotide having a sequence identity to the mature polypeptide coding sequence of SEQ ID NO: 2, SEQ ID NO: 5 or the cDNA sequence thereof of at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% and wherein the polypeptide is used for preventing, reducing or removing static electricity from an item.

In an embodiment, the polypeptide has been isolated. A polypeptide of the present invention preferably comprises or consists of the amino acid sequence of SEQ ID NO: 2 or an allelic variant thereof; or is a fragment thereof having DNase activity. In another aspect, the polypeptide comprises or consists of the mature polypeptide of SEQ ID NO: 2. In another aspect, the polypeptide comprises or consists of amino acids 1 to 206 of SEQ ID NO: 2.

In an embodiment, the polypeptide has been isolated. A polypeptide of the present invention preferably comprises or consists of the amino acid sequence of SEQ ID NO: 3 or an allelic variant thereof; or is a fragment thereof having DNase activity. In another aspect, the polypeptide comprises or consists of the mature polypeptide of SEQ ID NO: 3. In another aspect, the polypeptide comprises or consists of amino acids 1 to 206 of SEQ ID NO: 3.

In another embodiment, the present invention relates to an isolated polypeptide having DNase activity encoded by a polynucleotide that hybridizes under low stringency conditions with (i) the mature polypeptide coding sequence of SEQ ID NO: 1, (ii) the cDNA sequence thereof, or (iii) the full-length complement of (i) or (ii) (Sambrook et al., 1989, Molecular Cloning, A Laboratory Manual, 2d edition, Cold Spring Harbor, New York). In an embodiment, the polypeptide has been isolated.

In another embodiment, the present invention relates to an polypeptide having DNase activity encoded by a polynucleotide having a sequence identity to the mature polypeptide coding sequence of SEQ ID NO: 1 or the cDNA sequence thereof of at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%. In a further embodiment, the polypeptide has been isolated.

In another embodiment, the present invention relates to an polypeptide having DNase activity encoded by a polynucleotide having a sequence identity to the mature polypeptide coding sequence of SEQ ID NO: 6, SEQ ID NO: 7 or the cDNA sequence thereof of at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% and wherein the polypeptide is used for preventing, reducing or removing static electricity from an item.

In another embodiment, the present invention relates to variants of the mature polypeptide of SEQ ID NO: 2 comprising a substitution, deletion, and/or insertion at one or more (*e.g.,* several) positions. In an embodiment, the number of amino acid substitutions, deletions and/or insertions introduced into the mature polypeptide of SEQ ID NO: 2 is up to 10, *e.g.,* 1, 2, 3, 4, 5, 6, 7, 8, 9, or 10. The amino acid changes may be of a minor nature, that is conservative amino acid substitutions or insertions that do not significantly affect the folding and/or activity of the protein; small deletions, typically of 1-30 amino acids; small amino- or carboxyl-terminal extensions, such as an amino-terminal methionine residue; a small linker peptide of up to 20-25 residues; or a small extension that facilitates purification by changing net charge or another function, such as a poly-histidine tract, an antigenic epitope or a binding domain.

In another embodiment, the present invention relates to variants of the mature polypeptide of SEQ ID NO: 3 comprising a substitution, deletion, and/or insertion at one or more (*e.g.,* several) positions. In an embodiment, the number of amino acid substitutions, deletions and/or insertions introduced into the mature polypeptide of SEQ ID NO: 3 is up to 10, *e.g.,* 1, 2, 3, 4, 5, 6, 7, 8, 9, or 10. The amino acid changes may be of a minor nature, that is conservative amino acid substitutions or insertions that do not significantly affect the folding and/or activity of the protein; small deletions, typically of 1-30 amino acids; small amino- or carboxyl-terminal extensions, such as an amino-terminal methionine residue; a small linker peptide of up to 20-25 residues; or a small extension that facilitates purification by changing net charge or another function, such as a poly-histidine tract, an antigenic epitope or a binding domain.

The polypeptide having DNase activity can also be obtained from from *Trichoderma,* for example from *Trichoderma harzianum.* In an embodiment, the present invention relates to polypeptides having a sequence identity to the mature polypeptide of SEQ ID NO: 5 of at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%, which have DNase activity. In one aspect, the polypeptides differ by up to 10 amino acids, e.g., 1, 2, 3, 4, 5, 6, 7, 8, 9, or 10, from the mature polypeptide of SEQ ID NO: 5.

In an embodiment, the polypeptide has been isolated. A polypeptide of the present invention preferably comprises or consists of the amino acid sequence of SEQ ID NO: 5 or an allelic variant thereof; or is a fragment thereof having DNase activity. In another aspect, the polypeptide comprises or consists of the mature polypeptide of SEQ ID NO: 5. In another aspect, the polypeptide comprises or consists of amino acids 1 to 188 of SEQ ID NO: 5.

In another embodiment, the present invention relates to an isolated polypeptide having DNase activity encoded by a polynucleotide that hybridizes under low stringency conditions with (i) the mature polypeptide coding sequence of SEQ ID NO: 4, (ii) the cDNA sequence thereof, or (iii) the full-length complement of (i) or (ii) (Sambrook et al., 1989, Molecular Cloning, A Laboratory Manual, 2d edition, Cold Spring Harbor, New York). In an embodiment, the polypeptide has been isolated.

The polynucleotide of SEQ ID NO: 1 or SEQ ID NO: 4 or a subsequence thereof, as well as the polypeptide of SEQ ID NO: 5, SEQ ID NO: 2, SEQ ID NO: 3 or a fragment thereof, may be used to design nucleic acid probes to identify and clone DNA encoding polypeptides having DNase activity from strains of different genera or species according to methods well known in the art.

In particular, such probes can be used for hybridization with the genomic DNA or cDNA of a cell of interest, following standard Southern blotting procedures, in order to identify and isolate the corresponding gene therein. Such probes can be considerably shorter than the entire sequence, but should be at least 15, *e.g.,* at least 25, at least 35, or at least 70 nucleotides in length. Preferably, the nucleic acid probe is at least 100 nucleotides in length, *e.g.,* at least 200 nucleotides, at least 300 nucleotides, at least 400 nucleotides, at least 500 nucleotides, at least 600 nucleotides, at least 700 nucleotides, at least 800 nucleotides, or at least 900 nucleotides in length. Both DNA and RNA probes can be used. The probes are typically labeled for detecting the corresponding gene (for example, with $^{32}P$, $^{3}H$, $^{35}S$, biotin, or avidin). Such probes are encompassed by the present invention.

A genomic DNA or cDNA library prepared from such other strains may be screened for DNA that hybridizes with the probes described above and encodes a polypeptide having DNase activity. Genomic or other DNA from such other strains may be separated by agarose or polyacrylamide gel electrophoresis, or other separation techniques. DNA from the libraries or the separated DNA may be transferred to and immobilized on nitrocellulose or other suitable carrier

material. In order to identify a clone or DNA that hybridizes with SEQ ID NO: 4 or a subsequence thereof, the carrier material is used in a Southern blot.

For purposes of the present invention, hybridization indicates that the polynucleotide hybridizes to a labeled nucleic acid probe corresponding to (i) SEQ ID NO: 1 or SEQ ID NO: 4; (ii) the mature polypeptide coding sequence of SEQ ID NO: 1 or SEQ ID NO: 4; (iii) the cDNA sequence thereof; (iv) the full-length complement thereof; or (v) a subsequence thereof; under very low to very high stringency conditions. Molecules to which the nucleic acid probe hybridizes under these conditions can be detected using, for example, X-ray film or any other detection means known in the art.

In another embodiment, the present invention relates to an polypeptide having DNase activity encoded by a polynucleotide having a sequence identity to the mature polypeptide coding sequence of SEQ ID NO: 1 or SEQ ID NO: 4 or the cDNA sequence thereof of at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%. In a further embodiment, the polypeptide has been isolated.

In another embodiment, the present invention relates to variants of the mature polypeptide of SEQ ID NO: 2, SEQ ID NO: 3 or SEQ ID NO: 5 comprising a substitution, deletion, and/or insertion at one or more (*e.g.,* several) positions. In an embodiment, the number of amino acid substitutions, deletions and/or insertions introduced into the mature polypeptide of SEQ ID NO: 2, SEQ ID NO: 3 or SEQ ID NO: 5 is up to 10, *e.g.,* 1, 2, 3, 4, 5, 6, 7, 8, 9, or 10. The amino acid changes may be of a minor nature, that is conservative amino acid substitutions or insertions that do not significantly affect the folding and/or activity of the protein; small deletions, typically of 1-30 amino acids; small amino- or carboxyl-terminal extensions, such as an amino-terminal methionine residue; a small linker peptide of up to 20-25 residues; or a small extension that facilitates purification by changing net charge or another function, such as a poly-histidine tract, an antigenic epitope or a binding domain.

The polypeptide having DNase activity can also be obtained from from *Bacillus,* for example from *Bacillus substilis or Bacillus licheniformis.*

In an embodiment, the present invention relates to polypeptides having a sequence identity to the mature polypeptide of SEQ ID NO: 6 or SEQ ID NO: 7 of at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%, which have DNase activity. In one aspect, the polypeptides differ by up to 10 amino acids, e.g., 1, 2, 3, 4, 5, 6, 7, 8, 9, or 10, from the mature polypeptide of SEQ ID NO: 6 or SEQ ID NO: 7.

In an embodiment, the polypeptide has been isolated. A polypeptide of the present invention preferably comprises or consists of the amino acid sequence of SEQ ID NO: 6, SEQ ID NO: 7 or an allelic variant thereof; or is a fragment thereof having DNase activity. In another aspect, the polypeptide comprises or consists of the mature polypeptide of SEQ ID NO: 6 or SEQ ID NO: 7. In another aspect, the polypeptide comprises or consists of amino acids 1 to 110 of SEQ ID NO: 6 or amino acids 1 to 109 of SEQ ID NO: 7.

In another embodiment, the present invention relates to an polypeptide having DNase activity encoded by a polynucleotide having a sequence identity to the mature polypeptide coding sequence of SEQ ID NO: 6, SEQ ID NO: 7 or the cDNA sequence thereof of at least 60%, *e.g.,* at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%. In a further embodiment, the polypeptide has been isolated.

In another embodiment, the present invention relates to variants of the mature polypeptide of SEQ ID NO: 6 or SEQ ID NO: 7 comprising a substitution, deletion, and/or insertion at one or more (*e.g.,* several) positions. In an embodiment, the number of amino acid substitutions, deletions and/or insertions introduced into the mature polypeptide of SEQ ID NO: 6 or SEQ ID NO: 7 is up to 10, *e.g.,* 1, 2, 3, 4, 5, 6, 7, 8, 9, or 10. The amino acid changes may be of a minor nature, that is conservative amino acid substitutions or insertions that do not significantly affect the folding and/or activity of the protein; small deletions, typically of 1-30 amino acids; small amino- or carboxyl-terminal extensions, such as an amino-terminal methionine residue; a small linker peptide of up to 20-25 residues; or a small extension that facilitates purification by changing net charge or another function, such as a poly-histidine tract, an antigenic epitope or a binding domain.

Examples of conservative substitutions are within the groups of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions that do not generally alter specific activity are known in the art and are described, for example, by H. Neurath and R.L. Hill, 1979, In, The Proteins, Academic Press, New York. Common substitutions are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly.

Alternatively, the amino acid changes are of such a nature that the physico-chemical properties of the polypeptides are altered. For example, amino acid changes may improve the thermal stability of the polypeptide, alter the substrate specificity, change the pH optimum, and the like.

Essential amino acids in a polypeptide can be identified according to procedures known in the art, such as site-directed mutagenesis or alanine-scanning mutagenesis (Cunningham and Wells, 1989, Science 244: 1081-1085). In the latter technique, single alanine mutations are introduced at every residue in the molecule, and the resultant mutant molecules are tested for DNase activity to identify amino acid residues that are critical to the activity of the molecule. See also, Hilton et al., 1996, J. Biol. Chem. 271: 4699-4708. The active site of the enzyme or other biological interaction can also be determined by physical analysis of structure, as determined by such techniques as nuclear magnetic resonance, crystallography, electron diffraction, or photoaffinity labeling, in conjunction with mutation of putative contact site amino acids. See, for example, de Vos et al., 1992, Science 255: 306-312; Smith et al., 1992, J. Mol. Biol. 224: 899-904; Wlodaver et al., 1992, FEBS Lett. 309: 59-64. The identity of essential amino acids can also be inferred from an alignment with a related polypeptide.

Single or multiple amino acid substitutions, deletions, and/or insertions can be made and tested using known methods of mutagenesis, recombination, and/or shuffling, followed by a relevant screening procedure, such as those disclosed by Reidhaar-Olson and Sauer, 1988, Science 241: 53-57; Bowie and Sauer, 1989, Proc. Natl. Acad. Sci. USA 86: 2152-2156; WO 95/17413; or WO 95/22625. Other methods that can be used include error-prone PCR, phage display (e.g., Lowman et al., 1991, Biochemistry 30: 10832-10837; U.S. Patent No. 5,223,409; WO 92/06204), and region-directed mutagenesis (Derbyshire et al., 1986, Gene 46: 145; Ner et al., 1988, DNA 7: 127).

Mutagenesis/shuffling methods can be combined with high-throughput, automated screening methods to detect activity of cloned, mutagenized polypeptides expressed by host cells (Ness et al., 1999, Nature Biotechnology 17: 893-896). Mutagenized DNA molecules that encode active polypeptides can be recovered from the host cells and rapidly sequenced using standard methods in the art. These methods allow the rapid determination of the importance of individual amino acid residues in a polypeptide.

The polypeptide may be a hybrid polypeptide in which a region of one polypeptide is fused at the N-terminus or the C-terminus of a region of another polypeptide.

The polypeptide may be a fusion polypeptide or cleavable fusion polypeptide in which another polypeptide is fused at the N-terminus or the C-terminus of the polypeptide of the present invention. A fusion polypeptide is produced by fusing a polynucleotide encoding another polypeptide to a polynucleotide of the present invention. Techniques for producing fusion polypeptides are known in the art, and include ligating the coding sequences encoding the polypeptides so that they are in frame and that expression of the fusion polypeptide is under control of the same promoter(s) and terminator. Fusion polypeptides may also be constructed using intein technology in which fusion polypeptides are created post-translationally (Cooper et al., 1993, EMBO J. 12: 2575-2583; Dawson et al., 1994, Science 266: 776-779).

A fusion polypeptide can further comprise a cleavage site between the two polypeptides. Upon secretion of the fusion protein, the site is cleaved releasing the two polypeptides. Examples of cleavage sites include, but are not limited to, the sites disclosed in Martin et al., 2003, J. Ind. Microbiol. Biotechnol. 3: 568-576; Svetina et al., 2000, J. Biotechnol. 76: 245-251; Rasmussen-Wilson et al., 1997, Appl. Environ. Microbiol. 63: 3488-3493; Ward et al., 1995, Biotechnology 13: 498-503; and Contreras et al., 1991, Biotechnology 9: 378-381; Eaton et al., 1986, Biochemistry 25: 505-512; Collins-Racie et al., 1995, Biotechnology 13: 982-987; Carter et al., 1989, Proteins: Structure, Function, and Genetics 6: 240-248; and Stevens, 2003, Drug Discovery World 4: 35-48.

**Enzyme of the invention - Deoxyribonuclease (DNase)**

A polypeptide having DNase activity or a deoxyribonuclease (DNase) is any enzyme that catalyzes the hydrolytic cleavage of phosphodiester linkages in the DNA backbone, thus degrading DNA. The two terms polypeptide having DNase activity and DNase are used interchangeably.

According to the present invention, a DNase which is obtainable from a fungus is preferred; in particular a DNase which is obtainable from a *Aspergillus* is preferred; in particular a DNase which is obtainable from *Aspergillus oryzae* is preferred.. In one embodiment of the present invention, the polypeptide having deoxyribonuclease activity is not the S1 nuclease from *Aspergillus oryzae.*

The DNase used in the present invention includes the mature polypeptide of SEQ ID NO: 2, shown as amino acids 1 to 206 of SEQ ID NO: 2, which is obtained from *Aspergillus oryzae.* The polypeptide having DNase activity can be obtained from *Aspergillus,* for example from *Aspergillus oryzae.* In one embodiment of the invention the polypeptide having DNase activity is the claimed polypeptide.

One aspect of the present invention relates to isolated polypeptides having a sequence identity to the mature polypeptide of SEQ ID NO: 2 of at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%, which have DNase activity. In one aspect, the polypeptides differ by up to 10 amino acids, e.g., 1, 2, 3, 4, 5, 6, 7, 8, 9, or 10, from the mature polypeptide of SEQ ID NO: 2.

In an embodiment, the present invention relates to isolated polypeptides having a sequence identity to the mature polypeptide of SEQ ID NO: 3 of at least 60%, e.g., at least 65%, at least 70%, at least 75%, at least 80%, at

least 85%, at least 90%, at least 91%, at least 92%, at least 93%, at least 94%, at least 95%, at least 96%, at least 97%, at least 98%, at least 99%, or 100%, which have DNase activity. In one aspect, the polypeptides differ by up to 10 amino acids, e.g., 1, 2, 3, 4, 5, 6, 7, 8, 9, or 10, from the mature polypeptide of SEQ ID NO: 3.

In an embodiment, the present invention relates to isolated polypeptides having a sequence identity to SEQ ID NO: 8 of at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%, which have DNase activity. In one aspect, the polypeptides differ by up to 10 amino acids, e.g., 1, 2, 3, 4, 5, 6, 7, 8, 9, or 10, from the mature polypeptide of SEQ ID NO: 8.

A polypeptide of the present invention preferably comprises or consists of the amino acid sequence of SEQ ID NO: 2 or an allelic variant thereof; or is a fragment thereof having DNase activity. In another aspect, the polypeptide comprises or consists of the mature polypeptide of SEQ ID NO: 2. In another aspect, the polypeptide comprises or consists of amino acids 1 to 206 of SEQ ID NO: 2.

In an embodiment, the polypeptide has been isolated. A polypeptide of the present invention preferably comprises or consists of the amino acid sequence of SEQ ID NO: 3 or an allelic variant thereof; or is a fragment thereof having DNase activity. In another aspect, the polypeptide comprises or consists of the mature polypeptide of SEQ ID NO: 3. In another aspect, the polypeptide comprises or consists of amino acids 1 to 204 of SEQ ID NO: 3. One aspect of the present invention relates to a composition comprising or consisting of a polypeptide consisting of the amino acid sequence of SEQ ID NO: 8 and a polypeptide of the present invention consisting of the amino acid sequence of SEQ ID NO: 3.

In another embodiment, the present invention relates to an isolated polypeptide having DNase activity encoded by a polynucleotide that hybridizes under low stringency conditions with (i) the mature polypeptide coding sequence of SEQ ID NO: 1, (ii) the cDNA sequence thereof, or (iii) the full-length complement of (i) or (ii) (Sambrook et al., 1989, Molecular Cloning, A Laboratory Manual, 2d edition, Cold Spring Harbor, New York). In an embodiment, the polypeptide has been isolated.

In another embodiment, the present invention relates to an isolated polypeptide having DNase activity encoded by a polynucleotide that hybridizes under low-medium stringency conditions with (i) the mature polypeptide coding sequence of SEQ ID NO: 1, (ii) the cDNA sequence thereof, or (iii) the full-length complement of (i) or (ii). In an embodiment, the polypeptide has been isolated.

In another embodiment, the present invention relates to an isolated polypeptide having DNase activity encoded by a polynucleotide that hybridizes under medium stringency conditions with (i) the mature polypeptide coding sequence of SEQ ID NO: 1, (ii) the cDNA sequence thereof, or (iii) the full-length complement of (i) or (ii). In an embodiment, the polypeptide has been isolated.

In another embodiment, the present invention relates to an isolated polypeptide having DNase activity encoded by a polynucleotide that hybridizes under medium-high stringency conditions with (i) the mature polypeptide coding sequence of SEQ ID NO: 1, (ii) the cDNA sequence thereof, or (iii) the full-length complement of (i) or (ii). In an embodiment, the polypeptide has been isolated.

In another embodiment, the present invention relates to an isolated polypeptide having DNase activity encoded by a polynucleotide that hybridizes under high stringency conditions with (i) the mature polypeptide coding sequence of SEQ ID NO: 1, (ii) the cDNA sequence thereof, or (iii) the full-length complement of (i) or (ii). In an embodiment, the polypeptide has been isolated.

In another embodiment, the present invention relates to an isolated polypeptide having DNase activity encoded by a polynucleotide that hybridizes under very high stringency conditions with (i) the mature polypeptide coding sequence of SEQ ID NO: 1, (ii) the cDNA sequence thereof, or (iii) the full-length complement of (i) or (ii). In an embodiment, the polypeptide has been isolated.

In another embodiment, the present invention relates to an polypeptide having DNase activity encoded by a polynucleotide having a sequence identity to the mature polypeptide coding sequence of SEQ ID NO: 1 or the cDNA sequence thereof of at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%. In a further embodiment, the polypeptide has been isolated.

In another embodiment, the present invention relates to variants of the mature polypeptide of SEQ ID NO: 2 comprising a substitution, deletion, and/or insertion at one or more (*e.g.,* several) positions. In an embodiment, the number of amino acid substitutions, deletions and/or insertions introduced into the mature polypeptide of SEQ ID NO: 2 is up to 10, *e.g.,* 1, 2, 3, 4, 5, 6, 7, 8, 9, or 10. The amino acid changes may be of a minor nature, that is conservative amino acid substitutions or insertions that do not significantly affect the folding and/or activity of the protein; small deletions, typically of 1-30 amino acids; small amino- or carboxyl-terminal extensions, such as an amino-terminal methionine residue; a small linker peptide of up to 20-25 residues; or a small extension that facilitates purification by changing net charge or another function, such as a poly-histidine tract, an antigenic epitope or a binding domain.

In another embodiment, the present invention relates to variants of the mature polypeptide of SEQ ID NO: 3

comprising a substitution, deletion, and/or insertion at one or more (*e.g.,* several) positions. In an embodiment, the number of amino acid substitutions, deletions and/or insertions introduced into the mature polypeptide of SEQ ID NO: 3 is up to 10, *e.g.,* 1, 2, 3, 4, 5, 6, 7, 8, 9, or 10. The amino acid changes may be of a minor nature, that is conservative amino acid substitutions or insertions that do not significantly affect the folding and/or activity of the protein; small deletions, typically of 1-30 amino acids; small amino- or carboxyl-terminal extensions, such as an amino-terminal methionine residue; a small linker peptide of up to 20-25 residues; or a small extension that facilitates purification by changing net charge or another function, such as a poly-histidine tract, an antigenic epitope or a binding domain.

The DNase enzyme may comprise or consist of the amino acid sequence shown as amino acids -37 to 206 of SEQ ID NO: 2 or a fragment thereof that has DNase activity, such as the mature polypeptide. Or the DNase enzyme may comprise or consist of a fragment of amino acids -37 to 206 of SEQ ID NO: 2 or amino acids 1 to 206 of SEQ ID NO: 2 for which fragment one or more amino acids is deleted from the amino and/or carboxyl terminus of SEQ ID NO: 2.

The DNase enzyme may comprise or consist of the amino acid sequence shown as amino acids 1 to 206 of SEQ ID NO: 3 or a fragment thereof that has DNase activity, such as the mature polypeptide. Or the DNase enzyme may comprise or consist of a fragment of amino acids 1 to 206 of SEQ ID NO: 3 or amino acids 1 to 206 of SEQ ID NO: 3 for which fragment one or more amino acids is deleted from the amino and/or carboxyl terminus of SEQ ID NO: 3.

The DNase enzyme may comprise or consist of the amino acid sequence shown as amino acids 1 to 206 of SEQ ID NO: 8 or a fragment thereof that has DNase activity, such as the mature polypeptide. Or the DNase enzyme may comprise or consist of a fragment of amino acids 1 to 206 of SEQ ID NO: 8 or amino acids 1 to 206 of SEQ ID NO: 8 for which fragment one or more amino acids is deleted from the amino and/or carboxyl terminus of SEQ ID NO: 8.

The present invention also provides DNase polypeptides that are substantially homologous to the polypeptides above, and species homologs (paralogs or orthologs) thereof. The term "substantially homologous" is used herein to denote polypeptides being at least 80%, preferably at least 85%, more preferably at least 90%, more preferably at least 95%, even more preferably at least 97% identical, and most preferably at least 99% or more identical to the amino acid sequence of SEQ ID NO: 2 or to the amino acid sequence of SEQ ID NO: 3, or a fragment thereof that has DNase activity, or its orthologs or paralogs.

In another embodiment, the DNase of SEQ ID NO: 2 comprises a substitution, deletion, and/or insertion at one or more (*e.g.,* several) positions. In another embodiment, the DNase of SEQ ID NO: 3 comprises a substitution, deletion, and/or insertion at one or more (*e.g.,* several) positions. In an embodiment, the number of amino acid substitutions, deletions and/or insertions introduced into the mature polypeptide of SEQ ID NO: 2 or into the mature polypeptide of SEQ ID NO: 3 is not more than 10, e.g., 1, 2, 3, 4, 5, 6, 7, 8 or 9. The amino acid changes may be of a minor nature, that is conservative amino acid substitutions or insertions that do not significantly affect the folding and/or activity of the protein; small deletions, typically of 1-30 amino acids; small amino- or carboxyl-terminal extensions, such as an amino-terminal methionine residue; a small linker peptide of up to 20-25 residues; or a small extension that facilitates purification by changing net charge or another function, such as a poly-histidine tract, an antigenic epitope or a binding domain.

According to the present invention, a DNase which is obtainable from a fungus is preferred; in particular a DNase which is obtainable from a *Trichoderma* is preferred; in particular a DNase which is obtainable from *Trichoderma harzianum* is preferred.

The DNase used in the present invention includes the mature polypeptide of SEQ ID NO: 5, shown as amino acids 1 to 188 of SEQ ID NO: 5, which is obtained from *Trichoderma harzianum.*

The DNase enzyme may comprise or consist of the amino acid sequence shown as amino acids -17 to 188 of SEQ ID NO: 5 or a fragment thereof that has DNase activity, such as the mature polypeptide. Or the DNase enzyme may comprise or consist of a fragment of amino acids -17 to 188 of SEQ ID NO: 5 or amino acids 1 to 188 of SEQ ID NO: 5 for which fragment one or more amino acids is deleted from the amino and/or carboxyl terminus of SEQ ID NO: 5.

The present invention also provides DNase polypeptides that are substantially homologous to the polypeptides above, and species homologs (paralogs or orthologs) thereof. The term "substantially homologous" is used herein to denote polypeptides being at least 80%, preferably at least 85%, more preferably at least 90%, more preferably at least 95%, even more preferably at least 97% identical, and most preferably at least 99% or more identical to the amino acid sequence of SEQ ID NO: 5, or a fragment thereof that has DNase activity, or its orthologs or paralogs.

According to the present invention, a DNase which is obtainable from a bacterium is preferred; in particular a DNase which is obtainable from a *Bacillus* is preferred; in particular a DNase which is obtainable from *Bacillus subtilis* or *Bacillus licheniformis* is preferred.

The DNase used in the present invention includes the mature polypeptide of SEQ ID NO: 6, shown as amino acids 1 to 110 of SEQ ID NO: 6, which is derived from *Bacillus subtilis;* or the mature polypeptide of SEQ ID NO: 7, shown as amino acids 1 to 109 of SEQ ID NO: 7, which is derived from *Bacillus licheniformis.*

The DNase enzyme may comprise or consist of the amino acid sequence shown as amino acids -26 to 110 of SEQ ID NO: 6 or amino acids -33 to 109 of SEQ ID NO: 7, or a fragment thereof that has DNase activity, such as the mature polypeptide. A fragment of amino acids -26 to 110 of SEQ ID NO: 6, or amino acids 1 to 110 of SEQ ID NO: 6 is a polypeptide, which has one or more amino acids deleted from the amino and/or carboxyl terminus of SEQ ID NO:

6. A fragment of or amino acids -33 to 109 of SEQ ID NO: 7, or 1 to 109 of SEQ ID NO: 7 is a polypeptide, which has one or more amino acids deleted from the amino and/or carboxyl terminus of SEQ ID NO: 7.

The present invention also provides DNase polypeptides that are substantially homologous to the polypeptides above, and species homologs (paralogs or orthologs) thereof. The term "substantially homologous" is used herein to denote polypeptides being at least 80%, preferably at least 85%, more preferably at least 90%, more preferably at least 95%, even more preferably at least 97% identical, and most preferably at least 99% or more identical to the amino acid sequence of SEQ ID NO: 6 or SEQ ID NO: 7, or a fragment thereof that has DNase activity, or its orthologs or paralogs.

Examples of conservative substitutions are within the groups of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions that do not generally alter specific activity are known in the art and are described, for example, by H. Neurath and R.L. Hill, 1979, In, The Proteins, Academic Press, New York. Common substitutions are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly.

Alternatively, the amino acid changes are of such a nature that the physico-chemical properties of the polypeptides are altered. For example, amino acid changes may improve the thermal stability of the polypeptide, alter the substrate specificity, change the pH optimum, and the like.

Essential amino acids in a polypeptide can be identified according to procedures known in the art, such as site-directed mutagenesis or alanine-scanning mutagenesis (Cunningham and Wells, 1989, Science 244: 1081-1085). In the latter technique, single alanine mutations are introduced at every residue in the molecule, and the resultant mutant molecules are tested for DNase activity to identify amino acid residues that are critical to the activity of the molecule. See also, Hilton et al., 1996, J. Biol. Chem. 271: 4699-4708. The active site of the enzyme or other biological interaction can also be determined by physical analysis of structure, as determined by such techniques as nuclear magnetic resonance, crystallography, electron diffraction, or photoaffinity labeling, in conjunction with mutation of putative contact site amino acids. See, for example, de Vos et al., 1992, Science 255: 306-312; Smith et al., 1992, J. Mol. Biol. 224: 899-904; Wlodaver et al., 1992, FEBS Lett. 309: 59-64. The identity of essential amino acids can also be inferred from an alignment with a related polypeptide.

Single or multiple amino acid substitutions, deletions, and/or insertions can be made and tested using known methods of mutagenesis, recombination, and/or shuffling, followed by a relevant screening procedure, such as those disclosed by Reidhaar-Olson and Sauer, 1988, Science 241: 53-57; Bowie and Sauer, 1989, Proc. Natl. Acad. Sci. USA 86: 2152-2156; WO 95/17413; or WO 95/22625. Other methods that can be used include error-prone PCR, phage display (e.g., Lowman et al., 1991, Biochemistry 30: 10832-10837; U.S. Patent No. 5,223,409; WO 92/06204), and region-directed mutagenesis (Derbyshire et al., 1986, Gene 46: 145; Ner et al., 1988, DNA 7: 127).

Mutagenesis/shuffling methods can be combined with high-throughput, automated screening methods to detect activity of cloned, mutagenized polypeptides expressed by host cells (Ness et al., 1999, Nature Biotechnology 17: 893-896). Mutagenized DNA molecules that encode active polypeptides can be recovered from the host cells and rapidly sequenced using standard methods in the art. These methods allow the rapid determination of the importance of individual amino acid residues in a polypeptide.

The polypeptide may be a hybrid polypeptide in which a region of one polypeptide is fused at the N-terminus or the C-terminus of a region of another polypeptide.

The polypeptide may be a fusion polypeptide or cleavable fusion polypeptide in which another polypeptide is fused at the N-terminus or the C-terminus of the polypeptide of the present invention. A fusion polypeptide is produced by fusing a polynucleotide encoding another polypeptide to a polynucleotide of the present invention. Techniques for producing fusion polypeptides are known in the art, and include ligating the coding sequences encoding the polypeptides so that they are in frame and that expression of the fusion polypeptide is under control of the same promoter(s) and terminator. Fusion polypeptides may also be constructed using intein technology in which fusion polypeptides are created post-translationally (Cooper et al., 1993, EMBO J. 12: 2575-2583; Dawson et al., 1994, Science 266: 776-779).

A fusion polypeptide can further comprise a cleavage site between the two polypeptides. Upon secretion of the fusion protein, the site is cleaved releasing the two polypeptides. Examples of cleavage sites include, but are not limited to, the sites disclosed in Martin et al., 2003, J. Ind. Microbiol. Biotechnol. 3: 568-576; Svetina et al., 2000, J. Biotechnol. 76: 245-251; Rasmussen-Wilson et al., 1997, Appl. Environ. Microbiol. 63: 3488-3493; Ward et al., 1995, Biotechnology 13: 498-503; and Contreras et al., 1991, Biotechnology 9: 378-381; Eaton et al., 1986, Biochemistry 25: 505-512; Collins-Racie et al., 1995, Biotechnology 13: 982-987; Carter et al., 1989, Proteins: Structure, Function, and Genetics 6: 240-248; and Stevens, 2003, Drug Discovery World 4: 35-48.

The concentration of the DNase is typically in the range of 0.00004-100 ppm enzyme protein, such as in the range of 0.00008-100, in the range of 0.0001-100, in the range of 0.0002-100, in the range of 0.0004-100, in the range of 0.0008-100, in the range of 0.001-100 ppm enzyme protein, 0.01-100 ppm enzyme protein, preferably 0.05-50 ppm enzyme protein, more preferably 0.1-50 ppm enzyme protein, more preferably 0.1-30 ppm enzyme protein, more pref-

erably 0.5-20 ppm enzyme protein, and most preferably 0.5-10 ppm enzyme protein.

The DNase of the present invention may be added to a detergent composition in an amount corresponding to at least 0.002 mg of DNase protein, such as at least 0.004 mg of DNase protein, at least 0.006 mg of DNase protein, at least 0.008 mg of DNase protein, at least 0.01 mg of DNase protein, at least 0.1 mg of protein, preferably at least 1 mg of protein, more preferably at least 10 mg of protein, even more preferably at least 15 mg of protein, most preferably at least 20 mg of protein, and even most preferably at least 25 mg of protein. Thus, the detergent composition may comprise at least 0.00008% DNase protein, preferably at least 0.002%, 0.003%, 0.004%, 0.005%, 0.006%, 0.008%, 0.01%, 0.02%, 0.03%, 0.05%, 0.1%, 0.2%, 0.3%, 0.4%, 0.6%, 0.7%, 0.8%, 0.9% or 1.0% of DNase protein.

The DNase of the detergent composition of the invention may be stabilized using conventional stabilizing agents, e.g. a polyol such as propylene glycol or glycerol, a sugar or sugar alcohol, lactic acid, boric acid, or a boric acid derivative, e.g. an aromatic borate ester, or a phenyl boronic acid derivative such as 4-formylphenyl boronic acid, and the composition may be formulated as described in, for example, WO92/19709 and WO92/19708.

A polypeptide of the present invention may also be incorporated in the detergent formulations disclosed in WO97/07202, which is hereby incorporated by reference.

**Detergent compositions**

In one embodiment, the invention is directed to detergent compositions comprising an enzyme of the present invention in combination with one or more additional cleaning composition components. The choice of additional components is within the skill of the artisan and includes conventional ingredients, including the exemplary non-limiting components set forth below.

**Metal care agents**

Metal care agents may prevent or reduce the tarnishing, corrosion or oxidation of metals, including aluminium, stainless steel and non-ferrous metals, such as silver and copper. Suitable examples include one or more of the following:

(a) benzatriazoles, including benzotriazole or bis-benzotriazole and substituted derivatives thereof. Benzotriazole derivatives are those compounds in which the available substitution sites on the aromatic ring are partially or completely substituted. Suitable substituents include linear or branch-chain Ci-C20- alkyl groups and hydroxyl, thio, phenyl or halogen such as fluorine, chlorine, bromine and iodine;

(b) metal salts and complexes chosen from the group consisting of zinc, manganese, titanium, zirconium, hafnium, vanadium, cobalt, gallium and cerium salts and/or complexes, the metals being in one of the oxidation states II, III, IV, V or VI. In one aspect, suitable metal salts and/or metal complexes may be chosen from the group consisting of Mn(II) sulphate, Mn(II) citrate, Mn(II) stearate, Mn(II) acetylacetonate, KTiF6, KZrF6, CoSO4, Co(NOs)2 and Ce(NOs)3, zinc salts, for example zinc sulphate, hydrozincite, zinc acetate or zinc carbonate;

(c) silicates, including sodium or potassium silicate, sodium disilicate, sodium metasilicate, crystalline phyllosilicate and mixtures thereof.

Further suitable organic and inorganic redox-active substances that act as silver/copper corrosion inhibitors are disclosed in WO 94/26860 and WO 94/26859.

Preferably the composition of the invention comprises from 0.1 to 5% by weight of the composition of a metal care agent, preferably the metal care agent is a zinc salt.

**Surfactants**

The dish washing composition can include at least one non-ionic surfactant. Suitable nonionic surfactants include, but are not limited to low-foaming nonionic (LFNI) surfactants. A LFNI surfactant is most typically used in an automatic dishwashing composition because of the improved water- sheeting action (especially from glassware) which they confer to the automatic dishwashing composition. They also may encompass non-silicone, phosphate or nonphosphate polymeric materials which are known to defoam food soils encountered in automatic dishwashing. The LFNI surfactant may have a relatively low cloud point and a high hydrophilic-lipophilic balance (HLB). Cloud points of 1% solutions in water are typically below about 32°C. and alternatively lower, e.g., 0°C, for optimum control of sudsing throughout a full range of water temperatures. If desired, a biodegradable LFNI surfactant having the above properties may be used. A LFNI surfactant may include, but is not limited to: alkoxylated surfactants, especially ethoxylates derived from primary alcohols, and blends thereof with more sophisticated surfactants, such as the polyoxypropylene/polyoxyethylene/polyoxypropylene reverse block polymers. Suitable block polyoxyethylene-polyoxypropylene polymeric compounds that meet the requirements may include those based on ethylene glycol, propylene glycol, glycerol, trimethylolpropane and ethylenediamine, and mixtures thereof. Polymeric compounds made from a sequential ethoxylation and propoxylation of initiator compounds with a single reactive hydrogen atom, such as C 12- is aliphatic alcohols, do not generally provide satisfactory suds control in Automatic dishwashing compositions. However, certain of the block polymer surfactant compounds designated as PLURONIC(R) and TETRONIC(R) by the BASF-Wyandotte Corp., Wyandotte, Mich., are suitable in Automatic dishwashing compositions.

The LFNI surfactant can optionally include a propylene oxide in an amount up to about 15% by weight. Other LFNI surfactants can be prepared by the processes described in U.S. Pat. No. 4,223,163. The LFNI surfactant may also be derived from a straight chain fatty alcohol containing from about 16 to about 20 carbon atoms (C16-C20 alcohol), alternatively a Ci8 alcohol, condensed with an average of from about 6 to about 15 moles, or from about 7 to about 12 moles, and alternatively, from about 7 to about 9 moles of ethylene oxide per mole of alcohol. The ethoxylated nonionic surfactant so derived may have a narrow ethoxylate distribution relative to the average.

In certain embodiments, a LFNI surfactant having a cloud point below 30°C. may be present in an amount from about 0.01% to about 60%, or from about 0.5% to about 10% by weight, and alternatively, from about 1% to about 5% by weight of the composition

In preferred embodiments, the surfactant is a non-ionic surfactant or a non-ionic surfactant system having a phase inversion temperature, as measured at a concentration of 1% in distilled water, between 40 and 70°C, preferably between 45 and 65°C. By a "non-ionic surfactant system" is meant herein a mixture of two or more non-ionic surfactants. Preferred for use herein are non-ionic surfactant systems. They seem to have improved cleaning and finishing properties and stability in product than single non-ionic surfactants.

Suitable nonionic surfactants include: i) ethoxylated non-ionic surfactants prepared by the reaction of a monohydroxy alkanol or alkyphenol with 6 to 20 carbon atoms with preferably at least 12 moles particularly preferred at least 16 moles, and still more preferred at least 20 moles of ethylene oxide per mole of alcohol or alkylphenol; ii) alcohol alkoxylated surfactants having a from 6 to 20 carbon atoms and at least one ethoxy and propoxy group. Preferred for use herein are mixtures of surfactants i) and ii).

Another suitable non-ionic surfactants are epoxy-capped poly(oxyalkylated) alcohols represented by the formula:

$$R_1O[CH_2CH(CH_3)O]_x[CH_2CH_2O]_y[CH_2CH(OH)R_2] \qquad (I)$$

wherein $R_1$ is a linear or branched, aliphatic hydrocarbon radical having from 4 to 18 carbon atoms; $R_2$ is a linear or branched aliphatic hydrocarbon radical having from 2 to 26 carbon atoms; x is an integer having an average value of from 0.5 to 1.5, more preferably about 1; and y is an integer having a value of at least 15, more preferably at least 20.

Preferably, the surfactant of formula I has at least about 10 carbon atoms in the terminal epoxide unit $[CH_2CH(OH)R_2]$. Suitable surfactants of formula I are Olin Corporation's POLY-TERGENT(R) SLF- 18B nonionic surfactants, as described, for example, in WO 94/22800, published October 13, 1994 by Olin Corporation.

Preferably non-ionic surfactants and/or system herein have a Draves wetting time of less than 360 seconds, preferably less than 200 seconds, more preferably less than 100 seconds and especially less than 60 seconds as measured by the Draves wetting method (standard method ISO 8022 using the following conditions; 3-g hook, 5-g cotton skein, 0.1% by weight aqueous solution at a temperature of 25 °C). Amine oxides surfactants are also useful in the present invention as anti-redeposition surfactants include linear and branched compounds having the formula:

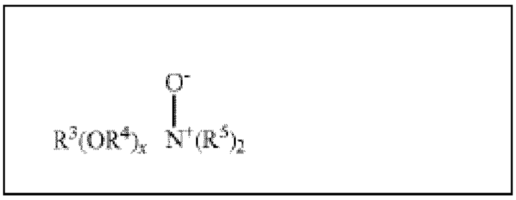

wherein $R^3$ is selected from an alkyl, hydroxyalkyl, acylamidopropoyl and alkyl phenyl group, or mixtures thereof, containing from 8 to 26 carbon atoms, preferably 8 to 18 carbon atoms; $R^4$ is an alkylene or hydroxyalkylene group containing from 2 to 3 carbon atoms, preferably 2 carbon atoms, or mixtures thereof; x is from 0 to 5, preferably from 0 to 3; and each $R^5$ is an alkyl or hydroxyalkyl group containing from 1 to 3, preferably from 1 to 2 carbon atoms, or a polyethylene oxide group containing from 1 to 3, preferable 1, ethylene oxide groups. The $R^5$ groups can be attached to each other, e.g., through an oxygen or nitrogen atom, to form a ring structure. These amine oxide surfactants in particular include $C_{10}$-$C_{18}$ alkyl dimethyl amine oxides and $C_8$-$C_{18}$ alkoxy ethyl dihydroxyethyl amine oxides. Examples of such materials include dimethyloctylamine oxide, diethyldecylamine oxide, bis-(2-hydroxyethyl)dodecylamine oxide, dimethyldodecylamine oxide, dipropyltetradecylamine oxide, methylethylhexadecylamine oxide, dodecylamidopropyl dimethylamine oxide, cetyl dimethylamine oxide, stearyl dimethylamine oxide, tallow dimethylamine oxide and dimethyl-2-hydroxyoctadecylamine oxide. Preferred are $C_{10}$-$C_{18}$ alkyl dimethylamine oxide, and $C_{10}$-$C_{18}$ acylamido alkyl dimethylamine oxide.

Surfactants and especially non-ionic surfactants may be present in amounts from 0 to 10% by weight, preferably from 0.1% to 10%, and most preferably from 0.25% to 6%.

**Sulfonated polymer**

The polymer, if used, is used in any suitable amount from about 0.1% to about 20%, preferably from 1% to about 15%, more preferably from 2% to 10% by weight of the composition. Sulfonated/carboxylated polymers are particularly suitable for the compositions contained in the pouch of the invention.

Suitable sulfonated/carboxylated polymers described herein may have a weight average molecular weight of less than or equal to about 100,000 Da, or less than or equal to about 75,000 Da, or less than or equal to about 50,000 Da, or from about 3,000 Da to about 50,000, preferably from about 5,000 Da to about 45,000 Da.

As noted herein, the sulfonated/carboxylated polymers may comprise (a) at least one structural unit derived from at least one carboxylic acid monomer having the general formula (I):

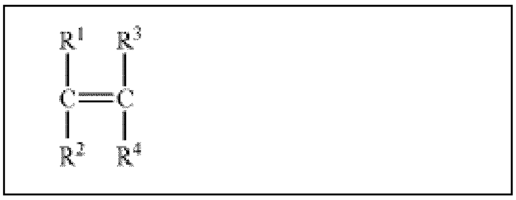

wherein $R^1$ to $R^4$ are independently hydrogen, methyl, carboxylic acid group or $CH_2COOH$ and wherein the carboxylic acid groups can be neutralized; (b) optionally, one or more structural units derived from at least one nonionic monomer having the general formula (II):

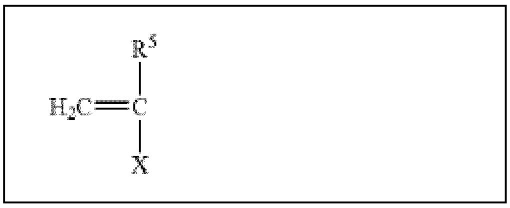

wherein $R^5$ i is hydrogen, $C_1$ to $C_6$ alkyl, or $C_1$ to $C_6$ hydroxyalkyl, and X is either aromatic (with $R^5$ being hydrogen or methyl when X is aromatic) or X is of the general formula (III):

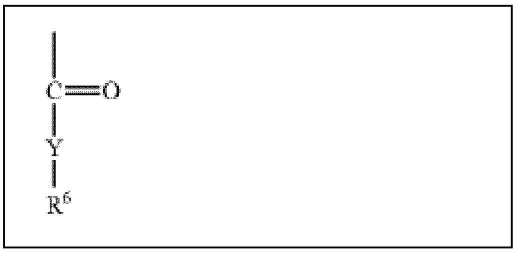

wherein $R^6$ is (independently of $R^5$) hydrogen, $C_1$ to $C_6$ alkyl, or $C_1$ to $C_6$ hydroxyalkyl, and Y is O or N; and at least one structural unit derived from at least one sulfonic acid monomer having the general formula (IV):

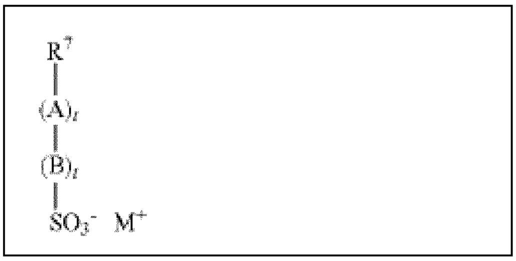

wherein $R^7$ is a group comprising at least one $sp^2$ bond, A is O, N, P, S or an amido or ester linkage, B is a mono- or polycyclic aromatic group or an aliphatic group, each t is independently 0 or 1 , and $M^+$ is a cation. In one aspect, $R^7$ is a $C_2$ to $C_6$ alkene. In another aspect, $R^7$ is ethene, butene or propene.

Preferred carboxylic acid monomers include one or more of the following: acrylic acid, maleic acid, itaconic acid, methacrylic acid, or ethoxylate esters of acrylic acids, acrylic and methacrylic acids being more preferred. Preferred sulfonated monomers include one or more of the following: sodium (meth) allyl sulfonate, vinyl sulfonate, sodium phenyl (meth) allyl ether sulfonate, or 2-acrylamido-methyl propane sulfonic acid. Preferred non-ionic monomers include one or more of the following: methyl (meth) acrylate, ethyl (meth) acrylate, t-butyl (meth) acrylate, methyl (meth) acrylamide, ethyl (meth) acrylamide, t-butyl (meth) acrylamide, styrene, or [alpha]-methyl styrene. Preferably, the polymer comprises the following levels of monomers: from about 40 to about 90%, preferably from about 60 to about 90% by weight of the polymer of one or more carboxylic acid monomer; from about 5 to about 50%, preferably from about 10 to about 40% by weight of the polymer of one or more sulfonic acid monomer; and optionally from about 1 % to about 30%, preferably from about 2 to about 20% by weight of the polymer of one or more non-ionic monomer. An especially preferred polymer comprises about 70% to about 80% by weight of the polymer of at least one carboxylic acid monomer and from about 20% to about 30% by weight of the polymer of at least one sulfonic acid monomer. 99

The carboxylic acid is preferably (meth)acrylic acid. The sulfonic acid monomer is preferably one of the following: 2-acrylamido methyl- 1-propanesulfonic acid, 2-methacrylamido-2-methyl- 1-propanesulfonic acid, 3-methacrylamido-2-hydroxypropanesulfonic acid, allysulfonic acid, methallysulfonic acid, allyloxybenzenesulfonic acid, methallyloxybenzensulfonic acid, 2- hydroxy-3-(2-propenyloxy)propanesulfonic acid, 2-methyl-2-propene-l-sulfonic acid, styrene sulfonic acid, vinylsulfonic acid, 3-sulfopropyl acrylate, 3-sulfopropyl methacrylate, sulfomethylacrylamid, sulfomethylmethacrylamide, and water soluble salts thereof. The unsaturated sulfonic acid monomer is most preferably 2-acrylamido-2-propanesulfonic acid (AMPS).

Preferred commercial available polymers include: Alcosperse 240, Aquatreat AR 540 and Aquatreat MPS supplied by Alco Chemical; Acumer 3100, Acumer 2000, Acusol 587G and Acusol 588G supplied by Rohm & Haas; Goodrich K-798, K-775 and K-797 supplied by BF Goodrich; and ACP 1042 supplied by ISP technologies Inc. Particularly preferred polymers are Acusol 587G and Acusol 588G supplied by Rohm & Haas.

In the polymers, all or some of the carboxylic or sulfonic acid groups can be present in neutralized form, i.e. the acidic hydrogen atom of the carboxylic and/or sulfonic acid group in some or all acid groups can be replaced with metal ions, preferably alkali metal ions and in particular with sodium ions.

**Hydrotropes**

A hydrotrope is a compound that solubilises hydrophobic compounds in aqueous solutions (or oppositely, polar substances in a non-polar environment). Typically, hydrotropes have both hydrophilic and a hydrophobic character (so-called amphiphilic properties as known from surfactants); however the molecular structure of hydrotropes generally do not favor spontaneous self-aggregation, see e.g. review by Hodgdon and Kaler (2007), Current Opinion in Colloid & Interface Science 12: 121-128. Hydrotropes do not display a critical concentration above which self-aggregation occurs as found for surfactants and lipids forming miceller, lamellar or other well defined meso-phases. Instead, many hydrotropes show a continuous-type aggregation process where the sizes of aggregates grow as concentration increases. However, many hydrotropes alter the phase behavior, stability, and colloidal properties of systems containing substances of polar and non-polar character, including mixtures of water, oil, surfactants, and polymers. Hydrotropes are classically used across industries from pharma, personal care, food, to technical applications. Use of hydrotropes in detergent compositions allow for example more concentrated formulations of surfactants (as in the process of compacting liquid detergents by removing water) without inducing undesired phenomena such as phase separation or high viscosity.

The detergent may contain 0-10% by weight, for example 0-5% by weight, such as about 0.5 to about 5%, or about 3% to about 5%, of a hydrotrope. Any hydrotrope known in the art for use in detergents may be utilized. Non-limiting examples of hydrotropes include sodium benzenesulfonate, sodium p-toluene sulfonate (STS), sodium xylene sulfonate (SXS), sodium cumene sulfonate (SCS), sodium cymene sulfonate, amine oxides, alcohols and polyglycolethers, sodium hydroxynaphthoate, sodium hydroxynaphthalene sulfonate, sodium ethylhexyl sulfate, and combinations thereof.

### Builders and Co-Builders

The detergent composition may contain about 0-65% by weight, such as about 5% to about 50% of a detergent builder or co-builder, or a mixture thereof. The builder and/or co-builder may particularly be a chelating agent that forms water-soluble complexes with Ca and Mg. Any builder and/or co-builder known in the art for use in detergents may be utilized. Non-limiting examples of builders include zeolites, diphosphates (pyrophosphates), triphosphates such as sodium triphosphate (STP or STPP), carbonates such as sodium carbonate, soluble silicates such as sodium metasilicate, layered silicates (*e.g.,* SKS-6 from Hoechst), ethanolamines such as 2-aminoethan-1-ol (MEA), diethanolamine (DEA, also known as 2,2'-iminodiethan-1-ol), triethanolamine (TEA, also known as 2,2',2''-nitrilotriethan-1-ol), and (carboxymethyl)inulin (CMI), and combinations thereof.

The detergent composition may also contain 0-50% by weight, such as about 5% to about 30%, of a detergent co-builder. The detergent composition may include include a co-builder alone, or in combination with a builder, for example a zeolite builder. Non-limiting examples of co-builders include homopolymers of polyacrylates or copolymers thereof, such as poly(acrylic acid) (PAA) or copoly(acrylic acid/maleic acid) (PAA/PMA). Further non-limiting examples include citrate, chelators such as aminocarboxylates, aminopolycarboxylates and phosphonates, and alkyl- or alkenylsuccinic acid. Additional specific examples include 2,2',2''-nitrilotriacetic acid (NTA), ethylenediaminetetraacetic acid (EDTA), diethylenetriaminepentaacetic acid (DTPA), iminodisuccinic acid (IDS), ethylenediamine-*N,N'*-disuccinic acid (EDDS), methylglycinediacetic acid (MGDA), glutamic acid-*N,N*-diacetic acid (GLDA), 1-hydroxyethane-1,1-diphosphonic acid (HEDP), ethylenediaminetetra(methylenephosphonic acid) (EDTMPA), diethylenetriaminepentakis(methylenephosphonic acid) (DTMPA or DTPMPA), *N*-(2-hydroxyethyl)iminodiacetic acid (EDG), aspartic acid-*N*-monoacetic acid (ASMA), aspartic acid-*N,N*-diacetic acid (ASDA), aspartic acid-*N*-monopropionic acid (ASMP), iminodisuccinic acid (IDA), *N*-(2-sulfomethyl)-aspartic acid (SMAS), *N*-(2-sulfoethyl)-aspartic acid (SEAS), *N*-(2-sulfomethyl)-glutamic acid (SMGL), *N*-(2-sulfoethyl)-glutamic acid (SEGL), *N*-methyliminodiacetic acid (MIDA), $\alpha$-alanine-*N,N*-diacetic acid (a-ALDA), serine-*N,N*-diacetic acid (SEDA), isoserine-*N,N*-diacetic acid (ISDA), phenylalanine-*N,N*-diacetic acid (PHDA), anthranilic acid-*N,N*-diacetic acid (ANDA), sulfanilic acid-*N,N*-diacetic acid (SLDA), taurine-*N,N*-diacetic acid (TUDA) and sulfomethyl-*N,N*-diacetic acid (SMDA), *N*-(2-hydroxyethyl)ethylenediamine-*N,N',N''*-triacetic acid (HEDTA), diethanolglycine (DEG), diethylenetriamine penta(methylenephosphonic acid) (DTPMP), aminotris(methylenephosphonic acid) (ATMP), and combinations and salts thereof. Further exemplary builders and/or co-builders are described in, e.g., WO 09/102854, US 5977053

### Bleaching Systems

The detergent may contain 0-30% by weight, such as about 1% to about 20%, of a bleaching system. Any bleaching system known in the art for use in detergents may be utilized. Suitable bleaching system components include bleaching catalysts, photobleaches, bleach activators, sources of hydrogen peroxide such as sodium percarbonate, sodium perborates and hydrogen peroxide-urea (1:1), preformed peracids and mixtures thereof. Suitable preformed peracids include, but are not limited to, peroxycarboxylic acids and salts, diperoxydicarboxylic acids, perimidic acids and salts, peroxymonosulfuric acids and salts, for example, Oxone (R), and mixtures thereof. Non-limiting examples of bleaching systems include peroxide-based bleaching systems, which may comprise, for example, an inorganic salt, including alkali metal salts such as sodium salts of perborate (usually mono- or tetra-hydrate), percarbonate, persulfate, perphosphate, persilicate salts, in combination with a peracid-forming bleach activator. The term bleach activator is meant herein as a compound which reacts with hydrogen peroxide to form a peracid via perhydrolysis. The peracid thus formed constitutes the activated bleach. Suitable bleach activators to be used herein include those belonging to the class of esters, amides, imides or anhydrides. Suitable examples are tetraacetylethylenediamine (TAED), sodium 4-[(3,5,5-trimethylhexanoyl)oxy]benzene-1-sulfonate (ISONOBS), 4-(dodecanoyloxy)benzene-1-sulfonate (LOBS), 4-(decanoyloxy)benzene-1-sulfonate, 4-(decanoyloxy)benzoate (DOBS or DOBA), 4-(nonanoyloxy)benzene-1-sulfonate (NOBS), and/or those disclosed in WO98/17767. A particular family of bleach activators of interest was disclosed in EP624154 and particulary preferred in that family is acetyl triethyl citrate (ATC). ATC or a short chain triglyceride like triacetin has the advantage that it is environmentally friendly Furthermore acetyl triethyl citrate and triacetin have good hydrolytical stability in the product upon storage and are efficient bleach activators. Finally ATC is multifunctional, as the citrate released in the perhydrolysis reaction may function as a builder. Alternatively, the bleaching system may comprise peroxyacids of, for example, the amide, imide, or sulfone type. The bleaching system may also comprise peracids such as 6-(phthalimido)peroxyhexanoic acid (PAP). The bleaching system may also include a bleach catalyst. In some embodiments the bleach component may be an organic catalyst selected from the group consisting of organic catalysts having the following formulae:

(i)

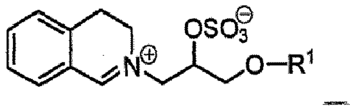

(ii)

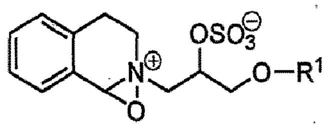

(iii) and mixtures thereof;

wherein each $R^1$ is independently a branched alkyl group containing from 9 to 24 carbons or linear alkyl group containing from 11 to 24 carbons, preferably each $R^1$ is independently a branched alkyl group containing from 9 to 18 carbons or linear alkyl group containing from 11 to 18 carbons, more preferably each $R^1$ is independently selected from the group consisting of 2-propylheptyl, 2-butyloctyl, 2-pentylnonyl, 2-hexyldecyl, dodecyl, tetradecyl, hexadecyl, octadecyl, isononyl, isodecyl, isotridecyl and isopentadecyl. Other exemplary bleaching systems are described, e.g. in WO2007/087258, WO2007/087244, WO2007/087259, EP1867708 (Vitamin K) and WO2007/087242. Suitable photobleaches may for example be sulfonated zinc or aluminium phthalocyanines.

Preferably the bleach component comprises a source of peracid in addition to bleach catalyst, particularly organic bleach catalyst. The source of peracid may be selected from (a) preformed peracid; (b) percarbonate, perborate or persulfate salt (hydrogen peroxide source) preferably in combination with a bleach activator; and (c) perhydrolase enzyme and an ester for forming peracid in situ in the presence of water in a textile treatment step.

**Metal care agents**

Metal care agents may prevent or reduce the tarnishing, corrosion or oxidation of metals, including aluminium, stainless steel and non-ferrous metals, such as silver and copper. Suitable examples include one or more of the following:

(a) benzatriazoles, including benzotriazole or bis-benzotriazole and substituted derivatives thereof. Benzotriazole derivatives are those compounds in which the available substitution sites on the aromatic ring are partially or completely substituted. Suitable substituents include linear or branch-chain Ci-C20- alkyl groups and hydroxyl, thio, phenyl or halogen such as fluorine, chlorine, bromine and iodine.

(b) metal salts and complexes chosen from the group consisting of zinc, manganese, titanium, zirconium, hafnium, vanadium, cobalt, gallium and cerium salts and/or complexes, the metals being in one of the oxidation states II, III, IV, V or VI. In one aspect, suitable metal salts and/or metal complexes may be chosen from the group consisting of Mn(II) sulphate, Mn(II) citrate, Mn(II) stearate, Mn(II) acetylacetonate, KTiF6, KZrF6, CoSO4, Co(NOs)2 and Ce(NOs)3, zinc salts, for example zinc sulphate, hydrozincite, zinc acetate or zinc carbonate; (c) silicates, including sodium or potassium silicate, sodium disilicate, sodium metasilicate, crystalline phyllosilicate and mixtures thereof.

Further suitable organic and inorganic redox-active substances that act as silver/copper corrosion inhibitors are disclosed in WO 94/26860 and WO 94/26859.

Preferably the composition of the invention comprises from 0.1 to 5% by weight of the composition of a metal care agent, preferably the metal care agent is a zinc salt.

**Polymers**

The detergent may contain 0-10% by weight, such as 0.5-5%, 2-5%, 0.5-2% or 0.2-1% of a polymer. Any polymer known in the art for use in detergents may be utilized. The polymer may function as a co-builder as mentioned above, or may provide antiredeposition, fiber protection, soil release, dye transfer inhibition, grease cleaning and/or anti-foaming properties. Some polymers may have more than one of the above-mentioned properties and/or more than one of the below-mentioned motifs. Exemplary polymers include (carboxymethyl)cellulose (CMC), poly(vinyl alcohol) (PVA), poly(vinylpyrrolidone) (PVP), poly(ethyleneglycol) or poly(ethylene oxide) (PEG), ethoxylated poly(ethyleneimine), carboxymethyl inulin (CMI), and polycarboxylates such as PAA, PAA/PMA, poly-aspartic acid, and lauryl methacrylate/acrylic acid copolymers , hydrophobically modified CMC (HM-CMC) and silicones, copolymers of terephthalic acid and oligomeric glycols, copolymers of poly(ethylene terephthalate) and poly(oxyethene terephthalate) (PET-POET), PVP, poly(vinylimidazole) (PVI), poly(vinylpyridine-*N*-oxide) (PVPO or PVPNO) and polyvinylpyrrolidone-vinylimidazole (PVPVI). Further exemplary polymers include sulfonated polycarboxylates, polyethylene oxide and polypropylene oxide (PEO-PPO) and diquaternium ethoxy sulfate. Other exemplary polymers are disclosed in, e.g., WO 2006/130575. Salts of the above-mentioned polymers are also contemplated.

**Fabric hueing agents**

The detergent compositions of the present invention may also include fabric hueing agents such as dyes or pigments, which when formulated in detergent compositions can deposit onto a fabric when said fabric is contacted with a wash liquor comprising said detergent compositions and thus altering the tint of said fabric through absorption/reflection of visible light. Fluorescent whitening agents emit at least some visible light. In contrast, fabric hueing agents alter the tint of a surface as they absorb at least a portion of the visible light spectrum. Suitable fabric hueing agents include dyes and dye-clay conjugates, and may also include pigments. Suitable dyes include small molecule dyes and polymeric dyes. Suitable small molecule dyes include small molecule dyes selected from the group consisting of dyes falling into the Colour Index (C.I.) classifications of Direct Blue, Direct Red, Direct Violet, Acid Blue, Acid Red, Acid Violet, Basic Blue, Basic Violet and Basic Red, or mixtures thereof, for example as described in WO2005/03274, WO2005/03275, WO2005/03276 and EP1876226 (hereby incorporated by reference). The detergent composition preferably comprises from about 0.00003 wt% to about 0.2 wt%, from about 0.00008 wt% to about 0.05 wt%, or even from about 0.0001 wt% to about 0.04 wt% fabric hueing agent. The composition may comprise from 0.0001 wt% to 0.2 wt% fabric hueing agent, this may be especially preferred when the composition is in the form of a unit dose pouch. Suitable hueing agents are also disclosed in, e.g. WO 2007/087257 and WO2007/087243.

**Enzymes**

The detergent additive as well as the detergent composition may comprise one or more additional enzymes such as a protease, lipase, cutinase, an amylase, carbohydrase, cellulase, pectinase, mannanase, arabinase, galactanase, xylanase, oxidase, *e.g.,* a laccase, and/or peroxidase.

In general the properties of the selected enzyme(s) should be compatible with the selected detergent, (*i.e.,* pH-optimum, compatibility with other enzymatic and non-enzymatic ingredients, etc.), and the enzyme(s) should be present in effective amounts.

**Cellulases**

Suitable cellulases include those of bacterial or fungal origin. Chemically modified or protein engineered mutants are included. Suitable cellulases include cellulases from the genera *Bacillus, Pseudomonas, Humicola, Fusarium, Thielavia, Acremonium, e.g.,* the fungal cellulases produced from *Humicola insolens, Myceliophthora thermophila* and *Fusarium oxysporum* disclosed in US 4,435,307, US 5,648,263, US 5,691,178, US 5,776,757 and WO 89/09259.

Especially suitable cellulases are the alkaline or neutral cellulases having colour care benefits. Examples of such cellulases are cellulases described in EP 0 495 257, EP 0 531 372, WO 96/11262, WO 96/29397, WO 98/08940. Other examples are cellulase variants such as those described in WO 94/07998, EP 0 531 315, US 5,457,046, US 5,686,593, US 5,763,254, WO 95/24471, WO 98/12307 and WO99/001544.

Other cellulases are endo-beta-1,4-glucanase enzyme having a sequence of at least 97% identity to the amino acid sequence of position 1 to position 773 of SEQ ID NO:2 of WO 2002/099091 or a family 44 xyloglucanase, which a xyloglucanase enzyme having a sequence of at least 60% identity to positions 40-559 of SEQ ID NO: 2 of WO 2001/062903.

Commercially available cellulases include Celluzyme™, and Carezyme™ (Novozymes A/S) Carezyme Premium™ (Novozymes A/S), Celluclean ™ (Novozymes A/S), Celluclean Classic™ (Novozymes A/S), Cellusoft™ (Novozymes A/S), Whitezyme™ (Novozymes A/S), Clazinase™, and Puradax HA™ (Genencor International Inc.), and KAC-500(B)™ (Kao Corporation).

**Proteases**

Suitable proteases include those of bacterial, fungal, plant, viral or animal origin e.g. vegetable or microbial origin. Microbial origin is preferred. Chemically modified or protein engineered mutants are included. It may be an alkaline protease, such as a serine protease or a metalloprotease. A serine protease may for example be of the S1 family, such

as trypsin, or the S8 family such as subtilisin. A metalloproteases protease may for example be a thermolysin from e.g. family M4 or other metalloprotease such as those from M5, M7 or M8 families.

The term "subtilases" refers to a sub-group of serine protease according to Siezen et al., Protein Engng. 4 (1991) 719-737 and Siezen et al. Protein Science 6 (1997) 501-523. Serine proteases are a subgroup of proteases characterized by having a serine in the active site, which forms a covalent adduct with the substrate. The subtilases may be divided into 6 sub-divisions, i.e. the Subtilisin family, the Thermitase family, the Proteinase K family, the Lantibiotic peptidase family, the Kexin family and the Pyrolysin family.

Examples of subtilases are those obtained from *Bacillus* such as *Bacillus lentus, B. alkalophilus, B. subtilis, B. amyloliquefaciens, Bacillus pumilus* and *Bacillus gibsonii* described in; US7262042 and WO09/021867, and *subtilisin lentus, subtilisin Novo, subtilisin Carlsberg, Bacillus licheniformis, subtilisin BPN', subtilisin 309, subtilisin 147* and *subtilisin 168* described in WO89/06279 and protease PD138 described in (WO93/18140). Other useful proteases may be those described in WO92/175177, WO01/016285, WO02/026024 and WO02/016547. Examples of trypsin-like proteases are trypsin (e.g. of porcine or bovine origin) and the *Fusarium* protease described in WO89/06270, WO94/25583 and WO05/040372, and the chymotrypsin proteases obtained from *Cellumonas* described in WO05/052161 and WO05/052146.

A further preferred protease is the alkaline protease from *Bacillus lentus* DSM 5483, as described for example in WO95/23221, and variants thereof which are described in WO92/21760, WO95/23221, EP1921147 and EP1921148.

Examples of metalloproteases are the neutral metalloprotease as described in WO07/044993 (Genencor Int.) such as those obtained from *Bacillus amyloliquefaciens.*

Examples of useful proteases are the variants described in: WO92/19729, WO96/034946, WO98/20115, WO98/20116, WO99/011768, WO01/44452, WO03/006602, WO04/03186, WO04/041979, WO07/006305, WO11/036263, WO11/036264, especially the variants with substitutions in one or more of the following positions: 3, 4, 9, 15, 27, 36, 57, 68, 76, 87, 95, 96, 97, 98, 99, 100, 101, 102, 103, 104, 106, 118, 120, 123, 128, 129, 130, 160, 167, 170, 194, 195, 199, 205, 206, 217, 218, 222, 224, 232, 235, 236, 245, 248, 252 and 274 using the BPN' numbering. More preferred the subtilase variants may comprise the mutations: S3T, V4I, S9R, A15T, K27R, *36D, V68A, N76D, N87S,R, *97E, A98S, S99G,D,A, S99AD, S101G,M,R S103A, V104I,Y,N, S106A, G118V,R, H120D,N, N123S, S128L, P129Q, S130A, G160D, Y167A, R170S, A194P, G195E, V199M, V205I, L217D, N218D, M222S, A232V, K235L, Q236H, Q245R, N252K, T274A (using BPN' numbering).

Suitable commercially available protease enzymes include those sold under the trade names Alcalase®, Duralase™, Durazym™, Relase®, Relase® Ultra, Savinase®, Savinase® Ultra, Primase®, Polarzyme®, Kannase®, Liquanase®, Liquanase® Ultra, Ovozyme®, Coronase®, Coronase® Ultra, Neutrase®, Everlase® and Esperase® (Novozymes A/S), those sold under the tradename Maxatase®, Maxacal®, Maxapem®, Purafect®, Purafect Prime®, Preferenz™, Purafect MA®, Purafect Ox®, Purafect OxP®, Puramax®, Properase®, Effectenz™, FN2®, FN3®, FN4®, Excellase®,, Opticlean® and Optimase® (Danisco/DuPont), Axapem™ (Gist-Brocases N.V.), BLAP (sequence shown in Figure 29 of US5352604) and variants hereof (Henkel AG) and KAP *(Bacillus alkalophilus* subtilisin) from Kao.

### Lipases and Cutinases

Suitable lipases and cutinases include those of bacterial or fungal origin. Chemically modified or protein engineered mutant enzymes are included. Examples include lipase from *Thermomyces,* e.g. from *T. lanuginosus* (previously named *Humicola lanuginosa)* as described in EP258068 and EP305216, cutinase from *Humicola,* e.g. *H. insolens* (WO96/13580), lipase from strains of *Pseudomonas* (some of these now renamed to *Burkholderia),* e.g. *P. alcaligenes* or *P. pseudoalcaligenes* (EP218272), *P. cepacia* (EP331376), *P. sp.* strain SD705 (WO95/06720 & WO96/27002), *P. wisconsinensis* (WO96/12012), GDSL-type *Streptomyces* lipases (WO10/065455), cutinase from *Magnaporthe grisea* (WO10/107560), cutinase from *Pseudomonas mendocina* (US5,389,536), lipase from *Thermobifida fusca* (WO11/084412), *Geobacillus stearothermophilus* lipase (WO11/084417), lipase from *Bacillus subtilis* (WO11/084599), and lipase from *Streptomyces griseus* (WO11/150157) and S. *pristinaespiralis* (WO12/137147).

Other examples are lipase variants such as those described in EP407225, WO92/05249, WO94/01541, WO94/25578, WO95/14783, WO95/30744, WO95/35381, WO95/22615, WO96/00292, WO97/04079, WO97/07202, WO00/34450, WO00/60063, WO01/92502, WO07/87508 and WO09/109500.

Preferred commercial lipase products include include Lipolase™, Lipex™; Lipolex™ and Lipoclean™ (Novozymes A/S), Lumafast (originally from Genencor) and Lipomax (originally from Gist-Brocades).

Still other examples are lipases sometimes referred to as acyltransferases or perhydrolases, e.g. acyltransferases with homology to *Candida antarctica* lipase A (WO10/111143), acyltransferase from *Mycobacterium smegmatis* (WO05/56782), perhydrolases from the CE 7 family (WO09/67279), and variants of the *M. smegmatis* perhydrolase in particular the S54V variant used in the commercial product Gentle Power Bleach from Huntsman Textile Effects Pte Ltd (WO10/100028).

## Amylases

Suitable amylases which can be used together with the enzyme of the invention may be an alpha-amylase or a glucoamylase and may be of bacterial or fungal origin. Chemically modified or protein engineered mutants are included. Amylases include, for example, alpha-amylases obtained from *Bacillus, e.g.,* a special strain of *Bacillus licheniformis,* described in more detail in GB 1,296,839.

Suitable amylases include amylases having SEQ ID NO: 2 in WO 95/10603 or variants having 90% sequence identity to SEQ ID NO: 3 thereof. Preferred variants are described in WO 94/02597, WO 94/18314, WO 97/43424 and SEQ ID NO: 4 of WO 99/019467, such as variants with substitutions in one or more of the following positions: 15, 23, 105, 106, 124, 128, 133, 154, 156, 178, 179, 181, 188, 190, 197, 201, 202, 207, 208, 209, 211, 243, 264, 304, 305, 391, 408, and 444.

Different suitable amylases include amylases having SEQ ID NO: 6 in WO 02/010355 or variants thereof having 90% sequence identity to SEQ ID NO: 6. Preferred variants of SEQ ID NO: 6 are those having a deletion in positions 181 and 182 and a substitution in position 193.

Other amylases which are suitable are hybrid alpha-amylase comprising residues 1-33 of the alpha-amylase obtained from *B. amyloliquefaciens* shown in SEQ ID NO: 6 of WO 2006/066594 and residues 36-483 of the *B. licheniformis* alpha-amylase shown in SEQ ID NO: 4 of WO 2006/066594 or variants having 90% sequence identity thereof. Preferred variants of this hybrid alpha-amylase are those having a substitution, a deletion or an insertion in one of more of the following positions: G48, T49, G107, H156, A181, N190, M197, 1201, A209 and Q264. Most preferred variants of the hybrid alpha-amylase comprising residues 1-33 of the alpha-amylase obtained from *B. amyloliquefaciens* shown in SEQ ID NO: 6 of WO 2006/066594 and residues 36-483 of SEQ ID NO: 4 are those having the substitutions:

M197T;
H156Y+A181T+N190F+A209V+Q264S; or
G48A+T49I+G107A+H156Y+A181T+N190F+I201F+A209V+Q264S.

Further amylases which are suitable are amylases having SEQ ID NO: 6 in WO 99/019467 or variants thereof having 90% sequence identity to SEQ ID NO: 6. Preferred variants of SEQ ID NO: 6 are those having a substitution, a deletion or an insertion in one or more of the following positions: R181, G182, H183, G184, N195, I206, E212, E216 and K269. Particularly preferred amylases are those having deletion in positions R181 and G182, or positions H183 and G184.

Additional amylases which can be used are those having SEQ ID NO: 1, SEQ ID NO: 3, SEQ ID NO: 2 or SEQ ID NO: 7 of WO 96/023873 or variants thereof having 90% sequence identity to SEQ ID NO: 1, SEQ ID NO: 2, SEQ ID NO: 3 or SEQ ID NO: 7. Preferred variants of SEQ ID NO: 1, SEQ ID NO: 2, SEQ ID NO: 3 or SEQ ID NO: 7 are those having a substitution, a deletion or an insertion in one or more of the following positions: 140, 181, 182, 183, 184, 195, 206, 212, 243, 260, 269, 304 and 476, using SEQ ID 2 of WO 96/023873 for numbering. More preferred variants are those having a deletion in two positions selected from 181, 182, 183 and 184, such as 181 and 182, 182 and 183, or positions 183 and 184. Most preferred amylase variants of SEQ ID NO: 1, SEQ ID NO: 2 or SEQ ID NO: 7 are those having a deletion in positions 183 and 184 and a substitution in one or more of positions 140, 195, 206, 243, 260, 304 and 476.

Other amylases which can be used are amylases having SEQ ID NO: 2 of WO 08/153815, SEQ ID NO: 10 in WO 01/66712 or variants thereof having 90% sequence identity to SEQ ID NO: 2 of WO 08/153815 or 90% sequence identity to SEQ ID NO: 10 in WO 01/66712. Preferred variants of SEQ ID NO: 10 in WO 01/66712 are those having a substitution, a deletion or an insertion in one of more of the following positions: 176, 177, 178, 179, 190, 201, 207, 211 and 264.

Further suitable amylases are amylases having SEQ ID NO: 2 of WO 09/061380 or variants having 90% sequence identity to SEQ ID NO: 2 thereof. Preferred variants of SEQ ID NO: 2 are those having a truncation of the C-terminus and/or a substitution, a deletion or an insertion in one of more of the following positions: Q87, Q98, S125, N128, T131, T165, K178, R180, S181, T182, G183, M201, F202, N225, S243, N272, N282, Y305, R309, D319, Q320, Q359, K444 and G475. More preferred variants of SEQ ID NO: 2 are those having the substitution in one of more of the following positions: Q87E,R, Q98R, S125A, N128C, T131I, T165I, K178L, T182G, M201L, F202Y, N225E,R, N272E,R, S243Q,A,E,D, Y305R, R309A, Q320R, Q359E, K444E and G475K and/or deletion in position R180 and/or S181 or of T182 and/or G183. Most preferred amylase variants of SEQ ID NO: 2 are those having the substitutions:

N128C+K178L+T182G+Y305R+G475K;
N128C+K178L+T182G+F202Y+Y305R+D319T+G475K;
S125A+N128C+K178L+T182G+Y305R+G475K; or
S125A+N128C+T131I+T165I+K178L+T182G+Y305R+G475K wherein the variants are C-terminally truncated and

optionally further comprises a substitution at position 243 and/or a deletion at position 180 and/or position 181.

Other suitable amylases are the alpha-amylase having SEQ ID NO: 12 in WO01/66712 or a variant having at least 90% sequence identity to SEQ ID NO: 12. Preferred amylase variants are those having a substitution, a deletion or an insertion in one of more of the following positions of SEQ ID NO: 12 in WO01/66712: R28, R118, N174; R181, G182, D183, G184, G186, W189, N195, M202, Y298, N299, K302, S303, N306, R310, N314; R320, H324, E345, Y396, R400, W439, R444, N445, K446, Q449, R458, N471, N484. Particular preferred amylases include variants having a deletion of D183 and G184 and having the substitutions R118K, N195F, R320K and R458K, and a variant additionally having substitutions in one or more position selected from the group: M9, G149, G182, G186, M202, T257, Y295, N299, M323, E345 and A339, most preferred a variant that additionally has substitutions in all these positions.

Other examples are amylase variants such as those described in WO2011/098531, WO2013/001078 and WO2013/001087.

Commercially available amylases are Duramyl™, Termamyl™, Fungamyl™, Stainzyme ™, Stainzyme Plus™, Natalase™, Liquozyme X and BAN™ (from Novozymes A/S), and Rapidase™, Purastar™/Effectenz™, Powerase and Preferenz S100 (from Genencor International Inc./DuPont).

**Peroxidases/Oxidases**

A peroxidase according to the invention is a peroxidase enzyme comprised by the enzyme classification EC 1.11.1.7, as set out by the Nomenclature Committee of the International Union of Biochemistry and Molecular Biology (IUBMB), or any fragment obtained therefrom, exhibiting peroxidase activity.

Suitable peroxidases include those of plant, bacterial or fungal origin. Chemically modified or protein engineered mutants are included. Examples of useful peroxidases include peroxidases from *Coprinopsis, e.g.,* from *C. cinerea* (EP 179,486), and variants thereof as those described in WO 93/24618, WO 95/10602, and WO 98/15257.

A peroxidase according to the invention also includes a haloperoxidase enzyme, such as chloroperoxidase, bromoperoxidase and compounds exhibiting chloroperoxidase or bromoperoxidase activity. Haloperoxidases are classified according to their specificity for halide ions. Chloroperoxidases (E.C. 1.11.1.10) catalyze formation of hypochlorite from chloride ions.

In an embodiment, the haloperoxidase of the invention is a chloroperoxidase. Preferably, the haloperoxidase is a vanadium haloperoxidase, i.e., a vanadate-containing haloperoxidase. In a preferred method of the present invention the vanadate-containing haloperoxidase is combined with a source of chloride ion.

Haloperoxidases have been isolated from many different fungi, in particular from the fungus group dematiaceous hyphomycetes, such as *Caldariomyces, e.g., C. fumago, Alternaria, Curvularia, e.g., C. verruculosa* and *C. inaequalis, Drechslera, Ulocladium* and *Botrytis.*

Haloperoxidases have also been isolated from bacteria such as *Pseudomonas, e.g., P. pyrrocinia* and *Streptomyces, e.g., S. aureofaciens.*

In an preferred embodiment, the haloperoxidase is derivable from *Curvularia* sp., in particular *Curvularia verruculosa* or *Curvularia inaequalis,* such as *C. inaequalis* CBS 102.42 as described in WO 95/27046; or *C. verruculosa* CBS 147.63 or *C. verruculosa* CBS 444.70 as described in WO 97/04102; or from *Drechslera hartlebii* as described in WO 01/79459, *Dendryphiella salina* as described in WO 01/79458, *Phaeotrichoconis crotalarie* as described in WO 01/79461, or *Geniculosporium* sp. as described in WO 01/79460.

An oxidase according to the invention include, in particular, any laccase enzyme comprised by the enzyme classification EC 1.10.3.2, or any fragment obtained therefrom exhibiting laccase activity, or a compound exhibiting a similar activity, such as a catechol oxidase (EC 1.10.3.1), an o-aminophenol oxidase (EC 1.10.3.4), or a bilirubin oxidase (EC 1.3.3.5).

Preferred laccase enzymes are enzymes of microbial origin. The enzymes may be obtained from plants, bacteria or fungi (including filamentous fungi and yeasts).

Suitable examples from fungi include a laccase derivable from a strain of *Aspergillus, Neurospora, e.g., N. crassa, Podospora, Botrytis, Collybia, Fomes, Lentinus, Pleurotus, Trametes, e.g., T. villosa* and *T. versicolor, Rhizoctonia, e.g., R. solani, Coprinopsis, e.g., C. cinerea, C. comatus, C. friesii,* and *C. plicatilis, Psathyrella, e.g., P. condelleana, Panaeolus, e.g., P. papilionaceus, Myceliophthora, e.g., M. thermophila, Schytalidium, e.g., S. thermophilum, Polyporus, e.g., P. pinsitus, Phlebia, e.g., P. radiata* (WO 92/01046), or *Coriolus, e.g., C. hirsutus* (JP 2238885).

Suitable examples from bacteria include a laccase derivable from a strain of *Bacillus.*

A laccase obtained from *Coprinopsis* or *Myceliophthora* is preferred; in particular a laccase obtained from *Coprinopsis cinerea,* as disclosed in WO 97/08325; or from *Myceliophthora thermophila,* as disclosed in WO 95/33836.

The detergent enzyme(s) may be included in a detergent composition by adding separate additives containing one or more enzymes, or by adding a combined additive comprising all of these enzymes. A detergent additive of the invention, *i.e.,* a separate additive or a combined additive, can be formulated, for example, as a granulate, liquid, slurry,

etc. Preferred detergent additive formulations are granulates, in particular non-dusting granulates, liquids, in particular stabilized liquids, or slurries.

Non-dusting granulates may be produced, *e.g.* as disclosed in US 4,106,991 and 4,661,452 and may optionally be coated by methods known in the art. Examples of waxy coating materials are poly(ethylene oxide) products (polyethyleneglycol, PEG) with mean molar weights of 1000 to 20000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in GB 1483591. Liquid enzyme preparations may, for instance, be stabilized by adding a polyol such as propylene glycol, a sugar or sugar alcohol, lactic acid or boric acid according to established methods. Protected enzymes may be prepared according to the method disclosed in EP 238,216.

**Other materials**

Any detergent components known in the art for use in detergents may also be utilized. Other optional detergent components include anti-corrosion agents, anti-shrink agents, anti-soil redeposition agents, anti-wrinkling agents, bactericides, binders, corrosion inhibitors, disintegrants/disintegration agents, dyes, enzyme stabilizers (including boric acid, borates, CMC, and/or polyols such as propylene glycol), fabric conditioners including clays, fillers/processing aids, fluorescent whitening agents/optical brighteners, foam boosters, foam (suds) regulators, perfumes, soil-suspending agents, softeners, suds suppressors, tarnish inhibitors, and wicking agents, either alone or in combination. Any ingredient known in the art for use in detergents may be utilized. The choice of such ingredients is well within the skill of the artisan.

**Dispersants**

The detergent compositions of the present invention can also contain dispersants. In particular powdered detergents may comprise dispersants. Suitable water-soluble organic materials include the homo- or co-polymeric acids or their salts, in which the polycarboxylic acid comprises at least two carboxyl radicals separated from each other by not more than two carbon atoms. Suitable dispersants are for example described in Powdered Detergents, Surfactant science series volume 71, Marcel Dekker, Inc.

**Dye Transfer Inhibiting Agents**

The detergent compositions of the present invention may also include one or more dye transfer inhibiting agents. Suitable polymeric dye transfer inhibiting agents include, but are not limited to, polyvinylpyrrolidone polymers, polyamine *N*-oxide polymers, copolymers of N-vinylpyrrolidone and *N*-vinylimidazole, polyvinyloxazolidones and polyvinylimidazoles or mixtures thereof. When present in a subject composition, the dye transfer inhibiting agents may be present at levels from about 0.0001 % to about 10%, from about 0.01% to about 5% or even from about 0.1% to about 3% by weight of the composition.

**Fluorescent whitening agent**

The detergent compositions of the present invention will preferably also contain additional components that may tint articles being cleaned, such as fluorescent whitening agent or optical brighteners. Where present the brightener is preferably at a level of about 0.01% to about 0.5%. Any fluorescent whitening agent suitable for use in a laundry detergent composition may be used in the composition of the present invention. The most commonly used fluorescent whitening agents are those belonging to the classes of diaminostilbene-sulfonic acid derivatives, diarylpyrazoline derivatives and bisphenyl-distyryl derivatives. Examples of the diaminostilbene-sulfonic acid derivative type of fluorescent whitening agents include the sodium salts of: 4,4'-bis-(2-diethanolamino-4-anilino-s-triazin-6-ylamino) stilbene-2,2'-disulfonate, 4,4'-bis-(2,4-dianilino-s-triazin-6-ylamino) stilbene-2.2'-disulfonate, 4,4'-bis-(2-anilino-4-(*N*-methyl-*N*-2-hydroxy-ethylamino)-s-triazin-6-ylamino) stilbene-2,2'-disulfonate, 4,4'-bis-(4-phenyl-1,2,3-triazol-2-yl)stilbene-2,2'-disulfonate and sodium 5-(2*H*-naphtho[1,2-*d*][1,2,3]triazol-2-yl)-2-[(*E*)-2-phenylvinyl]benzenesulfonate. Preferred fluorescent whitening agents are Tinopal DMS and Tinopal CBS available from Ciba-Geigy AG, Basel, Switzerland. Tinopal DMS is the disodium salt of 4,4'-bis-(2-morpholino-4-anilino-s-triazin-6-ylamino) stilbene-2,2'-disulfonate. Tinopal CBS is the disodium salt of 2,2'-bis-(phenyl-styryl)-disulfonate. Also preferred are fluorescent whitening agents is the commercially available Parawhite KX, supplied by Paramount Minerals and Chemicals, Mumbai, India. Other fluorescers suitable for use in the invention include the 1-3-diaryl pyrazolines and the 7-alkylaminocoumarins.

Suitable fluorescent brightener levels include lower levels of from about 0.01, from 0.05, from about 0.1 or even from about 0.2 wt % to upper levels of 0.5 or even 0.75 wt%.

**Soil release polymers**

The detergent compositions of the present invention may also include one or more soil release polymers which aid the removal of soils from fabrics such as cotton and polyester based fabrics, in particular the removal of hydrophobic soils from polyester based fabrics. The soil release polymers may for example be nonionic or anionic terephthalte based polymers, polyvinyl caprolactam and related copolymers, vinyl graft copolymers, polyester polyamides see for example Chapter 7 in Powdered Detergents, Surfactant science series volume 71, Marcel Dekker, Inc. Another type of soil release polymers are amphiphilic alkoxylated grease cleaning polymers comprising a core structure and a plurality of alkoxylate groups attached to that core structure. The core structure may comprise a polyalkylenimine structure or a polyalkanolamine structure as described in detail in WO 2009/087523 (hereby incorporated by reference). Furthermore random graft co-polymers are suitable soil release polymers. Suitable graft co-polymers are described in more detail in WO 2007/138054, WO 2006/108856 and WO 2006/113314 (hereby incorporated by reference). Other soil release polymers are substituted polysaccharide structures especially substituted cellulosic structures such as modified cellulose derivatives such as those described in EP 1867808 or WO 2003/040279 (both are hereby incorporated by reference). Suitable cellulosic polymers include cellulose, cellulose ethers, cellulose esters, cellulose amides and mixtures thereof. Suitable cellulosic polymers include anionically modified cellulose, nonionically modified cellulose, cationically modified cellulose, zwitterionically modified cellulose, and mixtures thereof. Suitable cellulosic polymers include methyl cellulose, carboxy methyl cellulose, ethyl cellulose, hydroxyl ethyl cellulose, hydroxyl propyl methyl cellulose, ester carboxy methyl cellulose, and mixtures thereof.

**Anti-redeposition agents**

The detergent compositions of the present invention may also include one or more anti-redeposition agents such as carboxymethylcellulose (CMC), polyvinyl alcohol (PVA), polyvinylpyrrolidone (PVP), polyoxyethylene and/or polyethyleneglycol (PEG), homopolymers of acrylic acid, copolymers of acrylic acid and maleic acid, and ethoxylated polyethyleneimines. The cellulose based polymers described under soil release polymers above may also function as anti-redeposition agents.

**Rheology Modifiers**

The detergent compositions of the present invention may also include one or more rheology modifiers, structurants or thickeners, as distinct from viscosity reducing agents. The rheology modifiers are selected from the group consisting of non-polymeric crystalline, hydroxyfunctional materials, polymeric rheology modifiers which impart shear thinning characteristics to the aqueous liquid matrix of a liquid detergent composition. The rheology and viscosity of the detergent can be modified and adjusted by methods known in the art, for example as shown in EP 2169040.

Other suitable adjunct materials include, but are not limited to, anti-shrink agents, anti-wrinkling agents, bactericides, binders, carriers, dyes, enzyme stabilizers, fabric softeners, fillers, foam regulators, hydrotropes, perfumes, pigments, sod suppressors, solvents, and structurants for liquid detergents and/or structure elasticizing agents.

**Formulation of detergent products**

The detergent composition of the invention may be in any convenient form, e.g., a bar, a homogenous tablet, a tablet having two or more layers, a pouch having one or more compartments, a regular or compact powder, a granule, a paste, a gel, or a regular, compact or concentrated liquid.

Pouches can be configured as single or multicompartments. It can be of any form, shape and material which is suitable for hold the composition, e.g. without allowing the release of the composition to release of the composition from the pouch prior to water contact. The pouch is made from water soluble film which encloses an inner volume. Said inner volume can be divided into compartments of the pouch. Preferred films are polymeric materials preferably polymers which are formed into a film or sheet. Preferred polymers, copolymers or derivates thereof are selected polyacrylates, and water soluble acrylate copolymers, methyl cellulose, carboxy methyl cellulose, sodium dextrin, ethyl cellulose, hydroxyethyl cellulose, hydroxypropyl methyl cellulose, malto dextrin, poly methacrylates, most preferably polyvinyl alcohol copolymers and, hydroxypropyl methyl cellulose (HPMC). Preferably the level of polymer in the film for example PVA is at least about 60%. Preferred average molecular weight will typically be about 20,000 to about 150,000. Films can also be of blended compositions comprising hydrolytically degradable and water soluble polymer blends such as polylactide and polyvinyl alcohol (known under the Trade reference M8630 as sold by MonoSol LLC, Indiana, USA) plus plasticisers like glycerol, ethylene glycerol, propylene glycol, sorbitol and mixtures thereof. The pouches can comprise a solid laundry cleaning composition or part components and/or a liquid cleaning composition or part components separated by the water soluble film. The compartment for liquid components can be different in composition than compartments containing solids: US2009/0011970 A1.

Detergent ingredients can be separated physically from each other by compartments in water dissolvable pouches or in different layers of tablets. Thereby negative storage interaction between components can be avoided. Different dissolution profiles of each of the compartments can also give rise to delayed dissolution of selected components in the wash solution.

A liquid or gel detergent , which is not unit dosed, may be aqueous, typically containing at least 20% by weight and up to 95% water, such as up to about 70% water, up to about 65% water, up to about 55% water, up to about 45% water, up to about 35% water. Other types of liquids, including without limitation, alkanols, amines, diols, ethers and polyols may be included in an aqueous liquid or gel. An aqueous liquid or gel detergent may contain from 0-30% organic solvent.

A liquid or gel detergent may be non-aqueous.

**Laundry soap bars**

The DNase of the invention may be added to laundry soap bars and used for hand washing laundry, fabrics and/or textiles. The term laundry soap bar includes laundry bars, soap bars, combo bars, syndet bars and detergent bars. The types of bar usually differ in the type of surfactant they contain, and the term laundry soap bar includes those containing soaps from fatty acids and/or synthetic soaps. The laundry soap bar has a physical form which is solid and not a liquid, gel or a powder at room temperature. The term solid is defined as a physical form which does not significantly change over time, i.e. if a solid object (e.g. laundry soap bar) is placed inside a container, the solid object does not change to fill the container it is placed in. The bar is a solid typically in bar form but can be in other solid shapes such as round or oval.

The laundry soap bar may contain one or more additional enzymes, protease inhibitors such as peptide aldehydes (or hydrosulfite adduct or hemiacetal adduct), boric acid, borate, borax and/or phenylboronic acid derivatives such as 4-formylphenylboronic acid, one or more soaps or synthetic surfactants, polyols such as glycerine, pH controlling compounds such as fatty acids, citric acid, acetic acid and/or formic acid, and/or a salt of a monovalent cation and an organic anion wherein the monovalent cation may be for example $Na^+$, $K^+$ or $NH_4^+$ and the organic anion may be for example formate, acetate, citrate or lactate such that the salt of a monovalent cation and an organic anion may be, for example, sodium formate.

The laundry soap bar may also contain complexing agents like EDTA and HEDP, perfumes and/or different type of fillers, surfactants e.g. anionic synthetic surfactants, builders, polymeric soil release agents, detergent chelators, stabilizing agents, fillers, dyes, colorants, dye transfer inhibitors, alkoxylated polycarbonates, suds suppressers, structurants, binders, leaching agents, bleaching activators, clay soil removal agents, anti-redeposition agents, polymeric dispersing agents, brighteners, fabric softeners, perfumes and/or other compounds known in the art.

The laundry soap bar may be processed in conventional laundry soap bar making equipment such as but not limited to: mixers, plodders, e.g a two stage vacuum plodder, extruders, cutters, logo-stampers, cooling tunnels and wrappers. The invention is not limited to preparing the laundry soap bars by any single method. The premix of the invention may be added to the soap at different stages of the process. For example, the premix containing a soap, DNase, optionally one or more additional enzymes, a protease inhibitor, and a salt of a monovalent cation and an organic anion may be prepared and and the mixture is then plodded. The DNase and optional additional enzymes may be added at the same time as the protease inhibitor for example in liquid form. Besides the mixing step and the plodding step, the process may further comprise the steps of milling, extruding, cutting, stamping, cooling and/or wrapping.

**Formulation of enzyme in co-granule**

The DNase may be formulated as a granule for example as a co-granule that combines one or more enzymes. Each enzyme will then be present in more granules securing a more uniform distribution of enzymes in the detergent. This also reduces the physical segregation of different enzymes due to different particle sizes. Methods for producing multi-enzyme co-granulates for the detergent industry are disclosed in the IP.com disclosure IPCOM000200739D.

Another example of formulation of enzymes by the use of co-granulates are disclosed in WO 2013/188331, which relates to a detergent composition comprising (a) a multi-enzyme co- granule; (b) less than 10 wt zeolite (anhydrous basis); and (c) less than 10 wt phosphate salt (anhydrous basis), wherein said enzyme co-granule comprises from 10 to 98 wt% moisture sink component and the composition additionally comprises from 20 to 80 wt% detergent moisture sink component. WO 2013/188331 also relates to a method of treating and/or cleaning a surface, preferably a fabric surface comprising the steps of (i) contacting said surface with the detergent composition as claimed and described herein in an aqueous wash liquor, (ii) rinsing and/or drying the surface.

The multi-enzyme co-granule may comprise a DNase and (a) one or more enzymes selected from the group consisting of first- wash lipases, cleaning cellulases, xyloglucanases, perhydrolases, peroxidases, lipoxygenases, laccases and mixtures thereof; and (b) one or more enzymes selected from the group consisting of hemicellulases, proteases, care cellulases, cellobiose dehydrogenases, xylanases, phospho lipases, esterases, cutinases, pectinases, mannanases, pectate lyases, keratinases, reductases, oxidases, phenoloxidases, ligninases, pullulanases, tannases, pentosanases, lichenases glucanases, arabinosidases, hyaluronidase, chondroitinase, amylases, and mixtures thereof.

The invention is further summarized in the following paragraphs:

1. Use of a polypeptide having DNase activity for preventing, reducing and/or removing a biofilm from an item, wherein the item is a hard surface.
2. Use according to paragraph 1 for preventing, reducing and/or removing stickiness of the item.
3. Use according to any of paragraphs 1 or 2 for pretreating biofilm stains on the item.
4. Use according to any of paragraphs 1-3 for preventing, reducing and/or removing adherence of soil to the item.
5. Use according to any of the preceding paragraphs, wherein malodor is prevented, reduced and/or removed from the item.
6. Use according to any of the preceding paragraphs, wherein the malodor is caused by E-2-nonenal.
7. Use according to any of the preceding paragraphs, wherein the amount of E-2-nonenal present on a wet item is prevented, reduced or removed.
8. Use according to any of the preceding paragraphs, wherein the amount of E-2-nonenal present on the dried item is prevented, reduced and/or removed.
9. Use according to any of the preceding paragraphs, wherein the polypeptide having DNase activity is sprayed onto the item.
10. Use according to any of the preceding paragraphs, wherein the item is exposed to a liquid solution comprising a polypeptide having DNase activity.
11. Use according to paragraph 10, wherein the liquid solution is a wash liquor.
12. Use according to any of the preceding paragraphs, wherein the polypeptide having DNase activity is of animal, vegetable or microbial origin.
13. Use according to paragraph 12, wherein the polypeptide is of human origin.
14. Use according to paragraph 12, wherein the polypeptide is obtained from mung bean.
15. Use according to paragraph 12, wherein the polypeptide is of bacterial or fungal origin.
16. Use according to paragraph 15, wherein the polypeptide is of fungal origin and the polypeptide is selected from the group consisting of:

    a) a polypeptide having at least 60% sequence identity to the mature polypeptide of SEQ ID NO: 2, a polypeptide having at least 60% sequence identity to the mature polypeptide of SEQ ID NO: 3, a polypeptide having at least 60% sequence identity to the mature polypeptide of SEQ ID NO: 5 or a polypeptide having at least 60% sequence identity to the mature polypeptide of SEQ ID NO: 8;
    b) a polypeptide encoded by a polynucleotide that hybridizes under low stringency conditions with

        i. the mature polypeptide coding sequence of SEQ ID NO: 1 or the mature polypeptide coding sequence of SEQ ID NO: 4;
        ii. the cDNA sequence thereof; or
        iii. the full-length complement of (i) or (ii);

    c) a polypeptide encoded by a polynucleotide having at least 60% sequence identity to the mature polypeptide coding sequence of SEQ ID NO: 1 or the cDNA sequence thereof or a polypeptide encoded by a polynucleotide having at least 60% sequence identity to the mature polypeptide coding sequence of SEQ ID NO: 4 or the cDNA sequence thereof;
    d) a variant of the mature polypeptide of SEQ ID NO: 2 comprising a substitution, deletion, and/or insertion at one or more positions, a variant of the mature polypeptide of SEQ ID NO: 3 comprising a substitution, deletion, and/or insertion at one or more positions, a variant of the mature polypeptide of SEQ ID NO: 5 comprising a substitution, deletion, and/or insertion at one or more positions or a variant of the mature polypeptide of SEQ ID NO: 8 comprising a substitution, deletion, and/or insertion at one or more positions ; and
    e) a fragment of the polypeptide of (a), (b), (c), or (d) that has DNase activity.

17. Use according to paragraph 15, wherein the polypeptide is of bacterial origin and the polypeptide is selected from the group consisting of:

    a) a polypeptide having at least 60% sequence identity to the mature polypeptide of SEQ ID NO: 6 or a polypeptide having at least 60% sequence identity to the mature polypeptide of SEQ ID NO: 7;

b) a variant of the mature polypeptide of SEQ ID NO: 6 comprising a substitution, deletion, and/or insertion at one or more positions or a variant of the mature polypeptide of SEQ ID NO: 7 comprising a substitution, deletion, and/or insertion at one or more positions; and

c) a fragment of the polypeptide of (a) or (b) that has DNase activity.

18. Use according to any of paragraphs 16-17, wherein the polypeptide is having at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% sequence identity to the mature polypeptide of SEQ ID NO: 2, to the mature polypeptide of SEQ ID NO: 3, to the mature polypeptide of SEQ ID NO: 5, to the mature polypeptide of SEQ ID NO: 6, to the mature polypeptide of SEQ ID NO: 7 or, to the mature polypeptide of SEQ ID NO: 8.

19. Use according to any of paragraphs 16-18, wherein the polypeptide comprises or consists of SEQ ID NO: 2 or the mature polypeptide of SEQ ID NO: 2, the polypeptide comprises or consists of SEQ ID NO: 3 or the mature polypeptide of SEQ ID NO: 3, the polypeptide comprises or consists of SEQ ID NO: 5 or the mature polypeptide of SEQ ID NO: 5, the polypeptide comprises or consists of SEQ ID NO: 6 or the mature polypeptide of SEQ ID NO: 6, the polypeptide comprises or consists of SEQ ID NO: 7 or the mature polypeptide of SEQ ID NO: 7 or the polypeptide comprises or consists of SEQ ID NO: 8 or the mature polypeptide of SEQ ID NO: 8.

20. Use according to any of paragraphs 16-19, wherein the mature polypeptide is amino acids 1 to 206 of SEQ ID NO: 2, amino acids 1 to 206 of SEQ ID NO: 3, amino acids 1 to 188 of SEQ ID NO: 5, amino acids 1 to 110 of SEQ ID NO: 6, amino acids 1 to 109 of SEQ ID NO: 7 or amino acids 1 to 206 of SEQ ID NO: 8.

21. Use according to any of paragraphs 16-20, wherein the polypeptide is a variant of the mature polypeptide of SEQ ID NO: 2, SEQ ID NO: 3, SEQ ID NO: 5, SEQ ID NO: 6, SEQ ID NO: 7 or SEQ ID NO: 8, wherein the variant comprises a substitution, deletion, and/or insertion at one or more positions or a variant of the mature polypeptide of SEQ ID NO: 2, SEQ ID NO: 3, SEQ ID NO: 5, SEQ ID NO: 6, SEQ ID NO: 7 or SEQ ID NO: 8 which comprises a substitution, deletion, and/or insertion at one or more positions.

22. Use according to any of paragraphs 16-21, wherein the polypeptide is a fragment of of SEQ ID NO: 2, SEQ ID NO: 3, SEQ ID NO: 5, SEQ ID NO: 6, SEQ ID NO: 7 or SEQ ID NO: 8, wherein the fragment has DNase activity.

23. Use according to any of preceding paragraphs, wherein the item is made of metal, glass, rubber, plastic, PVC, acrylics, nylon, wood, abs, acryl, polycarbonate, pvc, polypropylene, enamel, galvanized material, zinc, fajence, ceramics, earthenware, china or porcelain.

24. Use according to paragraph 23, wherein the item is made of iron, copper, magnesium, chromium, nickel, aluminium, titanium, molybdenum lead, gold, silver, brass, tin, or an alloy thereof.

25. Use according to any of paragraphs 23-24, wherein the item is made of stainless steel.

26. Use according to paragraph 23, wherein the item is made of rubber such as natural rubber or synthetic rubber.

27. Use according to any of the preceding paragraphs, wherein the biofilm comprises at least one strain of *Brevundimonas sp.,* at least one strain of *Pseudomonas fluorescens* and/or at least one strain of *Pseudomonas alcaliphila.*

28. A detergent composition comprising a polypeptide having deoxyribonuclease (DNase) activity and a metal care agent.

29. Composition according to paragraph 28, wherein the metal care agent is selected from the group consisting of:

a) benzatriazoles, including benzotriazole or bis-benzotriazole and substituted derivatives thereof, which derivatives include substituents with linear or branch-chain Ci-C20- alkyl groups and hydroxyl, thio, phenyl or halogen such as fluorine, chlorine, bromine and iodine;

b) metal salts and complexes chosen from the group consisting of zinc, manganese, titanium, zirconium, hafnium, vanadium, cobalt, gallium and cerium salts and/or complexes, the metals being in one of the oxidation states II, III, IV, V or VI, such as metal salts and/or metal complexes may be chosen from the group consisting of Mn(II) sulphate, Mn(II) citrate, Mn(II) stearate, Mn(II) acetylacetonate, KTiF6, KZrF6, CoSO4, Co(NOs)2 and Ce(NOs)3, zinc salts, for example zinc sulphate, hydrozincite, zinc acetate or zinc carbonate;

c) silicates, including sodium or potassium silicate, sodium disilicate, sodium metasilicate, crystalline phyllosilicate and mixtures thereof.

30. A detergent composition comprising a polypeptide having deoxyribonuclease (DNase) activity and a strong sequestering builder and/or a builder selected from the group consisting of sodium citrate, citric acid, alcanol amines, sodium carbonate, sodium bicarbonate and Amino-tris-(methylene-phosphonic acid) (AMP).

31. Composition according to any of the preceding composition paragraphs, wherein the composition further comprises surfactants, builders, flocculating aid, chelating agents, dye transfer inhibitors, enzymes, enzyme stabilizers, enzyme inhibitors, catalytic materials, bleach activators, hydrogen peroxide, sources of hydrogen peroxide, preformed peracids, polymeric dispersing agents, clay soil removal/anti-redeposition agents, brighteners, suds suppressors, dyes, perfumes, structure elasticizing agents, fabric softeners, carriers, hydrotropes, builders and co-builders, fabric hueing agents, anti-foaming agents, dispersants, processing aids, bacteriocides, fungicides and/or pigments or a mixture thereof.

32. Detergent composition according to any of the preceding composition paragraphs, wherein the detergent component is a surfactant.

33. Detergent composition according to any of the preceding composition paragraphs, wherein the detergent component is a builder.

34. Detergent composition according to any of the preceding composition paragraphs, wherein the detergent component is a clay soil removal/anti-redeposition agents.

35. Composition according to any of the preceding composition paragraphs, wherein the composition further comprises one or more enzymes selected from the group consisting of proteases, lipases, cutinases, amylases, carbohydrases, cellulases, pectinases, mannanases, arabinases, galactanases, xylanases, peroxidase and oxidases.

36. Composition according to any of the preceding composition paragraphs, wherein the polypeptide having DNase activity is of animal, vegetable or microbial origin.

37. Composition according to paragraph 36, wherein the polypeptide is of human origin.

38. Composition according to paragraph 36, wherein the polypeptide is obtained from mung bean.

39. Composition according to paragraph 36, wherein the polypeptide is of bacterial or fungal origin.

40. Composition according to paragraph 39, wherein the polypeptide is of fungal origin and the polypeptide is selected from the group consisting of:

a) a polypeptide having at least 60% sequence identity to the mature polypeptide of SEQ ID NO: 2, a polypeptide having at least 60% sequence identity to the mature polypeptide of SEQ ID NO: 3, a polypeptide having at least 60% sequence identity to the mature polypeptide of SEQ ID NO: 5 or a polypeptide having at least 60% sequence identity to the mature polypeptide of SEQ ID NO: 8;

b) a polypeptide encoded by a polynucleotide that hybridizes under low stringency conditions with

i. the mature polypeptide coding sequence of SEQ ID NO: 1 or the mature polypeptide coding sequence of SEQ ID NO: 4;

ii. the cDNA sequence thereof; or

iii. the full-length complement of (i) or (ii);

c) a polypeptide encoded by a polynucleotide having at least 60% sequence identity to the mature polypeptide coding sequence of SEQ ID NO: 1 or the cDNA sequence thereof or a polypeptide encoded by a polynucleotide having at least 60% sequence identity to the mature polypeptide coding sequence of SEQ ID NO: 4 or the cDNA sequence thereof;

d) a variant of the mature polypeptide of SEQ ID NO: 2 comprising a substitution, deletion, and/or insertion at one or more positions, a variant of the mature polypeptide of SEQ ID NO: 3 comprising a substitution, deletion, and/or insertion at one or more positions or a variant of the mature polypeptide of SEQ ID NO: 5 comprising a substitution, deletion, and/or insertion at one or more positions; and

e) a fragment of the polypeptide of (a), (b), (c), or (d) that has DNase activity.

41. Composition according to paragraph 39, wherein the polypeptide is of bacterial origin and the polypeptide is selected from the group consisting of:

a) a polypeptide having at least 60% sequence identity to the mature polypeptide of SEQ ID NO: 6 or a polypeptide having at least 60% sequence identity to the mature polypeptide of SEQ ID NO: 7;

b) a variant of the mature polypeptide of SEQ ID NO: 6 comprising a substitution, deletion, and/or insertion at one or more positions or a variant of the mature polypeptide of SEQ ID NO: 7 comprising a substitution, deletion, and/or insertion at one or more positions; and

c) a fragment of the polypeptide of (a) or (b) that has DNase activity.

42. Composition according to any of paragraphs 39-41, wherein the polypeptide is having at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% sequence identity to the mature polypeptide of SEQ ID NO: 2, to the mature polypeptide of SEQ ID NO: 3, to the mature polypeptide of SEQ ID NO: 5, to the mature polypeptide of SEQ ID NO: 6, to the mature polypeptide of SEQ ID NO: 7 or to the mature polypeptide of SEQ ID NO: 8.

43. Composition according to any of paragraphs 39-42, wherein the polypeptide comprises or consists of SEQ ID

NO: 2 or the mature polypeptide of SEQ ID NO: 2, the polypeptide comprises or consists of SEQ ID NO: 3 or the mature polypeptide of SEQ ID NO: 3, the polypeptide comprises or consists of SEQ ID NO: 5 or the mature polypeptide of SEQ ID NO: 5, the polypeptide comprises or consists of SEQ ID NO: 6 or the mature polypeptide of SEQ ID NO: 6, the polypeptide comprises or consists of SEQ ID NO: 7 or the mature polypeptide of SEQ ID NO: 7 or the polypeptide comprises or consists of SEQ ID NO: 8 or the mature polypeptide of SEQ ID NO: 8.

44. Composition according to any of paragraphs 39-43, wherein the mature polypeptide is amino acids 1 to 206 of SEQ ID NO: 2, amino acids 1 to 206 of SEQ ID NO: 3, amino acids 1 to 188 of SEQ ID NO: 5, amino acids 1 to 110 of SEQ ID NO: 6, amino acids 1 to 109 of SEQ ID NO: 7 or amino acids 1 to 206 of SEQ ID NO: 8.

45. Composition according to any of paragraphs 39-44, wherein the polypeptide is a variant of the mature polypeptide of SEQ ID NO: 2, SEQ ID NO: 3, SEQ ID NO: 5, SEQ ID NO: 6, SEQ ID NO: 7 or SEQ ID NO: 8, wherein the variant comprises a substitution, deletion, and/or insertion at one or more positions or a variant of the mature polypeptide of SEQ ID NO: 2, SEQ ID NO: 3, SEQ ID NO: 5, SEQ ID NO: 6, SEQ ID NO: 7 or SEQ ID NO: 8 which comprises a substitution, deletion, and/or insertion at one or more positions.

46. Composition according to any of paragraphs 39-45, wherein the polypeptide is a fragment of of SEQ ID NO: 2, SEQ ID NO: 3, SEQ ID NO: 5, SEQ ID NO: 6, SEQ ID NO: 7 or SEQ ID NO: 8, wherein the fragment has DNase activity.

47. Composition according to any of the preceding composition paragraphs, wherein biofilm is prevented, reduced or removed from a surface.

48. Composition according to paragraph 47, wherein the biofilm comprises at least one strain of *Brevundimonas sp.,* at least one strain of *Pseudomonas fluorescens* and/or at least one strain of *Pseudomonas alcaliphila.*

49. Composition according to any of the preceding composition paragraphs, wherein the surface is a hard surface.

50. Composition according to any of the preceding composition paragraphs, wherein the composition is a bar, a homogenous tablet, a tablet having two or more layers, a pouch having one or more compartments, a regular or compact powder, a granule, a paste, a gel, or a regular, compact or concentrated liquid.

51. Composition according to any of the preceding composition paragraphs, wherein the composition is a liquid detergent, a powder detergent or a granule detergent.

52. Detergent composition according to paragraph 51, wherein the composition is a liquid detergent composition, comprising a surfactant and a detergent builder in a total concentration of at least 3% by weight, and a detergent enzyme containing microcapsule, wherein the membrane of the microcapsule is produced by cross-linking of a polybranched polyamine having a molecular weight of more than 1 kDa.

53. Detergent composition according to paragraph 52, wherein the reactive amino groups of the polybranched polyamine constitute at least 15% of the molecular weight.

54. Detergent composition according to any of paragraphs 52-53, wherein the microcapsule is produced by using an acid chloride as crosslinking agent.

55. Detergent composition according to any of paragraphs 52-54, wherein the diameter of the microcapsule is at least, or above, 50 micrometers.

56. Detergent composition according to any of paragraphs 52-55, wherein the microcapsule contains at least 1% by weight of active enzyme.

57. Detergent composition according to any of paragraphs 52-56, which further includes an alcohol, such as a polyol.

58. Detergent composition according to any of paragraphs 52-57, wherein the surfactant is an anionic surfactant.

59. Detergent composition according to any of paragraphs 52-58, which is a liquid laundry or automatic dishwash detergent composition.

60. Detergent composition according to any of paragraphs 52-59, which contains less than 90% by weight of water.

61. Detergent composition according to any of paragraphs 52-60, wherein the detergent enzyme is a polypeptide having DNase activity, protease, amylase, lipase, cellulase, mannanase, pectinase, or oxidoreductase.

62. Detergent composition according to any of paragraphs 52-61, wherein the protease is a metalloprotease or an alkaline serine protease, such as a subtilisin.

63. Detergent composition according to any of paragraphs 52-62, wherein the polypeptide having DNase activity is the polypeptide according to any of paragraphs 47-54.

64. Detergent composition according to any of paragraphs 52-63, wherein the microcapsule is produced by interfacial polymerization using an acid chloride as crosslinking agent.

65. Detergent composition according to any of paragraphs 52-64, wherein the polybranched polyamine is a polyethyleneimine.

66. Detergent composition according to any of paragraphs 52-65, wherein the microcapsule comprises a source of Mg2+, Ca2+, or Zn2+ ions, such as a poorly soluble salt of Mg2+, Ca2+, or Zn2+.

67. Detergent composition according to any of paragraphs 30-66, wherein the strong sequestering builder is selected from the group consisting of ethylenediaminetetraacetic acid (EDTA), methylglycinediacetic acid (MGDA), Nitrilotriacetic acid (NTA), iminodisuccinic acid (IDS), ethylenediaminedisuccinic acid (EDDS), and L-glutamic acid N,N-diacetic acid tetra sodium salt (GLDA), ethylene diamine tetra(methylene phosphonic acid) (EDTMPA), nitrilo trimethylene phosphonic acid (NTMP), diethylenetriamine Penta(Methylene Phosphonic acid) (DTPMP), 1-Hydroxy Ethylidene-1,1-Diphosphonic Acid (HEDP, etidronic acid), triphosphates such as sodium triphosphate (STP or STPP) and pyrophosphate,

68. Detergent composition according to paragraphs 30-67, wherein the strong sequestering builder is a non-phosphorus builder.

69. Detergent composition according to paragraph 68, wherein the strong sequestering agent is selected from the group consisting of ethylenediaminetetraacetic acid (EDTA), methylglycinediacetic acid (MGDA), Nitrilotriacetic acid (NTA),), iminodisuccinic acid (IDS), ethylenediaminedisuccinic acid (EDDS), and L-glutamic acid N,N-diacetic acid tetra sodium salt (GLDA).

70. A cleaning method for preventing, reducing or removing a biofilm from an item comprising the steps of:

a) contacting an item to a composition according to any of paragraphs 28-69 or a liquid solution comprising a polypeptide having DNase activity;
b) completing at least one cleaning cycle; and
c) optionally rinsing the item;

wherein the item is a hard surface.

71. Method according to paragraph 70, wherein the composition according to any of paragraphs 28-69 is comprised in a liquid solution.

72. Method according to paragraph 70, wherein the item is a hard surface, which hard surface is the interior surface of a dishwashing machine or a washing machine for textile.

73. Method according to paragraph 72, wherein the interior surface of a dishwashing machine or washing machine comprises soap intake box, walls, windows, baskets, racks, nozzles, pumps, sump, filters, pipelines, tubes, joints, seals, gaskets, fittings, impellers, drums, drains, traps, coin traps inlet and outlets.

74. Method according to any of the preceding method paragraphs, wherein the hard surface is made of metal, glass, rubber, plastic, PVC, acrylics, ceramics, china or porcelain.

75. Method according to any of the preceding method paragraphs, wherein the item is a dishware.

76. Method according to any of the preceding method paragraphs, wherein the dishware is selected from the group consisting of plates, cups, glasses, bowls, pots, cutlery, spoons, knives, forks, serving utensils, ceramics, plastics, cutting boards, china and glass ware.

77. Method according to any of the preceding method paragraphs, wherein the dishware is cleaned simultaneously with the cleaning of the interior hard surface.

78. Method according to any of the preceding method paragraphs, wherein the liquid solution further comprises antistatic agents, surfactants, builders, flocculating aid, chelating agents, dye transfer inhibitors, enzymes, enzyme stabilizers, enzyme inhibitors, catalytic materials, bleach activators, hydrogen peroxide, sources of hydrogen peroxide, preformed peracids, polymeric dispersing agents, clay soil removal/anti-redeposition agents, brighteners, suds suppressors, dyes, perfumes, structure elasticizing agents, fabric softeners, carriers, hydrotropes, builders and co-builders, fabric hueing agents, anti-foaming agents, dispersants, processing aids, bacteriocides, fungicides and/or pigments or a mixture thereof.

79. Method according to any of the preceding method paragraphs, wherein the liquid solution further comprises one or more enzymes selected from the group consisting of proteases, lipases, cutinases, amylases, carbohydrases, cellulases, pectinases, mannanases, arabinases, galactanases, xylanases and oxidases.

80. Method according to any of the preceding method paragraphs, wherein the pH of the liquid solution is in the range of 1 to 11.

81. Method according to any of the preceding method paragraphs, wherein the pH of the liquid solution is in the range 5.5 to 11, such as in the range of 7 to 9, in the range of 7 to 8 or in the range of 7 to 8.5.

82. Method according to any of the preceding method paragraphs, wherein the temperature of the liquid solution is in the range of 5°C to 95°C, or in the range of 10°C to 80°C, in the range of 10°C to 70°C, in the range of 10°C to 60°C, in the range of 10°C to 50°C, in the range of 15°C to 40°C or in the range of 20°C to 30°C.

83. Method according to any of the preceding method paragraphs, wherein the temperature of the liquid solution is 30°C.

84. Method according to any of the preceding method paragraphs, wherein the item is rinsed after being exposed to the liquid solution.

85. Method according to any of the preceding method paragraphs, wherein the item is rinsed with water or with water comprising a conditioner.

86. Method according to any of the preceding method paragraphs, wherein the polypeptide having DNase activity is of animal, vegetable or microbial origin.

87. Method according to paragraph 86, wherein the polypeptide is of human origin.
88. Method according to paragraph 86, wherein the polypeptide is obtained from mung bean.
89. Method according to paragraph 86, wherein the polypeptide is of bacterial or fungal origin.
90. Method according to paragraph 89, wherein the polypeptide is of fungal origin and the polypeptide is selected from the group consisting of:

a) a polypeptide having at least 60% sequence identity to the mature polypeptide of SEQ ID NO: 2, a polypeptide having at least 60% sequence identity to the mature polypeptide of SEQ ID NO: 3, a polypeptide having at least 60% sequence identity to the mature polypeptide of SEQ ID NO: 5 or a polypeptide having at least 60% sequence identity to the mature polypeptide of SEQ ID NO: 8;
b) a polypeptide encoded by a polynucleotide that hybridizes under low stringency conditions with

i. the mature polypeptide coding sequence of SEQ ID NO: 1 or the mature polypeptide coding sequence of SEQ ID NO: 4;
ii. the cDNA sequence thereof, or
iii. the full-length complement of (i) or (ii);

c) a polypeptide encoded by a polynucleotide having at least 60% sequence identity to the mature polypeptide coding sequence of SEQ ID NO: 1 or the cDNA sequence thereof or a polypeptide encoded by a polynucleotide having at least 60% sequence identity to the mature polypeptide coding sequence of SEQ ID NO: 4 or the cDNA sequence thereof;
d) a variant of the mature polypeptide of SEQ ID NO: 2 comprising a substitution, deletion, and/or insertion at one or more positions, a variant of the mature polypeptide of SEQ ID NO: 3 comprising a substitution, deletion, and/or insertion at one or more positions or a variant of the mature polypeptide of SEQ ID NO: 5 comprising a substitution, deletion, and/or insertion at one or more positions; and
e) a fragment of the polypeptide of (a), (b), (c), or (d) that has DNase activity.

91. Method according to paragraph 89, wherein the polypeptide is of bacterial origin and the polypeptide is selected from the group consisting of:

a) a polypeptide having at least 60% sequence identity to the mature polypeptide of SEQ ID NO: 6 or a polypeptide having at least 60% sequence identity to the mature polypeptide of SEQ ID NO: 7;
b) a variant of the mature polypeptide of SEQ ID NO: 6 comprising a substitution, deletion, and/or insertion at one or more positions or a variant of the mature polypeptide of SEQ ID NO: 7 comprising a substitution, deletion, and/or insertion at one or more positions; and
c) a fragment of the polypeptide of (a) or (b) that has DNase activity.

92. Method according to any of paragraphs 89-91, wherein the polypeptide is having at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% sequence identity to the mature polypeptide of SEQ ID NO: 2, to the mature polypeptide of SEQ ID NO: 3, to the mature polypeptide of SEQ ID NO: 5, or to the mature polypeptide of SEQ ID NO: 6, to the mature polypeptide of SEQ ID NO: 7 or to the mature polypeptide of SEQ ID NO: 8.
93. Method according to any of paragraphs 89-92, wherein the polypeptide comprises or consists of SEQ ID NO: 2 or the mature polypeptide of SEQ ID NO: 2, the polypeptide comprises or consists of SEQ ID NO: 3 or the mature polypeptide of SEQ ID NO: 3, the polypeptide comprises or consists of SEQ ID NO: 5 or the mature polypeptide of SEQ ID NO: 5, the polypeptide comprises or consists of SEQ ID NO: 6 or the mature polypeptide of SEQ ID NO: 6, the polypeptide comprises or consists of SEQ ID NO: 7 or the mature polypeptide of SEQ ID NO: 7 or the polypeptide comprises or consists of SEQ ID NO: 8 or the mature polypeptide of SEQ ID NO: 8.
94. Method according to any of paragraphs 89-93, wherein the mature polypeptide is amino acids 1 to 206 of SEQ ID NO: 2, amino acids 1 to 206 of SEQ ID NO: 3, amino acids 1 to 188 of SEQ ID NO: 5, amino acids 1 to 110 of SEQ ID NO: 6, amino acids 1 to 109 of SEQ ID NO: 7 or the mature polypeptide is amino acids 1 to 206 of SEQ ID NO: 8.
95. Method according to any of paragraphs 89-94, wherein the polypeptide is a variant of the mature polypeptide of SEQ ID NO: 2, SEQ ID NO: 3, SEQ ID NO: 5, SEQ ID NO: 6, SEQ ID NO: 7 or SEQ ID NO: 3, wherein the variant comprises a substitution, deletion, and/or insertion at one or more positions or a variant of the mature polypeptide of SEQ ID NO: 2, SEQ ID NO: 3, SEQ ID NO: 5, SEQ ID NO: 6, SEQ ID NO: 7 or SEQ ID NO: 8 which comprises a substitution, deletion, and/or insertion at one or more positions.
96. Method according to any of paragraphs 89-95, wherein the polypeptide is a fragment of of SEQ ID NO: 2, SEQ

ID NO: 3, SEQ ID NO: 5, SEQ ID NO: 6, SEQ ID NO: 7 or SEQ ID NO: 8, wherein the fragment has DNase activity.
97. Method according to any of the preceding method paragraphs, wherein the concentration of the polypeptide in the wash liquor and/or liquid solution is in the range of 0.00004-100 ppm enzyme protein, such as in the range of 0.00008-100, in the range of 0.0001-100, in the range of 0.0002-100, in the range of 0.0004-100, in the range of 0.0008-100, in the range of 0.001-100 ppm enzyme protein, in the range of 0.01-100 ppm enzyme protein, in the range of 0.05-50 ppm enzyme protein, in the range of 0.1-50 ppm enzyme protein, in the range of 0.1-30 ppm enzyme protein, in the range of 0.5-20 ppm enzyme protein or in the range of 0.5-10 ppm enzyme protein.

**Assays and detergent compositions**

**Detergent compositions for laundry**

The below mentioned detergent composition can be used in combination with the polypeptide of the invention for preventing or reducing static electricity.

**Composition of Ariel Sensitive White & Color, liquid detergent composition:**

Aqua, Alcohol Ethoxy Sulfate, Alcohol Ethoxylate, Amino Oxide, Citrid Acid, C12-18 topped palm kernel fatty acid, Protease, Glycosidase, Amylase, Ethanol, 1,2 Propanediol, Sodium Formate, Calcium Chloride, Sodium hydroxide, Silicone Emulsion, Trans-sulphated EHDQ (the ingredients are listed in descending order).

**Composition of WFK IEC-A model detergent (powder)**

Ingredients: Linear sodium alkyl benzene sulfonate 8,8 %, Ethoxylated fatty alcohol C12-18 (7 EO) 4,7 %, Sodium soap 3,2 %, Anti foam DC2-4248S 3,9 %, Sodium aluminium silicate zeolite 4A 28,3 %, Sodium carbonate 11,6 %, Sodium salt of a copolymer from acrylic and maleic acid (Sokalan CP5) 2,4 %, Sodium silicate 3,0 %, Carboxymethylcellulose 1,2 %, Dequest 2066 2,8 %, Optical whitener 0,2 %, Sodium sulfate6,5 %, Protease 0,4 %.

**Composition of model detergent A (liquid)**

Ingredients: 12% LAS, 11% AEO Biosoft N25-7 (NI), 7% AEOS (SLES), 6% MPG (monopropylene glycol), 3% ethanol, 3% TEA, 2.75% cocoa soap, 2.75% soya soap, 2% glycerol, 2% sodium hydroxide, 2% sodium citrate, 1% sodium formiate, 0.2% DTMPA and 0.2% PCA (all percentages are w/w)

**Composition of Ariel Actilift (liquid)**

Ingredients: 5-15% Anionic surfactants; <5% Non-ionic surfactants, Phosphonates, Soap; Enzymes, Optical brighteners, Benzisothiazolinone, Methylisothiazolinone, Perfumes, Alpha-isomethyl ionone, Citronellol, Geraniol, Linalool.

**Composition of Ariel Actilift Colour & Style**

Aqua, Sodium Dodecylbenzenesulfonate, C14-C15 Pareth-7, Sodium Citrate, Propylene Glycol, Sodium Palm Kernelate, Sodium Laureth Sulfate, MEA Dodecylbenzenesulfonage, Sulfated Ethoxylated Hexamethylenediamine Quaternized, Sodium Cumenesulfonate, Perfume, Co-polymer of PEG/Vinyl Acetate, Sodium formate, Hydrogenated Castor Oil, Sodium Diethylenetriamine Pentamethylene Phosphonate, PEG/PPG-10/2 Propylheptyl Ether, Butyophenyl Methylpropional, Polyvinylpyridine-N-Oxide, Sorbitol, Glycerin, Ethanolamine, Sodium Hydroxide, Alpha-Isomethyl lonone, Protease, Calcium Chloride, Geraniol, Linalool, Citronelllol, Tripropylene Glycol, Glycosidase, Benzisothiazolinone, Dimethicone, Glycosidase, Sodium Acetate, Cellulase, Colorant, Glyceryl Stearate, Hydroxyethylcellulose, Silica.

**Composition of Ariel Actilift Colour & Style, new pack**

Ingredients: Aqua, Sodium Laureth Sulfate, Propylene Glycol, C14-C15 Pareth-7, Sodium citrate, Sodium Palm Kernelate, Alcohol, Sodium Formate, Sulfated Ethoxylated Hexamethylenediamine Quaternized, Sodium Hydroxide, Perfume, Polyvinylpyridine-N-Oxide, Sorbitol, Calcium Chloride, protease, Glycerin, Glucosidase, Glycosidase, Sodium Acetate, Colorant, Cellulase.

**Composition of Ariel Actilift Whites & Colours Coolclean, new pack**

Ingredients: Aqua, Sodium Laureth Sulfate, Propylene Glycol, C14-C15 Pareth-7, Sodium citrate, Sodium Palm Kernelate, Alcohol, Sodium Formate, Sulfated Ethoxylated Hexamethylenediamine Quaternized, Sodium Hydroxide, Perfume, Sorbitol, Calcium Chloride, protease, Glycerin, Glucosidase, Glycosidase, Sodium Acetate, Colorant, Cellulase.

**Composition of Ariel Sensitive White & Color**

Ingredients: Aqua, Sodium Laureth Sulfate, Propylene Glycol, C14-C15 Pareth-7, Sodium citrate, Sodium Palm Kernelate, Alcohol, Sodium Formate, Sulfated Ethoxylated Hexamethylenediamine Quaternized, Sodium Hydroxide, , Sorbitol, Calcium Chloride, protease, Glycerin, Glycosidase, Sodium Acetate, Cellulase, Silica.

**Composition of Ariel Actilift, regular**

Aqua, Sodium Dodecylbenzenesulfonate, C14-C15 Pareth-7, Sodium Citrate, Propylene Glycol, Sodium Palm Kernelate, Sodium Laureth Sulfate, MEA Dodecylbenzenesulfonage, Sulfated Ethoxylated Hexamethylenediamine Quaternized, Sodium Cumenesulfonate, Perfume, Co-polymer of PEG/Vinyl Acetate, Sodium formate, C12-C14 Pareth-7, Hydrogenated Castor Oil, Sodium Diethylenetriamine Pentamethylene Phosphonate, PEG/PPG-10/2 Propylheptyl Ether, Butyophenyl Methylpropional, Fluorescent Brightener 9, Sorbitol, Glycerin, Ethanolamine, Sodium Hydroxide, Alpha-Isomethyl Ionone, Protease, Calcium Chloride, Geraniol, Linalool, Citronelllol, Tripropylene Glycol, Sodium Chloride, Glycosidase, Benzisothiazolinone, Dimethicone, Glycosidase, Sodium Acetate, Cellulase, Colorant, Glyceryl Stearate, Hydroxyethylcellulose, Silica.

**Composition of Persil Small & Mighty (liquid)**

Ingredients: 15-30% Anionic surfactants, Non-ionic surfacts, 5-15% Soap, < 5% Polycarboxylates, Perfume, Phosphates, Optical Brighteners

**Composition of Fairy Non Bio (liquid)**

Ingredients: 15-30% Anionic Surfactants,5-15% Non-Ionic Surfactants, Soap, Benzisothiazolinone, Methylisothiazolinone, Perfumes

**Composition of Model detergent T (powder)**

Ingredients: 11% LAS, 2% AS/AEOS, 2% soap, 3% AEO, 15.15% sodium carbonate, 3% sodium slilcate, 18.75% zeolite, 0.15% chelant, 2% sodium citrate, 1.65% AA/MA copolymer, 2.5% CMC and 0.5% SRP (all percentages are w/w).

**Composition of Model detergent X (powder)**

Ingredients: 16.5% LAS, 15% zeolite, 12% sodium disilicate, 20% sodium carbonate, 1% sokalan, 35.5% sodium sulfate (all percentages are w/w).

**Composition of Ariel Actilift (powder)**

Ingredients: 15-30% Anionic surfactants, <5% Non-ionic surfactants, Phosphonates, Polycarboxylates, Zeolites; Enzymes, Perfumes, Hexyl cinnamal.

**Composition of Persil Megaperls (powder)**

Ingredients: 15 - 30 % of the following: anionic surfactants, oxygen-based bleaching agent and zeolites, less than 5 % of the following: non-ionic surfactants, phosphonates, polycarboxylates, soap, Further ingredients: Perfumes, Hexyl cinnamal, Benzyl salicylate, Linalool, optical brighteners, Enzymes and Citronellol.

**Gain Liquid, Original:**

Ingredients: Water, Alcohol Ethoxysulfate, Diethylene Glycol, Alcohol Ethoxylate, Ethanolamine, Linear Alkyl

Benzene Sulfonate, Sodium Fatty Acids, Polyethyleneimine Ethoxylate, Citric Acid, Borax, Sodium Cumene Sulfonate, Propylene Glycol, DTPA, Disodium Diaminostilbene Disulfonate, Dipropylethyl Tetramine, Sodium Hydroxide, Sodium Formate, Calcium Formate, Dimethicone, Amylase, Protease, Liquitint™, Hydrogenated Castor Oil, Fragrance

**Tide Liquid, Original:**

Ingredients: Linear alkylbenzene sulfonate , propylene glycol, citric acid, sodium hydroxide, borax, ethanolamine, ethanol , alcohol sulfate, polyethyleneimine ethoxylate, sodium fatty acids, diquaternium ethoxysulfate, protease, diethylene glycol, laureth-9, alkyldimethylamine oxide, fragrance, amylase, disodium diaminostilbene disulfonate, DTPA, sodium formate, calcium formate, polyethylene glycol 4000, mannanase, Liquitint™ Blue, dimethicone.

**Liquid Tide, Free and Gentle:** Water, sodium alcoholethoxy sulfate, propylene glycol, borax, ethanol, linear alkylbenzene sulfonate sodium, salt, polyethyleneimine ethoxylate, diethylene glycol, trans sulfated & ethoxylated hexamethylene diamine, alcohol ethoxylate, linear alkylbenzene sulfonate, MEA salt, sodium formate, sodium alkyl sulfate, DTPA, amine oxide, calcium formate, disodium diaminostilbene, disulfonate, amylase, protease, dimethicone, benzisothiazolinone

**Tide Coldwater Liquid, Fresh Scent:** Water, alcoholethoxy sulfate, linear alkylbenzene sulfonate, diethylene glycol, propylene glycol, ethanolamine, citric acid, Borax, alcohol sulfate, sodium hydroxide, polyethyleneimine, ethoxylate, sodium fatty acids, ethanol, protease, Laureth-9, diquaternium ethoxysulfate, lauramine oxide, sodium cumene, sulfonate, fragrance, DTPA, amylase, disodium, diaminostilbene, disulfonate, sodium formate, disodium distyrylbiphenyl disulfonate, calcium formate, polyethylene glycol 4000, mannanase, pectinase, Liquitint™ Blue, dimethicone

**Tide TOTALCARE™ Liquid, Cool Cotton:** Water, alcoholethoxy sulfate, propylene glycol, sodium fatty acids, laurtrimonium chloride, ethanol, sodium hydroxide, sodium cumene sulfonate, citric acid, ethanolamine, diethylene glycol, silicone polyether, borax, fragrance, polyethyleneimine ethoxylate, protease, Laureth-9,

DTPA, polyacrylamide quaternium chloride, disodium diaminostilbene disulfonate, sodium formate, Liquitint™ Orange, dipropylethyl tetraamine, dimethicone, cellulase,

**Liquid Tide Plus Bleach Alternative™, Vivid White and Bright, Original and Clean Breeze:** Water, sodium alcoholethoxy sulfate, sodium alkyl sulfate, MEA citrate, linear alkylbenzene sulfonate, MEA salt, propylene glycol, diethylene glycol, polyethyleneimine ethoxylate, ethanol, sodium fatty acids, ethanolamine, lauramine oxide, borax, Laureth-9, DTPA, sodium cumene sulfonate, sodium formate, calcium formate, linear alkylbenzene sulfonate, sodium salt, alcohol sulfate, sodium hydroxide, diquaternium ethoxysulfate, fragrance, amylase, protease, mannanase, pectinase, disodium diaminostilbene disulfonate, benzisothiazolinone, Liquitint™ Blue, dimethicone, dipropylethyl tetraamine.

**Liquid Tide HE, Original Scent:** Water, Sodium alcoholethoxy sulfate, MEA citrate, Sodium Alkyl Sulfate, alcohol ethoxylate, linear alkylbenzene sulfonate, MEA salt, sodium fatty acids, polyethyleneimine ethoxylate, diethylene glycol, propylene glycol, diquaternium ethoxysulfate, borax, polyethyleneimine, ethoxylate propoxylate, ethanol, sodium cumene sulfonate, fragrance, DTPA, disodium diaminostilbene disulfonate, Mannanase, cellulase, amylase, sodium formate, calcium formate, Lauramine oxide, Liquitint™ Blue, Dimethicone / polydimethyl silicone.

**Tide TOTALCARE HE Liquid, renewing Rain:**Water, alcoholethoxy sulfate, linear alkylbenzene sulfonate, alcohol ethoxylate, citric acid, Ethanolamine, sodium fatty acids, diethylene glycol, propylene glycol, sodium hydroxide, borax, polyethyleneimine ethoxylate, silicone polyether, ethanol, protease, sodium cumene sulfonate, diquaternium ethoxysulfate, Laureth-9, fragrance, amylase, DTPA, disodium diaminostilbene disulfonate, disodium distyrylbiphenyl disulfonate, sodium formate, calcium formate, mannanase, Liquitint™ Orange, dimethicone, polyacrylamide quaternium chloride, cellulase, dipropylethyl tetraamine.

**Tide liquid HE Free:** Water, alcoholethoxy sulfate, diethylene glycol, monoethanolamine citrate, sodium formate, propylene glycol, linear alkylbenzene sulfonates, ethanolamine, ethanol, polyethyleneimine ethoxylate, amylase, benzisothiazolin, borax, calcium formate, citric acid, diethylenetriamine pentaacetate sodium, dimethicone, diquaternium ethoxysulfate, disodium diaminostilbene disulfonate, Laureth-9, mannanase, protease, sodium cumene sulfonate, sodium fatty acids.

**Tide Coldwater HE Liquid, Fresh Scent:** Water, alcoholethoxy sulfate, MEA Citrate, alcohol sulfate, Alcohol ethoxylate, Linear alkylbenzene sulfonate MEA, sodium fatty acids, polyethyleneimine ethoxylate, diethylene glycol, propylene glycol, diquaternium ethoxysulfate, borax, polyethyleneimine ethoxylate propoxylate, ethanol, sodium cumene sulfonate, fragrance, DTPA, disodium diaminostilbene disulfonate, protease, mannanase, cellulase, amylase, sodium formate, calcium formate, lauramine oxide, Liquitint™ Blue, dimethicone.

**Tide for Coldwater HE Free Liquid:** Water, sodium alcoholethoxy sulfate, MEA Citrate, Linear alkylbenzene sulfonate: sodium salt, Alcohol ethoxylate, Linear alkylbenzene sulfonate: MEA salt, sodium fatty acids, polyethyleneimine ethoxylate, diethylene glycol, propylene glycol, diquaternium ethoxysulfate, Borax, protease, polyethyleneimine ethoxylate propoxylate, ethanol, sodium cumene sulfonate, Amylase, citric acid, DTPA, disodium diaminostilbene disulfonate, sodium formate, calcium formate, dimethicone.

**Tide Simply Clean & Fresh:** Water, alcohol ethoxylate sulfate, linear alkylbenzene sulfonate Sodium/Mea

salts, propylene glycol, diethylene glycol, sodium formate, ethanol, borax, sodium fatty acids, fragrance, lauramine oxide, DTPA, Polyethylene amine ethoxylate, calcium formate, disodium diaminostilbene disulfonate, dimethicone, tetramine, Liquitint™ Blue.

**Tide Pods, Ocean Mist, Mystic Forest, Spring Meadow: L**inear alkylbenzene sulfonates, C12-16 Pareth-9, propylene glycol, alcoholethoxy sulfate, water, polyethyleneimine ethoxylate, glycerine, fatty acid salts, PEG-136 polyvinyl acetate, ethylene Diamine disuccinic salt, monoethanolamine citrate, sodium bisulfite, diethylenetriamine pentaacetate sodium, disodium distyrylbiphenyl disulfonate, calcium formate, mannanase, exyloglucanase, sodium formate, hydrogenated castor oil, natalase, dyes, termamyl, subtilisin, benzisothiazolin, perfume.

**Tide to Go:** Deionized water, Dipropylene Glycol Butyl Ether, Sodium Alkyl Sulfate, Hydrogen Peroxide, Ethanol, Magnesium Sulfate, Alkyl Dimethyl Amine Oxide, Citric Acid, Sodium Hydroxide, Trimethoxy Benzoic Acid, Fragrance.

**Tide Stain Release Liquid:** Water, Alkyl Ethoxylate, Linear Alkylbenzenesulfonate, Hydrogen Peroxide, Diquaternium Ethoxysulfate, Ethanolamine, Disodium Distyrylbiphenyl Disulfonate, tetrabutyl Ethylidinebisphenol, F&DC Yellow 3, Fragrance.

**Tide Stain Release Powder:** Sodium percarbonate, sodium sulfate, sodium carbonate, sodium aluminosilicate, nonanoyloxy benzene sulfonate, sodium polyacrylate, water, sodium alkylbenzenesulfonate, DTPA, polyethylene glycol, sodium palmitate, amylase, protease, modified starch, FD&C Blue 1, fragrance.

**Tide Stain Release, Pre Treater Spray:**

Water, Alkyl Ethoxylate, MEA Borate, Linear Alkylbenzenesulfonate, Propylene Glycol, Diquaternium Ethoxysulfate, Calcium Chlorideenzyme, Protease, Ethanolamine, Benzoisothiazolinone, Amylase, Sodium Citrate, Sodium Hydroxide, Fragrance.

**Tide to Go Stain Eraser:** Water, Alkyl Amine Oxide, Dipropylene Glycol Phenyl Ether, Hydrogen Peroxide, Citric Acid, Ethylene Diamine Disuccinic Acid Sodium salt, Sodium Alkyl Sulfate, Fragrance.

**Tide boost with Oxi:**

Sodium bicarbonate, sodium carbonate, sodium percarbonate, alcohol ethoxylate, sodium chloride, maleic/acrylic copolymer, nonanoyloxy benzene sulfonate, sodium sulfate, colorant, diethylenetriamine pentaacetate sodium salt, hydrated aluminosilicate (zeolite), polyethylene glycol, sodium alkylbenzene sulfonate, sodium palmitate, starch, water, fragrance.

**Tide Stain Release boost Duo Pac:**

Polyvinyl Alcoholpouch film, wherein there is packed a liquid part and a powder part:

**Liquid Ingredients:** Dipropylene Glycol, diquaternium Ethoxysulfate, Water, Glycerin, LiquitintTM Orange, **Powder Ingredients:** sodium percarbonate, nonanoyloxy benzene sulfonate, sodium carbonate, sodium sulfate, sodium aluminosilicate, sodium polyacrylate, sodium alkylbenzenesulfonate, maleic/acrylic copolymer, water, amylase, polyethylene glycol, sodium palmitate, modified starch, protease, glycerine, DTPA, fragrance.

**Tide Ultra Stain Release:** Water, sodium alcoholethoxy sulfate, linear alkyl benzene sulfonate, sodium/MEA salts, MEA citrate, propylene glycol, polyethyleneimine ethoxylate, ethanol, diethylene glycol, polyethyleneimine propoxyethoxylate, sodium fatty acids, protease, borax, sodium cumene sulfonate, DTPA, fragrance, amylase, disodium diaminostilbene disulfonate, calcium formate, sodium formate, gluconase, dimethicone, Liquitint™ Blue, mannanase.

**Ultra Tide with a Touch of Downy® Powdered Detergent, April Fresh/Clean Breeze/April Essence:** Sodium Carbonate, Sodium Aluminosilicate, Sodium Sulfate, Linear Alkylbenzene Sulfonate, Bentonite, Water, Sodium Percarbonate, Sodium Polyacrylate, Silicate, Alkyl Sulfate, Nonanoyloxybenzenesulfonate, DTPA, Polyethylene Glycol 4000, Silicone, Ethoxylate, fragrance, Polyethylene Oxide, Palmitic Acid, Disodium Diaminostilbene Disulfonate, Protease, Liquitint™ Red, FD&C Blue 1, Cellulase.

**Ultra Tide with a Touch of Downy Clean Breeze:** Water, sodium alcoholethoxy sulfate, MEA citrate, linear alkyl benzene sulfonate: sodium/MEA salts, propylene glycol, polyethyleneimine ethoxylate, ethanol, diethylene glycol, polyethyleneimine, propoxyethoxylate, diquaternium ethoxysulfate, alcohol sulfate, dimethicone, fragrance, borax, sodium fatty acids, DTPA, protease, sodium bisulfite, disodium diaminostilbene disulfonate, amylase, gluconase, castor oil, calcium formate, MEA, styrene acrylate copolymer, sodium formate, Liquitint™ Blue.

**Ultra Tide with Downy Sun Blossom:** Water, sodium alcoholethoxy sulfate, MEA citrate, linear alkyl benzene sulfonate: sodium/MEA salts, propylene glycol, ethanol, diethylene glycol, polyethyleneimine propoxyethoxylate, polyethyleneimine ethoxylate, alcohol sulfate, dimethicone, fragrance, borax, sodium fatty acids, DTPA, protease, sodium bisulfite, disodium diaminostilbene disulfonate, amylase, castor oil, calcium formate, MEA, styrene acrylate copolymer, propanaminium propanamide, gluconase, sodium formate, Liquitint™ Blue.

**Ultra Tide with Downy April Fresh/ Sweet Dreams:** Water, sodium alcoholethoxy sulfate, MEA citrate, linear alkyl benzene sulfonate: sodium/MEA salts, propylene glycol, polyethyleneimine ethoxylate, ethanol, diethylene glycol, polyethyleneimin propoxyethoxylate, diquaternium ethoxysulfate, alcohol sulfate, dimethicone, fragrance, borax, sodium fatty acids, DTPA, protease, sodium bisulfite, disodium diaminostilbene disulfonate, amylase, gluconase, castor oil, calcium formate, MEA, styrene acrylate copolymer, propanaminium propanamide, sodium formate, Liquitint™ Blue.

**Ultra Tide Free Powdered Detergent:** Sodium Carbonate, Sodium Aluminosilicate, Alkyl Sulfate, Sodium Sulfate, Linear Alkylbenzene Sulfonate, Water, Sodium polyacrylate, Silicate, Ethoxylate, Sodium percarbonate, Polyethylene Glycol 4000, Protease, Disodium Diaminostilbene Disulfonate, Silicone, Cellulase.

**Ultra Tide Powdered Detergent, Clean Breeze/Spring Lavender/mountain Spring:** Sodium Carbonate, Sodium Aluminosilicate, Sodium Sulfate, Linear Alkylbenzene Sulfonate, Alkyl Sulfate, Sodium Percarbonate, Water, Sodium Polyacrylate, Silicate, Nonanoyloxybenzenesulfonate, Ethoxylate, Polyethylene Glycol 4000, Fragrance, DTPA, Disodium Diaminostilbene Disulfonate, Palmitic Acid, Protease, Silicone, Cellulase.

**Ultra Tide HE (high Efficiency) Pwdered Detergent, Clean Breeze:** Sodium Carbonate, Sodium Aluminosilicate, Sodium Sulfate, Linear Alkylbenzene Sulfonate, Water, Nonanoyloxybenzenesulfonate, Alkyl Sulfate, Sodium Polyacrylate, Silicate, Sodium Percarbonate, Ethoxylate, Polyethylene Glycol 4000, Fragrance, DTPA, Palmitic Acid, Disodium Diaminostilbene Disulfonate, Protease, Silicone, Cellulase.

**Ultra Tide Coldwater Powdered Detergent, Fresh Scent:** Sodium Carbonate, Sodium Aluminosilicate, Sodium Sulfate, Sodium Percarbonate, Alkyl Sulfate, Linear Alkylbenzene Sulfonate, Water, Nonanoyloxybenzenesulfonate, Sodium Polyacrylate, Silicate, Ethoxylate, Polyethylene Glycol 4000, DTPA, Fragrance, Natalase, Palmitic Acid, Protease, Disodium, Diaminostilbene Disulfonate, FD&C Blue 1, Silicone, Cellulase, Alkyl Ether Sulfate.

**Ultra Tide with bleach Powdered Detergent, Clean Breeze:** Sodium Carbonate, Sodium Aluminosilicate, Sodium Sulfate, Linear Alkylbenzene Sulfonate, Sodium Percarbonate, Nonanoyloxybenzenesulfonate, Alkyl Sulfate, Water, Silicate, Sodium Polyacrylate, Ethoxylate, Polyethylene Glycol 4000, Fragrance, DTPA, Palmitic Acid, Protease, Disodium Diaminostilbene Disulfonate, Silicone, FD&C Blue 1, Cellulase, Alkyl Ether Sulfate.

**Ultra Tide with Febreeze Freshness™ Powdered Detergent, Spring Renewal:**
Sodium Carbonate, Sodium Aluminosilicate, Sodium Sulfate, Linear Alkylbenzene Sulfonate, Sodium Percarbonate , Alkyl Sulfate, Water, Sodium Polyacrylate, Silicate, Nonanoyloxybenzenesulfonate, Ethoxylate, Polyethylene Glycol 4000, DTPA, Fragrance, Cellulase, Protease, Disodium Diaminostilbene Disulfonate, Silicone, FD&C Blue 1.

**Liquid Tide Plus with Febreeze Freshness - Sport HE Active Fresh:**
Water, Sodium alcoholethoxy sulfate, MEA citrate, linear alkylbenzene sulfonate, sodium salt, linear alkylbenzene sulfonate: MEA salt, alcohol ethoxylate, sodium fatty acids, propylene glycol, diethylene glycol, polyethyleneimine ethoxylate propoxylate, diquaternium ethoxysulfate,
Ethanol, sodium cumene sulfonate, borax, fragrance, DTPA, Sodium bisulfate, disodium diaminostilbene disulfonate, Mannanase, cellulase, amylase, sodium formate, calcium formate, Lauramine oxide, Liquitint™ Blue, Dimethicone / polydimethyl silicone.

**Tide Plus Febreeze Freshness Spring & Renewal:**
Water, sodium alcoholethoxy sulfate, linear alkyl benzene sulfonate: sodium/MEA salts, MEA citrate, propylene glycol, polyethyleneimine ethoxylate, fragrance, ethanol, diethylene glycol, polyethyleneimine propoxyethoxylate, protease, alcohol sulfate, borax, sodium fatty acids, DTPA, disodium diaminostilbene disulfonate, MEA, mannanase, gluconase, sodium formate, dimethicone, Liquitint™ Blue, tetramine.

**Liquid Tide Plus with Febreeze Freshness, Sport HE Victory Fresh:** Water, Sodium alcoholethoxy sulfate, MEA

citrate, linear alkylbenzene sulfonate, sodium salt, linear alkylbenzene sulfonate: MEA salt, alcohol ethoxylate, sodium fatty acids, propylene glycol, diethylene glycol, polyethyleneimine ethoxylate propoxylate, diquaternium ethoxysulfate, ethanol, sodium cumene sulfonate, borax, fragrance, DTPA, Sodium bisulfate, disodium diaminostilbene disulfonate, Mannanase, cellulase, amylase, sodium formate, calcium formate,
Lauramine oxide, Liquitint™ Blue, Dimethicone / polydimethyl silicone.

**Tide Vivid White + Bright Powder, Original:**
Sodium Carbonate, Sodium Aluminosilicate, Sodium Sulfate, Linear Alkylbenzene Sulfonate,
Sodium Percarbonate, Nonanoyloxybenzenesulfonate, Alkyl Sulfate, Water, Silicate, Sodium Polyacrylate
Ethoxylate, Polyethylene Glycol 4000, Fragrance, DTPA, Palmitic Acid, Protease, Disodium Diaminostilbene Disulfonate, Silicone, FD&C Blue 1, Cellulase, Alkyl Ether Sulfate.

The products named Tide, Ariel, Gain and Fairy are commercially available products supplied by Procter & Gamble. The products named Persil are commercially available products supplied by Unilever and Henkel.

| Ingredient | Amount (in wt %) |
|---|---|
| Anionic detersive surfactant (such as alkyl benzene sulphonate, alkyl ethoxylated sulphate and mixtures | from 8 wt % to 15 wt % thereof) |
| Non-ionic detersive surfactant (such as alkyl ethoxylated alcohol) | from 0.5 wt % to 4 wt % |
| Cationic detersive surfactant (such as quaternary ammonium compounds) | from 0 to 4 wt % |
| Other detersive surfactant (such as zwiterionic detersive surfactants, amphoteric surfactants and mixtures thereof) | from 0 wt % to 4 wt % |
| Carboxylate polymer (such as co-polymers of maleic acid and acrylic acid) | from 1 wt % to 4 wt % |
| Polyethylene glycol polymer (such as a polyethylene glycol polymer comprising poly vinyl acetate side chains) | from 0.5 wt % to 4 wt % |
| Polyester soil release polymer (such as Repel-o-tex from and/or Texcare polymers) | 0.1 to 2 wt % |
| Cellulosic polymer (such as carboxymethyl cellulose, methyl cellulose and combinations thereof) | from 0.5 wt % to 2 wt % |
| Other polymer (such as amine polymers, dye transfer inhibitor polymers, hexamethylenediamine derivative polymers, and mixtures thereof) | from 0 wt % to 4 wt % |
| Zeolite builder and phosphate builder (such as zeolite 4A and/or sodium tripolyphosphate) | from 0 wt % to 4 wt% |
| Other builder (such as sodium citrate and/or citric acid) | from 0 wt % to 3 wt % |
| Carbonate salt (such as sodium carbonate and/or sodium bicarbonate) | from 15 wt % to 30 wt % |
| Silicate salt (such as sodium silicate) | from 0 wt % to 10 wt % |
| Filler (such as sodium sulphate and/or bio-fillers) | from 10 wt % to 40 wt % |
| Source of available oxygen (such as sodium percarbonate) | from 10 wt % to 20 wt % |
| Bleach activator (such as tetraacetylethylene diamine (TAED) and/or nonanoyloxybenzenesulphonate (NOBS) | from 2 wt % to 8 wt % |
| Bleach catalyst (such as oxaziridinium-based bleach catalyst and/or transition metal bleach catalyst) | from 0 wt % to 0.1 wt % |
| Other bleach (such as reducing bleach and/or preformed peracid) | from 0 wt % to 10 wt % |
| Chelant (such as ethylenediamine-N’N’-disuccinic acid (EDDS) and/or hydroxyethane diphosphonic acid(HEDP) | from 0.2 wt % to 1 wt % |
| Photobleach (such as zinc and/or aluminium sulphonated phthalocyanine) | from 0 wt % to 0.1 wt % |
| Hueing agent (such as direct violet 99, acid red 52, acid blue 80, direct violet 9, solvent violet 13 and any combination thereof) | from 0 wt % to 1 wt % |

(continued)

| Ingredient | Amount (in wt %) |
|---|---|
| Brightener (such as brightener 15 and/or brightener 49) | from 0.1 wt % to 0.4 wt % |
| Protease (such as Savinase, Savinase Ultra, Purafect, FN3, FN4 and any combination thereof) | from 0.1 wt % to 0.4 wt % |
| Amylase (such as Termamyl, Termamyl ultra Natalase, Optisize, Stainzyme, Stainzyme Plus, and any combination thereof) | from 0.05 wt % to 0.2 wt % |
| Cellulase (such as Carezyme and/or Celluclean) | from 0.05 wt % to 0.2 wt % |
| Lipase (such as Lipex, Lipolex, Lipoclean and any combination thereof) | from 0.2 to 1 wt % |
| Other enzyme (such as xyloglucanase, cutinase, pectate lyase, mannanase, bleaching enzyme) | from 0 wt % to 2 wt % |
| Fabric softener (such as montmorillonite clay and/or polydimethylsiloxane (PDMS) | from 0 wt % to 4 wt % |
| Flocculant (such as polyethylene oxide) | from 0 wt % to 1 wt % |
| Suds suppressor (such as silicone and/or fatty acid) | from 0 wt % to 0.1 wt % |
| Perfume (such as perfume microcapsule, spray-on perfume, starch encapsulated perfume accords, perfume loaded zeolite, and any combination thereof) | from 0.1 wt % to 1 wt % |
| Aesthetics (such as coloured soap rings and/or coloured speckles/noodles) | from 0 wt % to 1 wt % |
| Miscellaneous | Balance |

| Ingredient | Amount |
|---|---|
| Carboxyl group-containing polymer (comprising from about 60% to about 70% by mass of an acrylic acid-based monomer (A); and from about 30% to about 40%) by mass of a sulfonic acid group-containing monomer (B); and wherein the average molecular weight is from about 23,000 to about 50,000 preferably in the range of from about 25,000 to about 38,000 as described in WO2014032269. | from about 0.5 wt% to about 1.5 wt% |
| Amylase (Stainzyme Plus(R), having an enzyme activity of 14 mg active enzyme/ g) | from about 0.1wt% to about 0.5 wt% |
| Anionic detersive surfactant (such as alkyl benzene sulphonate, alkyl ethoxylated sulphate and mixtures thereof) | from about 8 wt% to about 15 wt% |
| Non-ionic detersive surfactant (such as alkyl ethoxylated alcohol) | from about 0.5 wt% to 4 wt% |
| Cationic detersive surfactant (such as quaternary ammonium compounds) | from about 0 wt% to about 4 wt% |
| Other detersive surfactant (such as zwiterionic detersive surfactants, amphoteric surfactants and mixtures thereof) | from about 0 wt% to 4 wt% |
| Carboxylate polymer (such as co-polymers of maleic acid and acrylic acid) | from about 1 wt% to about 4 wt% |

(continued)

| Ingredient | Amount |
|---|---|
| Polyethylene glycol polymer (such as a polyethylene glycol polymer comprising poly vinyl acetate side chains) | from about 0 wt% to about 4 wt% |
| Polyester soil release polymer (such as Repel-O-Tex(R) and/or Texcare(R) polymers) | from about 0.1 wt% to about 2 wt% |
| Cellulosic polymer (such as carboxymethyl cellulose, methyl cellulose and combinations thereof) | from about 0.5wt% to about 2 wt% |
| Other polymer (such as amine polymers, dye transfer inhibitor polymers, hexamethylenediamine derivative polymers, and mixtures thereof) | from about 0 wt% to about 4 wt% |
| Zeolite builder and phosphate builder (such as zeolite 4A and/or sodium tripolyphosphate) | from about 0 wt% to about 4 wt% |
| Other builder (such as sodium citrate and/or citric acid) | from about 0 wt% to about 3 wt% |
| Carbonate salt (such as sodium carbonate and/or sodium bicarbonate) | from about 15 t% to about 30 wt% |
| Silicate salt (such as sodium silicate) | from about 0 wt% to about 10 wt% |
| Filler (such as sodium sulphate and/or bio-fillers) | from about 10 wt% to about 40 wt% |
| Source of available oxygen (such as sodium percarbonate) | from about 10wt% to about 20 wt% |
| Bleach activator (such as tetraacetylethylene diamine (TAED) and/or nonanoyloxybenzenesulphonate (NOBS) | from about 2 wt% to about 8 wt% |
| Bleach catalyst (such as oxaziridinium-based bleach catalyst and/or transition metal bleach catalyst) | from about 0 wt% to about 0.1 wt% |
| Other bleach (such as reducing bleach and/or pre formed peracid) | from about 0 wt% to about 10 wt% |
| Chelant (such as ethylenediamine-N'N'-disuccinic acid (EDDS) and/or hydroxyethane diphosphonic acid (HEDP) | from about 0.2wt% to about 1wt% |
| Photobleach (such as zinc and/or aluminium sulphonated phthalocyanine) | from about 0 wt% to about 0.1 wt% |

(continued)

| Ingredient | Amount |
|---|---|
| Hueing agent (such as direct violet 99, acid red 52, acid blue 80, direct violet 9, solvent violet 13 and any combination thereof) | from about 0 wt% to about 0.5 wt% |
| Brightener (such as brightener 15 and/or brightener 49) | from about 0.1wt% to about 0.4 wt% |
| Protease (such as Savinase, Polarzyme, Purafect, FN3, FN4 and any combination thereof, typically having an enzyme activity of from about 20 mg to about 100mg active enzyme/g) | from about 0.1wt% to about 1.5 wt% |
| Amylase (such as Termamyl(R), Termamyl Ultra(R), Natalase(R), Optisize HT Plus(R), Powerase (R), Stainzyme(R) and any combination thereof, typically having an enzyme activity of from about 10 mg to about 50 mg active enzyme/ g) | from about 0.05 wt% to about 0.2 wt% |
| Cellulase (such as Carezyme(R), Celluzyme(R) and/or Celluclean(R), typically having an enzyme activity of about from 10 to 50mg active enzyme/ g) | from about 0.05 wt% to 0.5 wt% |
| Lipase (such as Lipex(R), Lipolex(R), Lipoclean(R) and any combination thereof, typically having an enzyme activity of from about 10 mg to about 50 mg active enzyme/ g) | from about 0.2 wt% to about 1 wt% |
| Other enzyme (such as xyloglucanase (e.g., Whitezyme(R)), cutinase, pectate lyase, mannanase, bleaching enzyme, typically having an enzyme activity of from about 10 mg to about 50 mg active enzyme/g) | from 0 wt% to 2 wt% |
| Fabric softener (such as montmorillonite clay and/or polydimethylsiloxane (PDMS)) | from 0 wt% to 15 wt% |
| Flocculant (such as polyethylene oxide) | from 0 wt% to 1 wt% |
| Suds suppressor (such as silicone and/or fatty acid) | from 0 wt% to 0.1 wt% |
| Perfume (such as perfume microcapsule, spray-on perfume, starch encapsulated perfume accords, perfume loaded zeolite, and any combination thereof) | from 0.1 wt% to 1 wt% |
| Aesthetics (such as colored soap rings and/or colored speckles/noodles) | from 0 wt% to 1wt% |
| Miscellaneous | Balance |

All enzyme levels expressed as rug active enzyme protein per 100 g detergent composition. Surfactant ingredients can be obtained from BASF, Ludwigshafen, Germany (Lutensol(R)); Shell Chemicals, London, UK; Stepan, Northfield, Ill, USA; Huntsman, Huntsman, Salt Lake City, Utah, USA; Clariant, Sulzbach, Germany (Praepagen(R)). Sodium tripolyphosphate can be obtained from Rhodia, Paris, France. Zeolite can be obtained from Industrial Zeolite (UK) Ltd, Grays, Essex, UK. Citric acid and sodium citrate can be obtained from Jungbunzlauer, Basel, Switzerland. NOBS is sodium nonanoyloxybenzenesulfonate, supplied by Eastman, Batesville, Ark., USA.

TAED is tetraacetylethylenediamine, supplied under the Peractive(R) brand name by Clariant GmbH, Sulzbach, Germany.

Sodium carbonate and sodium bicarbonate can be obtained from Solvay, Brussels, Belgium.

Polyacrylate, polyacrylate/maleate copolymers can be obtained from BASF, Ludwigshafen, Germany.
Repel-O-Tex(R) can be obtained from Rhodia, Paris, France.

Texcare(R) can be obtained from Clariant, Sulzbach, Germany. Sodium percarbonate and sodium carbonate can be obtained from Solvay, Houston, Tex., USA.

Na salt of Ethylenediamine-N,N'-disuccinic acid, (S,S) isomer (EDDS) was supplied by Octel, Ellesmere Port, UK.

Hydroxy ethane di phosphonate (HEDP) was supplied by Dow Chemical, Midland, Mich., USA.

Enzymes Savinase(R), Savinase(R) Ultra, Stainzyme(R) Plus, Lipex(R), Lipolex(R), Lipoclean(R), Celluclean(R), Carezyme(R), Natalase(R), Stainzyme(R), Stainzyme(R) Plus, Termamyl(R), Termamyl(R) ultra, and Mannaway(R) can be obtained from Novozymes, Bagsvaerd, Denmark.

Enzymes Purafect(R), FN3, FN4 and Optisize can be obtained from Genencor International Inc., Palo Alto, California, US.

Direct violet 9 and 99 can be obtained from BASF DE, Ludwigshafen, Germany. Solvent violet 13 can be obtained from Ningbo Lixing Chemical Co., Ltd. Ningbo, Zhejiang, China. Brighteners can be obtained from Ciba Specialty Chemicals, Basel, Switzerland. All percentages and ratios are calculated by weight unless otherwise indicated. All percentages and ratios are calculated based on the total composition unless otherwise indicated. It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

### Detergent compositions for dishwash

### Cascade Platinum® Action Pacs® and Cascade Complete® Action Pacs®

Sodium Carbonate, Sodium Percarbonate, Sodium Silicate, Modified Polyacrylate, Methyl Glycine Diacetic Acid (Trisodium Salt), Sodium Sulfate, Protease, Amylase, Alcohol Alkoxylate, Polyethylene glycol, Hydrozincite, Amine Cobalt Salt, Water, Perfumes, Alcohol Alkoxylate, Trideceth-n, Dipropylene glycol, Water, Glycerine, Acid Red #33 and/or FD&C Yellow #5 and/or Acid Blue 182 and/or Dye Reactive Green 12.

### Cascade® Action Pacs®

Sodium Percarbonate, Sodium Silicate, Modified Polyacrylate, Methyl Glycine Diacetic Acid (Trisodium Salt), Sodium Sulfate, Protease, Amylase, Alcohol Alkoxylate, Polyethylene glycol, Hydrozincite, Amine Cobalt Salt, Water, Perfumes, Alcohol Alkoxylate, Trideceth-n, Dipropylene glycol, Water, Glycerine, Acid Red #33 and/or FD&C Yellow #5 and/or Acid Blue 182 and/or Dye Reactive Green 12.

### Cascade Gel

Water, Sodium Silicate, Sodium Polyacrylate, Sodium Hypochlorite, Sodium Carbonate, Sodium Sulfate, Sodium Benzoate, Sodium Hydroxide, Cross-linked polyacrylate, Zinc Carbonate, Nitric acid, Various perfumes.

### Cascade Complete® Gel

Water, Tetrasodium Glutamate Diacetate, Sodium Bicarbonate, Citric Acid, Sodium Silicate, Modified Polyacrylate, Xanthan Gum, Polyethyleneimine (sulfonated), Alcohol alkoxylates, Zinc Sulfate, Calcium Chloride, Protease, Amylase, Sodium Benzoate, Proxel GXL, Liquitint™ Bright Yellow and/or Liquitint™ Bright Brilliant Orange and/or Direct Blue 086, Perfume.

### Cascade® Powder

Sodium Carbonate, Sodium Sulfate, Water, Sodium Silicate, Sodium Percarbonate, Modified polyacrylate, Alcohol Alkoxylate, Polyethylene glycol, Hydrozincite, Amine Cobalt Salt, Protease, Amylase, Perfumes.

### Cascade Dishwasher Detergent®

Citric Acid, Alcohol Alkoxylates, Silica, Acid Blue 182, Perfumes, Alcohol Alkoxylates, Dipropylene Glycol, Trideceth-n, Water, Glycerine, Acid Blue 182.

**Fairy Liquid**

Aqua, Sodium laureth sulphate, Alcohol denat, Lauramine oxide, C9-11 pareth-8, Sodium chloride, 1,3-Cyclohexanedimethanamine, PPG (polypropylene glycols), Dimethyl aminoethyl methecrylate/hydroxyproply acrylate copolymer cirate, Parfum, Geraniol, Limonene, Colourant.

**Fairy Professional All in One Lemon Dishwasher**

>30% Phosphates; 5-15% Non-ionic surfactants, Oxygen-based bleaching agents; <5% Phosphonates, Polycarboxylates; Enzymes, Perfumes, Citronellol, Limonene, Linalool.

**Fairy Professional All in One Original Dishwasher Tablets**

>30% Phosphates; 5-15% Non-ionic surfactants, Oxygen-based bleaching agents; <5% Polycarboxylates; Enzymes; Perfumes, Geraniol.

**Fairy Professional All in One Platinum Dishwasher**

>30% Phosphates; 5-15% Non-ionic surfactants, Oxygen-based bleaching agents, Polycarboxylates; <5% Phosphonates; Enzymes, Perfumes, Citronellol, Limonene, Linalool.

**Fairy Professional Powder Bursts Tablets**

>30% Phosphates; 5-15%oxygen-based bleaching agents; <5% Non-Ionic surfactants, Polycarboxylater; Enzymes, Perfumes, and either (A) Citrol, Limonene, Linalool or (B) Geraniol, Limonene.

**Fairy Professional Original Washing Up Liquid and Fairy Professional Original Lemon Washing Up Liquid and Fairy Professional Extra Clean Washing Up Liquid**

15-30% Anionic surfactants; 5-15% Non-ionic surfactants; Phenoxyethanol, Methylisothiazolinone, Perfume.

**Fairy Professional Antibacterial Washing Up Liquid**

15-30% Anionic surfactants; 5-15% Non-ionic surfactants;Methylisothiazolinone, Phenoxyethanol: Disinfectants, Perfumes, Limonene, Citronellol.

**Cascade Professional Automatic Dishwashing Detergent**

15-40% Sodium carbonate; 1-5% Sodium carbonate peroxyhydrate; 1-5% sodium silicate.

**Bistro 141 Ware Wash Detergent**

1-5% Sodium hydroxide; 1-5% Potassium hydroxide; 5-15% Potassium tri-polyphosphate; 5-15% Dinatriummetasilicate, pentahydrate; 1-5% tetrasodium-EDTA; < 5% polycarboxylates.

**Bistro 741 Ware Wash Detergent**

5-15% Sodium hydroxide; 1-5% 2-Phosphonobutan-1,2,4-tricarboxylic acid; < 5% phosphates; < 5% polycarboxylates.

**Bistro 742 Ware Wash Detergent**

5-15% Potassium hydroxide; 5-15% Potassium silicate; 1-5% Disodiummetasilicate, pentahydrate; 1-5% Sodium carbonate; 5-15% Phosphates; < 5% Anionic surfactants; < 5% Phosphonates; < 5% Polycarboxylates.

**Bistro 743 Ware Wash Detergent**

30-60% Sodium carbonate; 5-15% Disodiummetasilicate, pentahydrate; 5-15% Sodium percarbonate; 5-15%

Sodium silicate; 1-5% Fatty alcohol alkoxylate; 5-15% phosphates; 5-15% Oxygen based bleaches; < 5% Nonionic surfactants; < 5% Polycarboxylates; < 1% Enzymes.

**Bistro CL 341 Ware Wash Detergent**

15-30% Sodium hypochlorite; 5-15% Potassium hydroxide; 5-15% Potassium tripolyphosphate; 5-15% Disodiummetasilicate, pentahydrate; 1-5% Sodium hydroxide; < 5% Polycarboxylates.

**Bistro Glas 345 Ware Wash Detergent**

5-15% Disodiummetasilicate, pentahydrate; 1-5% Potassium hydroxide; 1-5% Potassium tripolyphosphate; 1-5% sodium caprylyl limino dipropionate; 1-5% Fatty alcohol alkoxylate; < 1% Zinc sulfate monohydrate.

**Suma Nova L6 Ware Wash Detergent**

10-20% Tetrasodium-EDTA; 10-20% Sodium hydroxide.

**Suma Revoflow Max P2 Ware Wash Detergent**

50-75% Sodium hydroxide.

**Suma Alu Free L10 Ware Wash Detergent**

10-20% Dipotassium metasilicate.

**Suma Blend L7 Ware Wash Detergent**

10-20% Disodium/dipotassium metasilicate; 1-3% Sodium hypochlorite; 1-3% Potassium hydroxide.

**Suma Combi+ LA6 Ware Wash Detergent And Rinse Aid**

10-20% Tetrasodium-ethylendiamintetraacetate; 10-20% Sodium hydroxide.

**Topmatic Clean Ware Wash Detergent**

5-10% Sodium hydroxide.

**Apex Chlorine Free Machine Detergent**

>60% Sodium carbonate; <10% Alcohols, c13-15-branched and linear, butoxylated ethoxylated.

**Apex Metal Protection Ware Wash Detergent**

< 10% Zinc chloride; < 10% Sodium hydroxide.

**Apex Power Plus Ware Wash Detergent**

< 60% Sodium carbonate; < 10% Disodium metasilicate; < 10% Troclosene sodium, dihydrate; < 10% Phosphonic acid, (1-hydroxyethylidene)bis-,potassium salt.

**Apex Ultra LW Ware Wash Detergent**

30-60% Sodium carbonate; < 10% Troclosene sodium, dihydrate; < 10% Disodium metasilicate.

The products named Cascade and Fairy are commercially available products supplied by Procter & Gamble. The products named Bistro are commercially available products supplied by Novadan. The products named Suma are commercially available products supplied by Diversey. The products named Topmatic and Apex are commercially available products supplied by Ecolab.

### Enzyme assays

### Assay I

Testing of DNase activity

DNase activity was determined on DNase Test Agar with Methyl Green (BD, Franklin Lakes, NJ, USA), which was prepared according to the manual from supplier. Briefly, 21 g of agar was dissolved in 500 ml water and then autoclaved for 15 min at 121°C. Autoclaved agar was temperated to 48°C in water bath, and 20 ml of agar was poured into petridishes with and allowed to solidify by incubation o/n at room temperature. On solidified agar plates, 5 μl of enzyme solutions are added, and DNase activity are observed as colorless zones around the spotted enzyme solutions.

### Assay II

Analysis of E-2-nonenal on textile using an electronic nose

One way of testing for the presence of malodor on textiles is by using E-2-Nonenal as a marker for the malodor, as this compound contributes to the malodor on laundry.

Add a solution of E-2-nonenal to a 5 cm x 5 cm textile swatch and place the swatch in a 20 mL glass vial for GC analysis and cap the vial. Analyze 5 mL headspace from the capped vials in a Heracles II Electronic nose from Alpha M.O.S., France (double column gas chromatograph with 2 FIDs, column 1: MXT5 and column 2: MXT1701) after 20 minutes incubation at 40°C.

## Examples

### Example 1

**Prevention of adhesion and biofilm formation on surfaces relevant for laundering and dish washing**

In the present study, one strain of *Brevundimonas* sp. was used. The *Brevundimonas* sp. was pre-grown on Tryptone Soya Agar (TSA) (pH 7.3) (CM0131; Oxoid Ltd, Basingstoke, UK) for 2-5 days at 30°C. From a single colony, a loop-full was transferred to 10 mL of TSB (Tryptone Soya broth, Oxoid) and incubated for 1 day at 30°C with shaking (240 rpm). After propagation, *Brevundimonas* sp. was pelleted by centrifugation (Sigma Laboratory Centrifuge 6K15) (3000 g at 21°C in 7 min) and resuspended in 10 mL of TSB diluted twice with water. Optical density (OD) at 600 nm was measured using a spectophometer (POLARstar Omega (BMG Labtech, Ortenberg, Germany). Fresh TSB diluted twice with water was inoculated to an $OD_{600nm}$ of 0.03, and 3 mL was added into each well of a 12-well polystyrene flat-bottom microplate (3512; Corning Incorporated, Corning, NY, USA), in which coupons of steel (RD128-316), PVC (RD128-PVC), rubber (RD128-Si), porcelain (RD128-PL) and glass (RD128-GL) (all obtained from Biosurface Technologies Corporation, Bozeman, MT, USA) were placed. Ten ppm of DNAse from *Aspergillus oryzae* (SEQ ID NO: 2), *Trichoderma harizianum* (SEQ ID NO: 5), and *Bacillus licheniformis* (SEQ ID NO: 7),, respectively, was added to the wells containing coupons and *Brevundimonas* sp. Wells containing coupons and *Brevundimonas sp.* and not containing DNAse were included as controls. After incubation (72 h at 37°C), non-adherent cells were removed by rinsing coupons twice with 0.9% (w/v) NaCl. Adherent cells were visualized by adding 3 ml of 0.1% (w/v) crystal violet (C0775; Sigma-Aldrich, St. Louis, MO, USA) and left for 15 min at room temperature. Coupons were washed twice with 0.9% (w/v) and moved to a new 12-well polystyrene flat-bottom microplate. Bound crystal violet was eluted by the addition of 3 ml of 96% (w/v) ethanol (201145; Kemetyl, Køge, Denmark) and determined by measurement absorbance at 595 nm.

Table 1. Prevention of adhesion and biofilm formation on steel, PVC, rubber, porcelain and glass.

| | Biofilm (%) | | | | |
|---|---|---|---|---|---|
| | Steel | PVC | Rubber | Porcelain | Glass |
| Control (not added DNAse) | 100 | 100 | 100 | 100 | 100 |
| *Aspergillus oryzae* DNAse | 36 | 36 | 11 | 22 | 70 |
| *Trichoderma harzianum* DNAse | 52 | 49 | 14 | 47 | 87 |
| *Bacillus lichenformis* DNAse | 34 | 24 | 4 | 30 | 64 |

**Example 2**

**DNAse mediated reduction of stickiness of biofilm on plastic surface**

The *Brevundimonas* sp. used in Example 1 was also used to the present study, which were pre-grown according to the procedure described in Example 1. After propagation, the *Brevundimonas* sp. culture was diluted 100-fold in TSB. 125 μl of the 100-fold diluted culture was added to each well of a 96 well polystyrene plate with Nunclon Delta surface (Thermo Scientific, #167008). The plate was incubated at 15 °C for 3 days to allow for biofilm growth. The medium was removed from the wells and the wells were rinsed by adding 300 μl milliQ water. The water was then aspirated. To each well was added 150 μl either 0.1 ppm or 0.3 ppm DNase from *Aspergillus oryzae* (SEQ ID NO:2) diluted in Model A detergent (Model detergent A)supplemented with 0.7 g/l pigment soil (Pigmentschmutz 09V, wfk, Krefeld, Germany). The Model A detergent solution (the wash liquor) was prepared with water having a hardness of 15 °dH. As a control, 150 μl Model A detergent supplemented with 0.7 g/l pigment soil without DNase was added. The plate was incubated for 1 hour at 30 °C with shaking (800 rpm). The wash liquor was then aspirated from each well. The amount of soil adhering to the biofilm at the bottom of the wells was quantified with a DigiEye Imaging System (VeriVide) and the values reported are L-values in the Lab color space.

Table 2. Reduced stickiness of biofilm on plastic surface with DNase

| DNase concentration (ppm) | L-value | L-value(with DNase) - L-value(without DNase) |
|---|---|---|
| 0 | 77.78 | |
| 0.1 | 81.21 | 3.43 |
| 0.3 | 83.00 | 5.22 |

The present study demonstrates that the *Aspergillus oryzae* DNAse disclosed herein formulated in a detergent composition is capable of reducing the stickiness of biofilm on plastic surface less soil is deposited on the surface.

**Example 3**

**DNAse mediated removal from plastic surface**

The *Brevundimonas* sp. used in Example 1 was also used to the present study, which were pre-grown according to the procedure described in Example 1. After propagation, the

*Brevundimonas* sp. culture was diluted 1000-fold in TSB. 125 μl of the 1000-fold diluted culture was added to each well of a 96 well polystyrene plate with Nunclon Delta surface (Thermo Scientific, #167008). The plate was incubated at 15 °C for 2 days to allow for biofilm growth. The medium was removed from the wells and the wells were rinsed by adding 300 μl milliQ water. The water was then aspirated. To each well was added 150 μl either 0.03 ppm, 0.1 ppm, 0.3 ppm or 1 ppm DNase from *Aspergillus oryzae* (SEQ ID NO:2) diluted in water. As a control, 150 μl pure milliQ water without DNase was added. The plate was incubated at 30 °C for 1 hour with shaking (1000 rpm). The enzyme solutions were aspirated and 300 μl milliQ water was added to rinse the wells. The water was aspirated, 150 μl 0.1 % (w/v) crystal violet (C0775; Sigma-Aldrich, St. Louis, MO, USA) was added to each well to stain the remaining biofilm and the plate was incubated at room temperature for 30 minutes. The crystal violet was removed and 300 μl milliQ water was added to rinse the wells. The water was aspirated, 150 μl 96 % ethanol (201145; Kemetyl, Køge, Denmark) was added to each well to dissolve the crystal violet and the plate was incubated at 25 °C for 30 minutes with shaking (800 rpm). The amount of crystal violet was quantified by measuring absorbance at 595 nm (POLARstar Omega, BMG Labtech, Ortenberg, Germany).

Table 3. Biofilm removal from plastic surface with DNase

| DNase concentration (ppm) | $Abs_{590nm}$ | $Abs_{590nm(without\ DNase)}$-$Abs_{590nm(with\ DNase)}$ |
|---|---|---|
| 0 | 1.263 | |
| 0.03 | 1.062 | 0.201 |
| 0.1 | 1.003 | 0.260 |
| 0.3 | 0.953 | 0.310 |
| 1 | 0.858 | 0.405 |

The present study demonstrates that the *Aspergillus oryzae* DNAse disclosed herein is capable of removing biofilm from a plastic surface.

**Example 4**

In the present study, one strain of *Brevundimonas* sp., one strain of *Pseudomonas fluorescens* (Pseudomonas 1) and one strain of *Pseudomonas alcaliphila* (Pseudomonas 2) were used.

All three bacterial stains were pre-grown on Tryptone Soya Agar (TSA) (pH 7.3) (CM0131; Oxoid Ltd, Basingstoke, UK) for 2-5 days at 30°C. From a single colony, a loop-full was transferred to 10 mL of TSB (Tryptone Soya broth, Oxoid) and incubated for 1 day at 30°C with shaking (240 rpm). After propagation, all three bacterial strains were pelleted by centrifugation (Sigma Laboratory Centrifuge 6K15) (3000 g at 21°C in 7 min). *Brevundimonas* sp. was resuspended in 10 mL of TSB diluted twice with water, whereas *P. fluorescens* and *P. alcaliphila* were diluted in undiluted TSB. Optical density (OD) at 600 nm was measured using a spectophometer (POLARstar Omega (BMG Labtech, Ortenberg, Germany). Fresh TSB diluted twice with water was inoculated to an $OD_{600nm}$ of 0.03 with *Brevundimonas* sp., whereas fresh undiluted TSB was inoculated to an $OD_{600nm}$ of 0.03 with *P. fluorescens* and *P. alcaliphila,* respectively. 100 μl was added into each well of a 96-well polystyrene flat-bottom microplate (161093; Nunc, Roskilde, Denmark). Plates with *Brevundimonas* sp. were incubated for 24 h and 48 h at 15°C. Plates with *P. fluorescens* and *P. alcaliphila* were incubated for 48 h at 30°C.

After incubation, non-adherent cells were removed by washing two times with 0.9% (w/v) NaCl (Merck). Adherent cells were treated with 200 μl of three Automatic Dishwashing (ADW) powder model detergents and one liquid model detergent.

ADW model A (with percarbonate and with TAED) containing MGDA (29%), Sodium citrate (17%), Sodium carbonate (17%), Sodium percarbonate (10%), Sodium silicate (5%), Sodium sulphate (10%), Acusol 588G (5%), TAED (3%) and Surfac 23-6.5 (4%) was prepared by dissolving 3.94 g in 1000 ml water with hardness 15 °dH.

ADW model A1 (with percarbonate and without TAED) containing MGDA (30%), Sodium citrate (18%), Sodium carbonate (18%), Sodium percarbonate (10%), Sodium silicate (5%), Sodium sulphate (11%), Acusol 588G (5%) and Surfac 23-6.5 (4%) was prepared by dissolving 3.83 g in 1000 ml water with hardness 15 °dH.

ADW model A2 (without percarbonate and without TAED) containing MGDA (33%), Sodium citrate (20%), Sodium carbonate (20%), Sodium silicate (6%), Sodium sulphate (12%), Acusol 588G (5%) and Surfac 23-6.5 (5%) was prepared by dissolving 3.45 g in 1000 ml water with hardness 15 °dH.

ADW liquid detergent containing Na-citrate (2.9%), Na-formiate (0.2%), GLDA (28.7%), HEDP (0.3%), PCA polymer (1.0%), water (9.6%), 37 w/w% HCI (5.2%), 10M HCI (3.7%) and NaOH (0.2%) was prepared by dissolving 3.70 g in 1000 ml water with hardness 15 °dH.

All treatments took place at 45°C for 30 and 60 min. ADW detergents were removed and wells were rinsed twice with 0.9% (w/w) NaCl. To quantify adherent cells, 200 μl of 0.1% (w/v) crystal violet (C0775; Sigma-Aldrich, St. Louis, MO, USA) was added and left for 15 minutes at room temperature. The wells were washed twice with 0.9% (w/v) NaCl, and bound crystal violet was eluted by the addition of 200 μl 96% (w/v) ethanol (201145; Kemetyl, Køge, Denmark) and determined by measurement at 595 nm.

| Detergent | Strain | Age of biofilm (h) | Washing time (h) | OD595nm Without DNase | OD595nm With DNase | Biofilm reduction (%) |
|---|---|---|---|---|---|---|
| ADW model A | Brevundimonas | 24 | 0.5 | 0.704 | 0.576 | 18 |
| | Brevundimonas | 24 | 1 | 0.773 | 0.602 | 22 |
| | Brevundimonas | 48 | 0.5 | 0.647 | 0.394 | 39 |
| | Brevundimonas | 48 | 1 | 0.599 | 0.461 | 23 |
| | Pseudomonas 1 | 48 | 0.5 | 0.243 | 0.226 | 7.3 |
| | Pseudomonas 1 | 48 | 1 | - | - | - |
| | Pseudomonas 2 | 48 | 0.5 | - | - | - |
| | Pseudomonas 2 | 48 | 1 | - | - | - |
| | | | | | | |

(continued)

| Detergent | Strain | Age of biofilm (h) | Washing time (h) | OD595nm Without DNase | OD595nm With DNase | Biofilm reduction (%) |
|---|---|---|---|---|---|---|
| ADW model A1 | Brevundimonas | 24 | 0.5 | 0.654 | 0.454 | 31 |
| | Brevundimonas | 24 | 1 | 0.564 | 0.404 | 28 |
| | Brevundimonas | 48 | 0.5 | 0.534 | 0.404 | 24 |
| | Brevundimonas | 48 | 1 | 0.473 | 0.570 | 25 |
| | Pseudomonas 1 | 48 | 0.5 | - | - | - |
| | Pseudomonas 1 | 48 | 1 | 0.358 | 0.264 | 26 |
| | Pseudomonas 2 | 48 | 0.5 | 0.205 | 0.168 | 18 |
| | Pseudomonas 2 | 48 | 1 | 0.194 | 0.178 | 8.2 |
| | | | | | | |
| ADW model A2 | Brevundimonas | 24 | 0.5 | 0.546 | 0.428 | 22 |
| | Brevundimonas | 24 | 1 | 0.381 | 0.316 | 17 |
| | Brevundimonas | 48 | 0.5 | 0.616 | 0.418 | 32 |
| | Brevundimonas | 48 | 1 | 0.338 | 0.272 | 20 |
| | Pseudomonas 1 | 48 | 0.5 | 0.311 | 0.224 | 28 |
| | Pseudomonas 1 | 48 | 1 | - | - | - |
| | Pseudomonas 2 | 48 | 0.5 | 0.173 | 0.158 | 8.5 |
| | Pseudomonas 2 | 48 | 1 | - | - | - |
| | | | | | | |
| ADW liquid | Brevundimonas | 24 | 0.5 | 0.652 | 0.547 | 16 |
| | Brevundimonas | 24 | 1 | - | - | - |
| | Brevundimonas | 48 | 0.5 | 0.522 | 0.501 | 4.0 |
| | Brevundimonas | 48 | 1 | 0.459 | 0.441 | 10 |
| | Pseudomonas 1 | 48 | 0.5 | - | - | - |
| | Pseudomonas 1 | 48 | 1 | 0.295 | 0.248 | 16 |
| | Pseudomonas 2 | 48 | 0.5 | 0.213 | 0.196 | 7.9 |
| | Pseudomonas 2 | 48 | 1 | 0.260 | 0.208 | 20 |

## Claims

1. Use of a polypeptide having DNase activity for preventing, reducing and/or removing a biofilm from an item, wherein the item is a hard surface.

2. Use according to claim 1 for preventing, reducing and/or removing adherence of soil to the item.

3. Use according to any of the preceding claims, wherein the polypeptide having DNase activity is of animal, vegetable or microbial origin.

4. Use according to any of the preceding claims, wherein the biofilm comprises at least one strain of *Brevundimonas sp.* or at least one strain of *Pseudomonas sp.*

5. A detergent composition comprising a polypeptide having deoxyribonuclease (DNase) activity and a metal care agent.

6. A detergent composition comprising a polypeptide having deoxyribonuclease (DNase) activity and a strong sequestering builder and/or a builder selected from the group consisting of sodium citrate, citric acid, alcanol amines, sodium carbonate, sodium bicarbonate and Amino-tris-(methylene-phosphonic acid) (AMP).

**7.** Composition according to any of claims 5 or 6, wherein the composition further comprises surfactants, builders, flocculating aid, chelating agents, dye transfer inhibitors, enzymes, enzyme stabilizers, enzyme inhibitors, catalytic materials, bleach activators, hydrogen peroxide, sources of hydrogen peroxide, preformed peracids, polymeric dispersing agents, clay soil removal/anti-redeposition agents, brighteners, suds suppressors, dyes, perfumes, structure elasticizing agents, fabric softeners, carriers, hydrotropes, builders and co-builders, fabric hueing agents, antifoaming agents, dispersants, processing aids, bacteriocides, fungicides and/or pigments or a mixture thereof.

**8.** Composition according to any of the claims 5-7, wherein the composition further comprises one or more enzymes selected from the group consisting of proteases, lipases, cutinases, amylases, carbohydrases, cellulases, pectinases, mannanases, arabinases, galactanases, xylanases, peroxidase and oxidases.

**9.** Composition according to any of the claims 5-8, wherein the polypeptide having DNase activity is of animal, vegetable, and/or microbial origin.

**10.** Detergent composition according to any of claims 6-9, wherein the strong sequestering builder is selected from the group consisting of EDTA, EDTMP, NTMP, DTPMP, MGDA, NTA, HEDP, STPP, IDS, GLDA, Pyrophosphate and EDDS.

**11.** A cleaning method for preventing, reducing and/or removing a biofilm from an item comprising the steps of:

a) contacting an item to a composition according to any of claims 5-10 or a liquid solution comprising a polypeptide having DNase activity;
b) completing at least one cleaning cycle; and
c) optionally rinsing the item;

wherein the item is a hard surface.

**12.** Method according to claim 11, wherein the item is a hard surface, which hard surface is the interior surface of a dishwashing machine or a washing machine for a textile.

**13.** Method according to claim 11, wherein the item is a dishware selected from the group consisting of plates, cups, glasses, bowls, pots, cutlery, spoons, knives, forks, serving utensils, ceramics, plastics, cutting boards, china and glass ware.

**14.** Method according to any of claims 11-13, wherein the dishware is cleaned simultaneously with the cleaning of the interior hard surface.

**15.** Method according to any of claims 11-14, wherein the polypeptide having DNase activity is of animal, vegetable, and/or microbial origin.

Europäisches Patentamt
European Patent Office
Office européen des brevets

# EUROPEAN SEARCH REPORT

Application Number
EP 18 19 2650

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2011/098579 A1 (UNIV NEWCASTLE [GB]; BURGESS JAMES GRANT [GB]; HALL MICHAEL JOHN [GB];) 18 August 2011 (2011-08-18)<br>* paragraph [0063] *<br>* paragraph [0105] *<br>* paragraph [0155] *<br>* paragraph [0159] *<br>* examples 1,5 *<br>----- | 1-4 | INV.<br>C11D3/386 |
| X | G. V. TETZ ET AL: "Effect of DNase and Antibiotics on Biofilm Characteristics", ANTIMICROBIAL AGENTS AND CHEMOTHERAPY, vol. 53, no. 3, 1 March 2009 (2009-03-01), pages 1204-1209, XP055002507, ISSN: 0066-4804, DOI: 10.1128/AAC.00471-08<br>* page 1204, right-hand column, paragraph 2 - page 1205, left-hand column, paragraph 3 *<br>* figure 2 *<br>----- | 1,3,4 | |
| X | TETZ VICTOR V ET AL: "Effect of extracellular DNA destruction by DNase I on characteristics of forming biofilms", DNA AND CELL BIOLOGY, MARY ANN LIEBERT, NEW YORK, NY, US, vol. 29, no. 8, 1 August 2010 (2010-08-01), pages 399-405, XP009155541, ISSN: 1044-5498, DOI: 10.1089/DNA.2009.1011 [retrieved on 2010-05-22]<br>* page 399, right-hand column, paragraph 1 - page 400, right-hand column, paragraph 4 *<br>* figure 1 *<br>-----<br>-/-- | 1,3 | TECHNICAL FIELDS SEARCHED (IPC)<br>C11D |

The present search report has been drawn up for all claims

1

| Place of search | Date of completion of the search | Examiner |
|---|---|---|
| Munich | 12 March 2019 | Ulbrecht, Matthias |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 18 19 2650

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 01/46512 A2 (LEE CLARENCE C [US]) 28 June 2001 (2001-06-28) * page 7, lines 10-21 * * page 2, line 15 - page 3, line 19 * * claims 1-20 * ----- | 1-15 | |
| X,P | WO 2014/087011 A1 (NOVOZYMES AS [DK]) 12 June 2014 (2014-06-12) * page 12, line 11 - page 16, line 37 * * examples 1,3 * * tables 1,2 * * claims 1,4,15 * ----- | 1-15 | |

TECHNICAL FIELDS SEARCHED (IPC)

The present search report has been drawn up for all claims

| Place of search | Date of completion of the search | Examiner |
|---|---|---|
| Munich | 12 March 2019 | Ulbrecht, Matthias |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 2

# ANNEX TO THE EUROPEAN SEARCH REPORT ON EUROPEAN PATENT APPLICATION NO. EP 18 19 2650

This annex lists the patent family members relating to the patent documents cited in the above-mentioned European search report. The members are as contained in the European Patent Office EDP file on
The European Patent Office is in no way liable for these particulars which are merely given for the purpose of information.

12-03-2019

| Patent document cited in search report | Publication date | Patent family member(s) | Publication date |
|---|---|---|---|
| WO 2011098579 A1 | 18-08-2011 | DK 2533801 T3 | 06-11-2017 |
| | | EP 2533801 A1 | 19-12-2012 |
| | | EP 3241559 A1 | 08-11-2017 |
| | | ES 2645376 T3 | 05-12-2017 |
| | | GB 2477914 A | 24-08-2011 |
| | | US 2013052250 A1 | 28-02-2013 |
| | | US 2017333601 A1 | 23-11-2017 |
| | | US 2017340779 A1 | 30-11-2017 |
| | | WO 2011098579 A1 | 18-08-2011 |
| WO 0146512 A2 | 28-06-2001 | NONE | |
| WO 2014087011 A1 | 12-06-2014 | BR 112015012982 A2 | 12-09-2017 |
| | | CA 2893454 A1 | 12-06-2014 |
| | | CN 104837979 A | 12-08-2015 |
| | | EP 2929004 A1 | 14-10-2015 |
| | | JP 2016507597 A | 10-03-2016 |
| | | US 2015299623 A1 | 22-10-2015 |
| | | WO 2014087011 A1 | 12-06-2014 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2011098579 A
- WO 9901534 A
- WO 9724177 A
- EP 14164424 A
- EP 14164429 A
- WO 9517413 A
- WO 9522625 A
- US 5223409 A
- WO 9206204 A
- WO 9219709 A
- WO 9219708 A
- WO 9707202 A
- WO 9426860 A
- WO 9426859 A
- US 4223163 A
- WO 9422800 A
- WO 09102854 A
- US 5977053 A
- WO 9817767 A
- EP 624154 A
- WO 2007087258 A
- WO 2007087244 A
- WO 2007087259 A
- EP 1867708 A
- WO 2007087242 A
- WO 2006130575 A
- WO 200503274 A
- WO 200503275 A
- WO 200503276 A
- EP 1876226 A
- WO 2007087257 A
- WO 2007087243 A
- US 4435307 A
- US 5648263 A
- US 5691178 A
- US 5776757 A
- WO 8909259 A
- EP 0495257 A
- EP 0531372 A
- WO 9611262 A
- WO 9629397 A
- WO 9808940 A
- WO 9407998 A
- EP 0531315 A
- US 5457046 A
- US 5686593 A
- US 5763254 A
- WO 9524471 A
- WO 9812307 A
- WO 99001544 A
- WO 2002099091 A
- WO 2001062903 A
- US 7262042 B
- WO 09021867 A
- WO 8906279 A
- WO 9318140 A
- WO 92175177 A
- WO 01016285 A
- WO 02026024 A
- WO 02016547 A
- WO 8906270 A
- WO 9425583 A
- WO 05040372 A
- WO 05052161 A
- WO 05052146 A
- WO 9523221 A
- WO 9221760 A
- EP 1921147 A
- EP 1921148 A
- WO 07044993 A
- WO 9219729 A
- WO 96034946 A
- WO 9820115 A
- WO 9820116 A
- WO 99011768 A
- WO 0144452 A
- WO 03006602 A
- WO 0403186 A
- WO 04041979 A
- WO 07006305 A
- WO 11036263 A
- WO 11036264 A
- US 5352604 A
- EP 258068 A
- EP 305216 A
- WO 9613580 A
- EP 218272 A
- EP 331376 A
- WO 9506720 A
- WO 9627002 A
- WO 9612012 A
- WO 10065455 A
- WO 10107560 A
- US 5389536 A
- WO 11084412 A
- WO 11084417 A
- WO 11084599 A
- WO 11150157 A

- WO 12137147 A
- EP 407225 A
- WO 9205249 A
- WO 9401541 A
- WO 9425578 A
- WO 9514783 A
- WO 9530744 A
- WO 9535381 A
- WO 9522615 A
- WO 9600292 A
- WO 9704079 A
- WO 0034450 A
- WO 0060063 A
- WO 0192502 A
- WO 0787508 A
- WO 09109500 A
- WO 10111143 A
- WO 0556782 A
- WO 0967279 A
- WO 10100028 A
- GB 1296839 A
- WO 9510603 A
- WO 9402597 A
- WO 9418314 A
- WO 9743424 A
- WO 99019467 A
- WO 02010355 A
- WO 2006066594 A
- WO 96023873 A
- WO 08153815 A
- WO 0166712 A
- WO 09061380 A
- WO 2011098531 A
- WO 2013001078 A
- WO 2013001087 A
- EP 179486 A
- WO 9324618 A
- WO 9510602 A
- WO 9815257 A
- WO 9527046 A
- WO 9704102 A
- WO 0179459 A
- WO 0179458 A
- WO 0179461 A
- WO 0179460 A
- WO 9201046 A
- JP 2238885 A
- WO 9708325 A
- WO 9533836 A
- US 4106991 A
- US 4661452 A
- GB 1483591 A
- EP 238216 A
- WO 2009087523 A
- WO 2007138054 A
- WO 2006108856 A
- WO 2006113314 A
- EP 1867808 A
- WO 2003040279 A
- EP 2169040 A
- US 20090011970 A1
- WO 2013188331 A
- WO 2014032269 A

**Non-patent literature cited in the description**


- **NEEDLEMAN ; WUNSCH.** *J. Mol. Biol.,* 1970, vol. 48, 443-453
- **RICE et al.** EMBOSS: The European Molecular Biology Open Software Suite. *Trends Genet.,* 2000, vol. 16, 276-277
- **RICE et al.** *EM-BOSS: The European Molecular Biology Open Software Suite,* 2000
- **SAMBROOK et al.** Molecular Cloning, A Laboratory Manual. Cold Spring Harbor, 1989
- **H. NEURATH ; R.L. HILL.** The Proteins. Academic Press, 1979
- **CUNNINGHAM ; WELLS.** *Science,* January 1998, vol. 244, 1081-1085
- **HILTON et al.** *J. Biol. Chem.,* 1996, vol. 271, 4699-4708
- **DE VOS et al.** *Science,* 1992, vol. 255, 306-312
- **SMITH et al.** *J. Mol. Biol.,* 1992, vol. 224, 899-904
- **WLODAVER et al.** *FEBS Lett.,* 1992, vol. 309, 59-64
- **REIDHAAR-OLSON ; SAUER.** *Science,* 1988, vol. 241, 53-57
- **BOWIE ; SAUER.** *Proc. Natl. Acad. Sci. USA,* 1989, vol. 86, 2152-2156
- **LOWMAN et al.** *Biochemistry,* 1991, vol. 30, 10832-10837
- **DERBYSHIRE et al.** *Gene,* 1986, vol. 46, 145
- **NER et al.** *DNA,* 1988, vol. 7, 127
- **NESS et al.** *Nature Biotechnology,* 1999, vol. 17, 893-896
- **COOPER et al.** *EMBO J.,* 1993, vol. 12, 2575-2583
- **DAWSON et al.** *Science,* 1994, vol. 266, 776-779
- **MARTIN et al.** *J. Ind. Microbiol. Biotechnol.,* 2003, vol. 3, 568-576
- **SVETINA et al.** *J. Biotechnol.,* 2000, vol. 76, 245-251
- **RASMUSSEN-WILSON et al.** *Appl. Environ. Microbiol.,* 1997, vol. 63, 3488-3493

- **WARD et al.** *Biotechnology,* 1995, vol. 13, 498-503
- **CONTRERAS et al.** *Biotechnology,* 1991, vol. 9, 378-381
- **EATON et al.** *Biochemistry,* 1986, vol. 25, 505-512
- **COLLINS-RACIE et al.** *Biotechnology,* 1995, vol. 13, 982-987
- **CARTER et al.** *Proteins: Structure, Function, and Genetics,* 1989, vol. 6, 240-248
- **STEVENS.** *Drug Discovery World,* 2003, vol. 4, 35-48
- **CUNNINGHAM ; WELLS.** *Science,* 1989, vol. 244, 1081-1085
- **HODGDON ; KALER.** *Current Opinion in Colloid & Interface Science,* 2007, vol. 12, 121-128
- **SIEZEN et al.** *Protein Engng.,* 1991, vol. 4, 719-737
- **SIEZEN et al.** *Protein Science,* 1997, vol. 6, 501-523
- Powdered Detergents, Surfactant science series volume 71. Marcel Dekker, Inc, vol. 71
- Chapter 7 in Powdered Detergents, Surfactant science series volume 71. Marcel Dekker, Inc, vol. 71

SEQUENCE LISTING

```
<110>  Novozymes A/S

<120>  Use of polypeptide

<130>  12950-WO-PCT

<140>  -
<141>  2015-04-29

<160>  8

<170>  PatentIn version 3.5

<210>  1
<211>  910
<212>  DNA
<213>  Aspergillus oryzae


<220>
<221>  exon
<222>  (1)..(242)

<220>
<221>  Intron
<222>  (243)..(308)

<220>
<221>  exon
<222>  (309)..(494)

<220>
<221>  Intron
<222>  (495)..(555)

<220>
<221>  exon
<222>  (556)..(714)

<220>
<221>  Intron
<222>  (715)..(765)

<220>
<221>  exon
<222>  (766)..(907)

<220>
<221>  Intron
<222>  (908)..(910)

<400>  1
atg cag ctt act aag tcc ctc ctg gta ttc gcg ctt tac atg ttt ggc        48
Met Gln Leu Thr Lys Ser Leu Leu Val Phe Ala Leu Tyr Met Phe Gly
1               5                   10                  15

act cag cac gtt cta gct gtg cct gtc aat ccc gag cct gat gct acg        96
Thr Gln His Val Leu Ala Val Pro Val Asn Pro Glu Pro Asp Ala Thr
            20                  25                  30

agc gtc gaa aat gtt gcc ctt aaa aca ggc agc ggt gat agc cag agc       144
```

```
Ser Val Glu Asn Val Ala Leu Lys Thr Gly Ser Gly Asp Ser Gln Ser
        35                  40                  45

gat ccc atc aag gcg gac ttg gag gtc aaa ggc caa agt gct ttg cct        192
Asp Pro Ile Lys Ala Asp Leu Glu Val Lys Gly Gln Ser Ala Leu Pro
    50                  55                  60

ttc gac gtc gac tgc tgg gct atc ctg tgc aag ggc gcc ccg aat gtc        240
Phe Asp Val Asp Cys Trp Ala Ile Leu Cys Lys Gly Ala Pro Asn Val
65                  70                  75                  80

ct  gtatgtcttc ctttattgaa gctcttgatg tggcttgtat gtttgactaa             292
Leu

tatatcgcac ccttag g cag cgc gtg aat gaa aag acg aaa aat agt aat        342
                    Gln Arg Val Asn Glu Lys Thr Lys Asn Ser Asn
                                85                  90

cgc gat cgg agc ggt gcg aac aaa ggg cct ttc aaa gat cct cag aaa        390
Arg Asp Arg Ser Gly Ala Asn Lys Gly Pro Phe Lys Asp Pro Gln Lys
        95                  100                 105

tgg ggc atc aaa gcc ctt cca cct aag aat cca tcc tgg agc gca caa        438
Trp Gly Ile Lys Ala Leu Pro Pro Lys Asn Pro Ser Trp Ser Ala Gln
    110                 115                 120

gac ttc aaa tca ccc gaa gaa tac gca ttt gcg tct tcc ctt caa ggc        486
Asp Phe Lys Ser Pro Glu Glu Tyr Ala Phe Ala Ser Ser Leu Gln Gly
125                 130                 135                 140

gga acc aa  gtatgctaag atcatcactg cttcaatcaa tgtgttgtta                534
Gly Thr Asn

gctgactccg atgtgaccaa g t gcc atc cta gcg ccc gtc aac ctc gct tct      586
                          Ala Ile Leu Ala Pro Val Asn Leu Ala Ser
                              145                 150

cag aac tcc caa ggc ggc gtc ttg aac ggt ttc tac tcg gcg aac aaa        634
Gln Asn Ser Gln Gly Gly Val Leu Asn Gly Phe Tyr Ser Ala Asn Lys
    155                 160                 165

gta gca caa ttt gat cct agc aag ccc caa cag aca aag gga aca tgg        682
Val Ala Gln Phe Asp Pro Ser Lys Pro Gln Gln Thr Lys Gly Thr Trp
170                 175                 180                 185

ttt cag atc act aag ttc aca ggt gca gct gg  gtaagaactt ccagtaccat      734
Phe Gln Ile Thr Lys Phe Thr Gly Ala Ala Gly
            190                 195

ggtcatatgc aatttactaa gaaaatacta g t cct tac tgc aag gct ctg ggg       787
                                     Pro Tyr Cys Lys Ala Leu Gly
                                                 200

agt aat gat aag agt gtg tgc gat aag aac aag aat att gca ggg gac        835
Ser Asn Asp Lys Ser Val Cys Asp Lys Asn Lys Asn Ile Ala Gly Asp
    205                 210                 215

tgg ggc ttc gac ccg gcg aaa tgg gca tat cag tat gat gag aag aat        883
Trp Gly Phe Asp Pro Ala Lys Trp Ala Tyr Gln Tyr Asp Glu Lys Asn
220                 225                 230                 235
```

```
aac aag ttc aac tat gtt ggt aag taa                                910
Asn Lys Phe Asn Tyr Val Gly Lys
                240


<210>  2
<211>  243
<212>  PRT
<213>  Aspergillus oryzae


<220>
<221>  SIGNAL
<222>  (1)..(22)

<220>
<221>  PROPEP
<222>  (23)..(37)

<220>
<221>  PEPTIDE
<222>  (38)..(243)

<400>  2

Met Gln Leu Thr Lys Ser Leu Leu Val Phe Ala Leu Tyr Met Phe Gly
1               5                   10                  15


Thr Gln His Val Leu Ala Val Pro Val Asn Pro Glu Pro Asp Ala Thr
            20                  25                  30


Ser Val Glu Asn Val Ala Leu Lys Thr Gly Ser Gly Asp Ser Gln Ser
        35                  40                  45


Asp Pro Ile Lys Ala Asp Leu Glu Val Lys Gly Gln Ser Ala Leu Pro
    50                  55                  60


Phe Asp Val Asp Cys Trp Ala Ile Leu Cys Lys Gly Ala Pro Asn Val
65                  70                  75                  80


Leu Gln Arg Val Asn Glu Lys Thr Lys Asn Ser Asn Arg Asp Arg Ser
                85                  90                  95


Gly Ala Asn Lys Gly Pro Phe Lys Asp Pro Gln Lys Trp Gly Ile Lys
            100                 105                 110


Ala Leu Pro Pro Lys Asn Pro Ser Trp Ser Ala Gln Asp Phe Lys Ser
        115                 120                 125


Pro Glu Glu Tyr Ala Phe Ala Ser Ser Leu Gln Gly Gly Thr Asn Ala
    130                 135                 140


Ile Leu Ala Pro Val Asn Leu Ala Ser Gln Asn Ser Gln Gly Gly Val
145                 150                 155                 160
```

Leu Asn Gly Phe Tyr Ser Ala Asn Lys Val Ala Gln Phe Asp Pro Ser
165 170 175

Lys Pro Gln Gln Thr Lys Gly Thr Trp Phe Gln Ile Thr Lys Phe Thr
180 185 190

Gly Ala Ala Gly Pro Tyr Cys Lys Ala Leu Gly Ser Asn Asp Lys Ser
195 200 205

Val Cys Asp Lys Asn Lys Asn Ile Ala Gly Asp Trp Gly Phe Asp Pro
210 215 220

Ala Lys Trp Ala Tyr Gln Tyr Asp Glu Lys Asn Asn Lys Phe Asn Tyr
225 230 235 240

Val Gly Lys

<210> 3
<211> 204
<212> PRT
<213> Aspergillus oryzae

<220>
<221> PEPTIDE
<222> (1)..(204)

<400> 3

Lys Thr Gly Ser Gly Asp Ser Gln Ser Asp Pro Ile Lys Ala Asp Leu
1 5 10 15

Glu Val Lys Gly Gln Ser Ala Leu Pro Phe Asp Val Asp Cys Trp Ala
20 25 30

Ile Leu Cys Lys Gly Ala Pro Asn Val Leu Gln Arg Val Asn Glu Lys
35 40 45

Thr Lys Asn Ser Asn Arg Asp Arg Ser Gly Ala Asn Lys Gly Pro Phe
50 55 60

Lys Asp Pro Gln Lys Trp Gly Ile Lys Ala Leu Pro Pro Lys Asn Pro
65 70 75 80

Ser Trp Ser Ala Gln Asp Phe Lys Ser Pro Glu Glu Tyr Ala Phe Ala
85 90 95

Ser Ser Leu Gln Gly Gly Thr Asn Ala Ile Leu Ala Pro Val Asn Leu

100 105 110

Ala Ser Gln Asn Ser Gln Gly Gly Val Leu Asn Gly Phe Tyr Ser Ala
115 120 125

Asn Lys Val Ala Gln Phe Asp Pro Ser Lys Pro Gln Gln Thr Lys Gly
130 135 140

Thr Trp Phe Gln Ile Thr Lys Phe Thr Gly Ala Ala Gly Pro Tyr Cys
145 150 155 160

Lys Ala Leu Gly Ser Asn Asp Lys Ser Val Cys Asp Lys Asn Lys Asn
165 170 175

Ile Ala Gly Asp Trp Gly Phe Asp Pro Ala Lys Trp Ala Tyr Gln Tyr
180 185 190

Asp Glu Lys Asn Asn Lys Phe Asn Tyr Val Gly Lys
195 200

<210> 4
<211> 868
<212> DNA
<213> Trichoderma harzianum

<220>
<221> exon
<222> (1)..(75)

<220>
<221> Intron
<222> (76)..(154)

<220>
<221> exon
<222> (155)..(288)

<220>
<221> Intron
<222> (289)..(362)

<220>
<221> exon
<222> (363)..(519)

<220>
<221> Intron
<222> (520)..(615)

<220>
<221> exon
<222> (616)..(867)

<400> 4
atg aag ctg tcc atc tct gtc gct ctt act tcg gcc atc gcg gtt ctc 48

```
Met Lys Leu Ser Ile Ser Val Ala Leu Thr Ser Ala Ile Ala Val Leu
1               5                   10                  15

gcc gcc ccg gct cct atg cct aca ccg gtatgtagca tcaatgcaac         95
Ala Ala Pro Ala Pro Met Pro Thr Pro
            20                  25

atgacataac ttgtatctcg actatatatc agactggcta atgcttcaac tcattacag 154

ccc ggt att ccc acg gaa agc agc gcc aga acc caa ctt gcc ggc ctg  202
Pro Gly Ile Pro Thr Glu Ser Ser Ala Arg Thr Gln Leu Ala Gly Leu
                30                  35                  40

act gtt gcc gtt gct ggc tct gga act ggt tac tcc cgc gac ctg ttt  250
Thr Val Ala Val Ala Gly Ser Gly Thr Gly Tyr Ser Arg Asp Leu Phe
            45                  50                  55

ccc act tgg gat gcc atc tct ggt aac tgc aac gct cg  gtatgataac   298
Pro Thr Trp Asp Ala Ile Ser Gly Asn Cys Asn Ala Arg
        60                  65                  70

atcctaggac ctttcaagct tcggaaatac aacacaaagg ctaacaaagt ggatgtgcaa 358

atag c gaa tat gtg ttg aag cga gat ggt gaa ggt gtc caa gtc aac   405
       Glu Tyr Val Leu Lys Arg Asp Gly Glu Gly Val Gln Val Asn
                       75                  80

aat gct tgt gaa tct cag tcc ggc acc tgg atc aga tcc tta tga caa  453
Asn Ala Cys Glu Ser Gln Ser Gly Thr Trp Ile Arg Ser Leu     Gln
85                  90                  95

cgc cag ttt cac aaa tgc atc cag ctt gga tat tga cca cat ggt gcc  501
Arg Gln Phe His Lys Cys Ile Gln Leu Gly Tyr     Pro His Gly Ala
100                 105                 110

tct aaa gaa tgc ctg gat cgtgagtttt ctcctttttc actgcgtatc         549
Ser Lys Glu Cys Leu Asp
115                 120

tccgttccct accttttgc gatactatat catgccacat cactaatatg gacaaatttc 609

tcgcca gtc cgg tgc ctc aag ctg gac cac agc cca acg tga agc cct   657
       Val Arg Cys Leu Lys Leu Asp His Ser Pro Thr     Ser Pro
                       125                 130

cgc caa cga cgt ctc ccg tcc cca act ctg ggc cgt ctc cgc aag cgc  705
Arg Gln Arg Arg Leu Pro Ser Pro Thr Leu Gly Arg Leu Arg Lys Arg
    135                 140                 145

aaa ccg ctc caa ggg cga ccg cag ccc aga cca gtg gaa gcc tcc tct  753
Lys Pro Leu Gln Gly Arg Pro Gln Pro Arg Pro Val Glu Ala Ser Ser
150                 155                 160                 165

gac cag ctt cta ctg cac cta cgc caa gtc gtg gat cga tgt caa gag  801
Asp Gln Leu Leu Leu His Leu Arg Gln Val Val Asp Arg Cys Gln Glu
                170                 175                 180

ctt cta taa gct gac aat cac cag tgc cga gaa gac agc tct gag cag  849
Leu Leu     Ala Asp Asn His Gln Cys Arg Glu Asp Ser Ser Glu Gln
                185                 190                 195

cat gtt aga tac ttg cta g                                        868
His Val Arg Tyr Leu Leu
```

200

<210> 5
<211> 205
<212> PRT
<213> Trichoderma harzianum

<220>
<221> SIGNAL
<222> (1)..(17)

<220>
<221> PEPTIDE
<222> (18)..(205)

<400> 5

```
Met Lys Leu Ser Ile Ser Val Ala Leu Thr Ser Ala Ile Ala Val Leu
1               5                   10                  15

Ala Ala Pro Ala Pro Met Pro Thr Pro Pro Gly Ile Pro Thr Glu Ser
            20                  25                  30

Ser Ala Arg Thr Gln Leu Ala Gly Leu Thr Val Ala Val Ala Gly Ser
        35                  40                  45

Gly Thr Gly Tyr Ser Arg Asp Leu Phe Pro Thr Trp Asp Ala Ile Ser
    50                  55                  60

Gly Asn Cys Asn Ala Arg Glu Tyr Val Leu Lys Arg Asp Gly Glu Gly
65                  70                  75                  80

Val Gln Val Asn Asn Ala Cys Glu Ser Gln Ser Gly Thr Trp Ile Ser
                85                  90                  95

Pro Tyr Asp Asn Ala Ser Phe Thr Asn Ala Ser Ser Leu Asp Ile Asp
            100                 105                 110

His Met Val Pro Leu Lys Asn Ala Trp Ile Ser Gly Ala Ser Ser Trp
        115                 120                 125

Thr Thr Ala Gln Arg Glu Ala Leu Ala Asn Asp Val Ser Arg Pro Gln
    130                 135                 140

Leu Trp Ala Val Ser Ala Ser Ala Asn Arg Ser Lys Gly Asp Arg Ser
145                 150                 155                 160

Pro Asp Gln Trp Lys Pro Pro Leu Thr Ser Phe Tyr Cys Thr Tyr Ala
                165                 170                 175
```

```
Lys Ser Trp Ile Asp Val Lys Ser Phe Tyr Lys Leu Thr Ile Thr Ser
            180                 185                 190

Ala Glu Lys Thr Ala Leu Ser Ser Met Leu Asp Thr Cys
        195                 200                 205

<210>  6
<211>  136
<212>  PRT
<213>  Bacillus subtilis

<220>
<221>  SIGNAL
<222>  (1)..(26)

<220>
<221>  PEPTIDE
<222>  (27)..(136)

<400>  6

Met Lys Lys Trp Met Ala Gly Leu Phe Leu Ala Ala Ala Val Leu Leu
1               5                   10                  15

Cys Leu Met Val Pro Gln Gln Ile Gln Gly Ala Ser Ser Tyr Asp Lys
            20                  25                  30

Val Leu Tyr Phe Pro Leu Ser Arg Tyr Pro Glu Thr Gly Ser His Ile
        35                  40                  45

Arg Asp Ala Ile Ala Glu Gly His Pro Asp Ile Cys Thr Ile Asp Arg
    50                  55                  60

Asp Gly Ala Asp Lys Arg Arg Glu Glu Ser Leu Lys Gly Ile Pro Thr
65                  70                  75                  80

Lys Pro Gly Tyr Asp Arg Asp Glu Trp Pro Met Ala Val Cys Glu Glu
                85                  90                  95

Gly Gly Ala Gly Ala Asp Val Arg Tyr Val Thr Pro Ser Asp Asn Arg
            100                 105                 110

Gly Ala Gly Ser Trp Val Gly Asn Gln Met Ser Ser Tyr Pro Asp Gly
        115                 120                 125

Thr Arg Val Leu Phe Ile Val Gln
    130                 135

<210>  7
<211>  142
<212>  PRT
```

<213> Bacillus licheniformis

<220>
<221> SIGNAL
<222> (1)..(33)

<220>
<221> PEPTIDE
<222> (34)..(136)

<400> 7

```
Met Ile Lys Lys Trp Ala Val His Leu Leu Phe Ser Ala Leu Val Leu
1               5                   10                  15

Leu Gly Leu Ser Gly Gly Ala Ala Tyr Ser Pro Gln His Ala Glu Gly
            20                  25                  30

Ala Ala Arg Tyr Asp Asp Ile Leu Tyr Phe Pro Ala Ser Arg Tyr Pro
        35                  40                  45

Glu Thr Gly Ala His Ile Ser Asp Ala Ile Lys Ala Gly His Ser Asp
    50                  55                  60

Val Cys Thr Ile Glu Arg Ser Gly Ala Asp Lys Arg Arg Gln Glu Ser
65                  70                  75                  80

Leu Lys Gly Ile Pro Thr Lys Pro Gly Phe Asp Arg Asp Glu Trp Pro
                85                  90                  95

Met Ala Met Cys Glu Glu Gly Gly Lys Gly Ala Ser Val Arg Tyr Val
            100                 105                 110

Ser Ser Ser Asp Asn Arg Gly Ala Gly Ser Trp Val Gly Asn Arg Leu
        115                 120                 125

Ser Gly Phe Ala Asp Gly Thr Arg Ile Leu Phe Ile Val Gln
    130                 135                 140
```

<210> 8
<211> 206
<212> PRT
<213> Aspergillus oryzae

<400> 8

```
Ala Leu Lys Thr Gly Ser Gly Asp Ser Gln Ser Asp Pro Ile Lys Ala
1               5                   10                  15

Asp Leu Glu Val Lys Gly Gln Ser Ala Leu Pro Phe Asp Val Asp Cys
            20                  25                  30
```

```
Trp Ala Ile Leu Cys Lys Gly Ala Pro Asn Val Leu Gln Arg Val Asn
        35                  40                  45

Glu Lys Thr Lys Asn Ser Asn Arg Asp Arg Ser Gly Ala Asn Lys Gly
    50                  55                  60

Pro Phe Lys Asp Pro Gln Lys Trp Gly Ile Lys Ala Leu Pro Pro Lys
65                  70                  75                  80

Asn Pro Ser Trp Ser Ala Gln Asp Phe Lys Ser Pro Glu Glu Tyr Ala
                85                  90                  95

Phe Ala Ser Ser Leu Gln Gly Gly Thr Asn Ala Ile Leu Ala Pro Val
            100                 105                 110

Asn Leu Ala Ser Gln Asn Ser Gln Gly Gly Val Leu Asn Gly Phe Tyr
        115                 120                 125

Ser Ala Asn Lys Val Ala Gln Phe Asp Pro Ser Lys Pro Gln Gln Thr
    130                 135                 140

Lys Gly Thr Trp Phe Gln Ile Thr Lys Phe Thr Gly Ala Ala Gly Pro
145                 150                 155                 160

Tyr Cys Lys Ala Leu Gly Ser Asn Asp Lys Ser Val Cys Asp Lys Asn
                165                 170                 175

Lys Asn Ile Ala Gly Asp Trp Gly Phe Asp Pro Ala Lys Trp Ala Tyr
            180                 185                 190

Gln Tyr Asp Glu Lys Asn Asn Lys Phe Asn Tyr Val Gly Lys
        195                 200                 205
```